# EUROPEAN PATENT SPECIFICATION

(11) **EP 3 720 972 B1**
(45) Date of publication and mention of the grant of the patent: **02.09.2026**
(21) Application number: 18829644.6
(22) Date of filing: 07.12.2018
(51) Int. Cl.: C12Q 1/6841

(54) **MULTIPLEX LABELING OF MOLECULES BY SEQUENTIAL HYBRIDIZATION BARCODING WITH RAPID SWITCHING AND REHYBRIDZATION OF PROBES**
MULTIPLEXMARKIERUNG VON MOLEKÜLEN DURCH BARCODIERUNG MIT SEQUENZIELLER HYBRIDISIERUNG MIT SCHNELLEM WECHSEL UND REHYBRIDISIERUNG VON SONDEN
MARQUAGE MULTIPLEX DE MOLÉCULES PAR MARQUAGE PAR CODE-BARRES SÉQUENTIEL D'HYBRIDATION AVEC COMMUTATION RAPIDE ET RÉHYBRIDATION DE SONDES

(30) Priority: 08.12.2017 US 201762596337 P
(43) Date of publication of application: 14.10.2020
(73) Proprietor: California Institute of Technology, Pasadena, CA 91125 (US)
(72) Inventor: CAI, Long, Pasadena CA 91125 (US); TAKEI, Yodai, Pasadena CA 91125 (US)
(74) Representative: Murgitroyd & Company
(86) International application number: PCT/US2018/064616
(87) International publication number: WO 2019/113547

(56) References cited:
- WO-A1-2015/118029
- WO-A1-2016/018960
- WO-A1-2017/189525
- WO-A1-2018/026873
- WO-A1-2018/044939
- WO-A2-2014/182528
- US-A1- 2016 369 329
- US-A1- 2017 212 983
- HARRY M T CHOI ET AL: "Programmable in situ amplification for multiplexed imaging of mRNA expression", NATURE BIOTECHNOLOGY, vol. 28, no. 11, 31 October 2010 (2010-10-31), New York, pages 1208 - 1212, XP055554132, ISSN: 1087-0156, DOI: 10.1038/nbt.1692
- HARRY M. T. CHOI ET AL: "Next-Generation in Situ Hybridization Chain Reaction: Higher Gain, Lower Cost, Greater Durability", ACS NANO, vol. 8, no. 5, 27 May 2014 (2014-05-27), US, pages 4284 - 4294, XP055409053, ISSN: 1936-0851, DOI: 10.1021/nn405717p
- HARRY M. T. CHOI ET AL: "Third-generation in situ hybridization chain reaction: multiplexed, quantitative, sensitive, versatile, robust", DEVELOPMENT, vol. 145, no. 12, 15 June 2018 (2018-06-15), GB, pages dev165753, XP055590305, ISSN: 0950-1991, DOI: 10.1242/dev.165753
- HUSS DAVID ET AL: "Combinatorial Analysis of mRNA Expression Patterns in Mouse Embryos Using Hybridization Chain Reaction", COLD SPRING HARBOR PROTOCOL, vol. 2015, no. 3, 1 March 2015 (2015-03-01), United States, pages pdb.prot083832, XP055856719, ISSN: 1940-3402, Retrieved from the Internet <URL:http://cshprotocols.cshlp.org/content/2015/3/pdb.prot083832.full.pdf> DOI: 10.1101/pdb.prot083832
- JIN HUANG ET AL: "A supersandwich fluorescence in situ hybridization strategy for highly sensitive and selective mRNA imaging in tumor cells", CHEMICAL COMMUNICATIONS, vol. 52, no. 2, 1 January 2016 (2016-01-01), UK, pages 370 - 373, XP055554128, ISSN: 1359-7345, DOI: 10.1039/C5CC08503A
- CHEE-HUAT LINUS ENG ET AL: "Profiling the transcriptome with RNA SPOTs", NATURE METHODS, vol. 14, no. 12, 13 November 2017 (2017-11-13), New York, pages 1153 - 1155, XP055555537, ISSN: 1548-7091, DOI: 10.1038/nmeth.4500
- ANONYMOUS: "Supplementary Protocol for RNA SPOTs : Protocol Exchange", 13 November 2017 (2017-11-13), XP055555720, Retrieved from the Internet <URL:https://www.nature.com/protocolexchange/protocols/6353#/author-information> [retrieved on 20190212]
- ERIC LUBECK ET AL: "Single-cell in situ RNA profiling by sequential hybridization", NATURE METHODS, vol. 11, no. 4, 1 April 2014 (2014-04-01), New York, pages 360 - 361, XP055444270, ISSN: 1548-7091, DOI: 10.1038/nmeth.2892
- SHAH SHEEL ET AL: "In Situ Transcription Profiling of Single Cells Reveals Spatial Organization of Cells in the Mouse Hippocampus", NEURON, vol. 92, no. 2, 1 April 2014 (2014-04-01), pages 342 - 357, XP029778129, ISSN: 0896-6273, DOI: 10.1016/J.NEURON.2016.10.001
- MARLON STOECKIUS ET AL: "Simultaneous epitope and transcriptome measurement in single cells", NATURE METHODS, vol. 14, no. 9, 31 July 2017 (2017-07-31), New York, pages 865 - 868, XP055556042, ISSN: 1548-7091, DOI: 10.1038/nmeth.4380
- SHAH SHEEL ET AL: "seqFISH Accurately Detects Transcripts in Single Cells and Reveals Robust Spatial Organization in the Hippocampus", NEURON, CELL PRESS, US, vol. 94, no. 4, 17 May 2017 (2017-05-17), pages 752, XP085026697, ISSN: 0896-6273, DOI: 10.1016/J.NEURON.2017.05.008

## Description

### CROSS-REFERENCE TO RELATED APPLICATIONS

This application claims the benefit of United States Provisional Application Serial No. 62/596,337, filed December 8, 2017.

### STATEMENT REGARDING FEDERALLY SPONSORED RESEARCH OR

### DEVELOPMENT

This invention was made with government support under Grant No. HD075605 awarded by the National Institutes of Health. The U.S. government has certain rights in the invention.

### BACKGROUND OF THE INVENTION

Transcription profiling of cells are essential for many purposes. Microscopy imaging which can resolve multiple mRNAs in single cells can provide valuable information regarding transcript abundance and localization, which are important for understanding the molecular basis of cell identify and developing treatment for diseases. Therefore, there is a need for new and improved methods for profile transcripts in cells by, for example, microscopy imaging.

### SUMMARY OF THE INVENTION

The present invention provides certain insights into challenges or defects associated with existing technologies for profiling transcripts or DNA loci in cells, particularly for single cells. Moreover, the present invention provides new technologies for achieving effective such profiling, including of single cells. Provided technologies are broadly useful, including for example for profiling of isolated cells, cells in tissues, cells in organs, and/or cells in organisms.

For example, the present invention provides the insight that existing technologies such as single cell RNA-seq or qPCR require single cells to be isolated and put into multi-well format, which is a multiple step process that can be cost prohibitive, labor intensive and prone to artifacts. Furthermore, the present invention recognizes that existing *in situ* sequencing technologies that use enzymatic reactions to convert the mRNA into a DNA template first can be highly inefficient (for example in the mRNA to DNA conversion process), so that, often, only a small fraction of the RNAs are converted and detected. The present invention provides the particular insight that one major downside of such low efficiency, which is estimated at 1% for RT and 10% for PLA, is that it can introduce significant noise ad bias in the gene expression measurements. The present invention further recognizes that existing spectral mRNA barcoding technologies that utilize single molecule fluorescence in situ hybridization (smFISH) require distinct fluorophores for scale up, and may be limited in the number of barcodes that can be generated. smFISH also requires splitting probes into barcoding subsets during hybridization. Because smFISH often uses two or more colors for a target, it produces high density of objects in the image, which can increase the complexity of data analysis.

Among other things, the present inventions provides new technologies for profiling, for example, transcripts and/or DNA loci, that overcome one or more or all of the problems associated with methods prior to the present invention. In some embodiments, the present invention provides methods for detecting multiple targets, *e*.*g*., transcripts or DNA loci, in a cell through a sequential barcoding scheme that permits multiplexing of different targets.

In some embodiments, the present invention provides methods, comprising steps of:
(a) performing a first contacting step that involves contacting a cell comprising a plurality of nucleic acids with a first plurality of detectably labeled oligonucleotides, each of which targets a nucleic acid and is labeled with a detectable moiety, so that the composition comprises atleast:
   (i) a first oligonucleotide targeting a first nucleic acid and labeled with a first detectable moiety; and
   (ii) a second oligonucleotide targeting a second nucleic acid and labeled with a second detectable moiety;
(b) imaging the cell after the first contacting step so that interaction by oligonucleotides of the first plurality with their targets is detected;
(c) performing a second contacting step that involves contacting the cell with a second plurality of detectably labeled oligonucleotides, which second plurality includes oligonucleotides targeting overlapping nucleic acids that are targeted by the first plurality, so that the second plurality comprises at least:
   (i) a third oligonucleotide, optionally identical in sequence to the first oligonucleotide, targeting the first nucleic acid; and
   (ii) a fourth oligonucleotide, optionally identical in sequence to the second oligonucleotide, targeting the second nucleic acid,

   wherein the second plurality differs from the first plurality in that at least one of the oligonucleotides present in the second plurality is labeled with a different detectable moiety than the corresponding oligonucleotide targeting the same nucleic acid in the first plurality, so that, in the second plurality:
      (iii) the third oligonucleotide is labeled with the first detectable moiety, the second detectable moiety or a third detectable moiety; and
      (iv) the fourth oligonucleotide is labeled with the first detectable moiety, the second detectable moiety, the third detectable moiety, or a fourth detectable moiety,
   wherein either the third oligonucleotide is labeled with a different detectable moiety than was the first oligonucleotide, or the fourth oligonucleotide is labeled with a different detectable moiety than was the second oligonucleotide, or both;
(d) imaging the cell after the second contacting step so that interaction by oligonucleotides of the second plurality with their targets is detected; and
(e) optionally repeating the contacting and imaging steps, each time with a new plurality of detectably labeled oligonucleotides comprising oligonucleotides that target overlapping nucleic acids targeted by the first and second pluralities, wherein each utilized plurality differs from each other utilized plurality, due to at least one difference in detectable moiety labeling of oligonucleotides targeting the same nucleic acid.

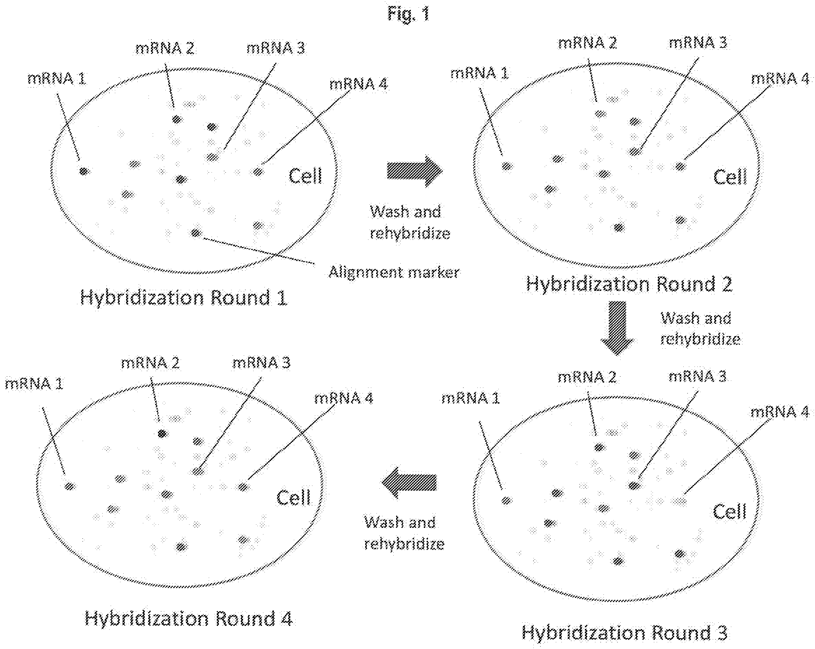

In some embodiments, the present invention (e.g., as represented in Figure 1), provides methods comprising steps of:
(a) performing a first contacting step that involves contacting a cell comprising a plurality of transcripts and DNA loci with a first plurality of detectably labeled oligonucleotides, each of which targets a transcript or DNA locus and is labeled with a detectable moiety, so that the composition comprises at least:
   (i) a first oligonucleotide targeting a first transcript or DNA locus and labeled with a first detectable moiety; and
   (ii) a second oligonucleotide targeting a second transcript or DNA locus and labeled with a second detectable moiety;
(b) imaging the cell after the first contacting step so that recognition by oligonucleotides of the first plurality with their targets is detected;
(c) performing a second contacting step that involves contacting the cell with a second plurality of detectably labeled oligonucleotides, which second plurality includes oligonucleotides targeting overlapping transcripts and/or DNA loci that are targeted by the first plurality, so that the second plurality comprises at least:
   (i) a third oligonucleotide, optionally identical in sequence to the first oligonucleotide, targeting the first transcript or DNA locus; and
   (ii) a fourth oligonucleotide, optionally identical in sequence to the second oligonucleotide, targeting the second transcript or DNA locus,
   wherein the second plurality differs from the first plurality in that at least one of the oligonucleotides present in the second plurality is labeled with a different detectable moiety than the corresponding oligonucleotide targeting the same transcript or DNA locus in the first plurality, so that, in the second plurality:
   (iii) the third oligonucleotide is labeled with the first detectable moiety, the second detectable moiety or a third detectable moiety; and
   (iv) the fourth oligonucleotide is labeled with the first detectable moiety, the second detectable moiety, the third detectable moiety, or a fourth detectable moiety, wherein either the third oligonucleotide is labeled with a different detectable moiety than was the first oligonucleotide, or the fourth oligonucleotide is labeled with a different detectable moiety than was the second oligonucleotide, or both;
(d) imaging the cell after the second contacting step so that recognition by oligonucleotides of the second plurality with their targets is detected; and
(e) optionally repeating the contacting and imaging steps, each time with a new plurality of detectably labeled oligonucleotides comprising oligonucleotides that target overlapping transcripts or DNA loci targeted by the first and second pluralities, wherein each utilized plurality differs from each other utilized plurality, due to at least one difference in detectable moiety labeling of oligonucleotides targeting the same transcript or DNA locus.

In some embodiments, a nucleic acid targeted by a detectably labeled oligonucleotide is or comprises a transcript and/or DNA locus. In some embodiments, a nucleic acid targeted by a detectably labeled oligonucleotide is or comprises a transcript. In some embodiments, a nucleic acid targeted by a detectably labeled oligonucleotide is a transcript. In some embodiments, a nucleic acid targeted by a detectably labeled oligonucleotide is or comprises a DNA locus. In some embodiments, a nucleic acid targeted by a detectably labeled oligonucleotide is a DNA locus. In some embodiments, each plurality of detectably labelled oligonucleotides used in a contacting step targets the same transcripts and/or DNA locus.

In some embodiments, a plurality of detectably labeled oligonucleotides utilized in a contacting step is referred to as a set of detectably labeled oligonucleotides. In some embodiments, targets of a set of detectably labeled oligonucleotides are referred to as a set of targets. In some embodiments, a target in a set is or comprises a transcript. In some embodiments, a target in a set is a transcript. In some embodiments, each target in a set is or comprises a transcript. In some embodiments, each target in a set is transcript. In some embodiments, a target in a set is or comprises a DNA locus. In some embodiments, a target in a set is a DNA locus. In some embodiments, each target in a set is or comprises a DNA locus. In some embodiments, each target in a set is DNA locus.

In some embodiments, provided methods optionally comprise a step of removing a plurality of detectably labeled oligonucleotides after an imaging step. In some embodiments, provided methods comprises a step of removing a plurality of detectably labeled oligonucleotides after each imaging step. In some embodiments, the step of removing comprises contacting a plurality of detectably labeled oligonucleotides with an enzyme that digests a detectably labeled oligonucleotide. In some embodiments, the step of removing comprises contacting the plurality of detectably labeled oligonucleotides with a DNase. In some embodiments, a step of removing comprises contacting a plurality of detectably labeled oligonucleotides with an RNase. In some embodiments, a step of removing comprises photobleaching.

In some embodiments, each set comprises two or more detectably labeled oligonucleotides targeting the same transcript and/or DNA locus. In some embodiments, two or more detectably labeled oligonucleotides in a set targeting the same transcript and/or DNA locus produce the same detectable signal. In some embodiments, all detectably labeled oligonucleotides in a set targeting the same transcript and/or DNA locus produce the same detectable signal. In some embodiments, wherein the detectably labeled oligonucleotides are labeled with fluorophore, a detectable signal is a certain color. In some embodiments, all detectably labeled oligonucleotides in a set targeting the same transcript and/or DNA locus are labelled with fluorophores providing the same detectable color.

In some embodiments, two or more detectably labeled oligonucleotides in a set targeting the same transcript and/or DNA locus have the same detectable label. In some embodiments, all detectably labeled oligonucleotides in a set targeting the same transcript and/or DNA locus have the same detectable label. In some embodiments, all detectably labeled oligonucleotides targeting the same transcript and/or DNA locus have the same fluorophore.

In some embodiments, the present invention provides compositions useful for conducting provided methods.

In some embodiments, the present invention provides compositions comprising a plurality of detectably labeled oligonucleotides, each of which targets a nucleic acid and is labeled with a detectable moiety, so that the composition comprises at least:
(i) a first oligonucleotide targeting a first nucleic acid and labeled with a first detectable moiety; and
(ii) a second oligonucleotide targeting a second nucleic acid and labeled with a second detectable moiety.

In some embodiments, the present invention provides a kit comprising a plurality of detectably labeled oligonucleotides, each of which targets a nucleic acid and is labeled with a detectable moiety, so that the kit comprises at least:
(i) a first oligonucleotide targeting a first nucleic acid and labeled with a first detectable moiety;
(ii) a second oligonucleotide targeting a second nucleic acid and labeled with a second detectable moiety.
(iii) a third oligonucleotide, optionally identical in sequence to the first oligonucleotide, targeting the first nucleic acid and labeled with the first, the second or a third detectable moiety; and
(iv) a fourth oligonucleotide, optionally identical in sequence to the second oligonucleotide, targeting the nucleic acid, and labeled with the first, the second, the third or a fourth detectable moiety,
wherein either the third oligonucleotide is labeled with a different detectable moiety than the first oligonucleotide, or the fourth oligonucleotide is labeled with a different detectable moiety than the second oligonucleotide, or both.

In some embodiments, a detectable moiety is or comprises a fluorophore.

In some embodiments, a plurality of detectably labeled oligonucleotides target two or more nucleic acids ("targets"). In some embodiments, a target is or comprises a transcript. In some embodiments, a target is a transcript. In some embodiments, a target is an RNA. In some embodiments, a target is mRNA. In some embodiments, a target is tRNA. In some embodiments, a target is rRNA. In some embodiments, a target is a non-coding RNA. In some embodiments, a target is or comprises a DNA locus. In some embodiments, a transcript is a DNA locus. In some embodiments, a target is a locus of a transcript. In some embodiments, different transcripts of a DNA sequence, such as splicing variants of a gene, constitutes different targets, wherein one or more of the variant can be independently targeted and detected or quantified. In some embodiments, the present invention provides methods, compositions or kits to detect individual splicing variants. In some embodiments, the present invention provides methods, compositions, or kits for detecting single nucleotide polymorphisms (SNPs).

In some embodiments, provided methods quantify a target, *e*.*g*., a transcript or a DNA locus.

In some embodiments, oligonucleotides targeting the same target have the same set of sequences, *i*.*e*., when applied at different steps, the differences among the oligonucleotides are within the moieties, not the sequences.

In one aspect, disclosed herein is a composition comprising a plurality of primary probes, a first plurality of bridge probes, and first plurality of readout probes.

In some embodiments, each primary probe in the plurality of primary probes comprises: a primary binding sequence that binds to a complementary target sequence in a target nucleic acid molecule, and a first overhang sequence connected to one end of the primary binding sequence.

In some embodiments, each bridge probe in the first plurality of bridge probes comprises a binding sequence that specifically binds to all or a part of the first overhang sequence of a primary probe of the plurality of primary probes, and one or more readout binding targets connected in series and linked to the binding sequence.

In some embodiments, each readout probe in the first plurality of readout probes comprises: a readout binding sequence that specifically binds to a first readout binding target of the one or more readout binding targets of a bridge probe of the first plurality of bridge probes, and a signal moiety linked to the readout binding sequence via a cleavable linker.

In these embodiments, the signal moiety is capable of emitting a first detectable visual signal upon binding of each readout probe from the first plurality of readout probes to the first readout binding target of one of the one or more readout binding targets.

In some embodiments, the composition further comprises: a second plurality of readout probes, wherein each readout probe comprises: a readout binding sequence that specifically binds to a second readout binding target of the one or more readout binding targets in a bridge probe of the first plurality of bridge probes, and a signal moiety linked to the readout binding sequence via a cleavable linker.

In these embodiments, the signal moiety is capable of emitting a second detectable visual signal upon binding of each readout probe from the second plurality of readout probes to the second readout binding target of the one or more readout binding targets.

In some embodiments, the composition further comprises: a second overhang sequence, linked to the other end of the primary binding sequence.

In some embodiments, the composition further comprises: a second plurality of bridge probes, wherein each bridge probe comprises: a binding sequence that specifically binds to all or a part of the second overhang sequence of a primary probe of the plurality of primary probes, and one or more additional readout binding targets connected in series and linked to the binding sequence.

In some embodiments, the composition further comprises: a third plurality of readout probes, wherein each readout probe comprises: a readout binding sequence that specifically binds to a first additional readout binding target of the one or more additional readout binding targets in a bridge probe of the second plurality of bridge probes, and a signal moiety linked to the readout binding sequence via a cleavable linker.

In these embodiments, the signal moiety is capable of emitting a third detectable visual signal upon binding of each readout probe from the third plurality of readout probes to the first additional readout binding target of the one or more additional readout binding targets.

In some embodiments, the composition further comprises: a fourth plurality of readout probes. Each readout probe in the fourth plurality of readout probes comprises: a readout binding sequence that specifically binds to a second additional readout binding target of the one or more additional readout binding targets in a bridge probe of the second plurality of bridge probes, and a signal moiety linked to the readout binding sequence via a cleavable linker.

In these embodiments, the signal moiety is capable of emitting a fourth detectable visual signal upon binding of each readout probe from the fourth plurality of readout probes to the second additional readout binding target of the one or more additional readout binding targets.

In some embodiments, the cleavable linker is selected from the group consisting of an enzyme cleavable linker, a nucleophile/base sensitive linker, reduction sensitive linker, a photo-cleavable linker, an electrophile/acid sensitive linker, a metal-assisted cleavable linker, and an oxidation sensitive linker.

In some embodiments, the cleavable linker is a disulfide bond or a nucleic acid restriction site. In some embodiments, the one or more readout binding targets comprises three or more readout binding targets.

In some embodiments where second overhang is present, the additional one or more readout binding targets comprises three or more readout binding targets.

In one aspect, disclosed herein is a sequential hybridization method utilizing a plurality of primary probes, a first plurality of bridge probes, and first plurality of readout probes. In some embodiments, the method comprises the steps of: a) contacting a target nucleic acid molecule with a plurality of primary probes, where each primary probe comprises: a primary binding sequence that binds to a complementary target sequence within the target nucleic acid molecule, and a first overhang sequence connected to one end of the primary binding sequence;
b) contacting, after step a) the target nucleic acid molecule with a first plurality of bridge probes, where each bridge probe comprises: a binding sequence that specifically binds to all or a part of the first overhang sequence of a primary probe of the plurality of primary probes, and one or more readout binding targets connected in series and linked to the binding sequence; and c) contacting, after step b) the target nucleic acid molecule with a first plurality of readout probes, wherein each readout probe comprises: a readout binding sequence that specifically binds to a first readout binding target of the one or more readout binding targets of a primary probe of the plurality of primary probes, and a signal moiety linked to the readout binding sequence via a cleavable linker.

In these embodiments, the signal moiety is capable of emitting a first detectable visual signal upon binding of each readout probe from the first plurality of readout probes to the first readout binding target of the one or more readout binding targets of a bridge probe of the first plurality of bridge probes.

In some embodiments, the method further comprises the steps of: c1) imaging the target nucleic acid molecule after step c) so that interactions between the first plurality of readout probes and the first readout binding target of the one or more readout binding targets of a primary bridge probe are detected by the presence of first detectable visual signal; and c2) applying, after step c1) a cleaving agent to cleave the linker, thereby eliminating the signal moiety from each readout probe in the first plurality of readout probes.

In some embodiments, the method further comprises: d) contacting, after step c), the target nucleic acid molecule with a second plurality of readout probes. Each readout probe comprises: a readout binding sequence that specifically binds to a second readout binding target of the one or more readout binding targets of a bridge probe, and a signal moiety linked to the readout binding sequence via a cleavable linker.

In these embodiments, the signal moiety is capable of emitting a second detectable visual signal upon binding of each readout probe from the second plurality of readout probes to the second readout binding target of the one or more readout binding targets of a bridge probe of the first plurality of bridge probes.

In some embodiments, the method further comprises: d1) imaging the target nucleic acid molecule after step d) so that interactions between the second plurality of readout probes and the second readout binding target of the one or more readout binding targets of a bridge probe are detected by the presence of second detectable visual signal; and d2) applying a cleaving agent to cleave the linker, thereby eliminating the signal moiety from each readout probe in the second plurality of readout probes.

In some embodiments, each primary probe in the plurality of primary probes further comprises: a second overhang sequence connected to the other end of the primary binding sequence.

In some embodiments, the method further comprises: e) contacting, after step d), the target nucleic acid molecule with a second plurality of bridge probes. Each bridge probe comprises: a binding sequence that specifically binds to all or a part of the second overhang sequence of a primary probe of the plurality of primary probes, and one or more additional readout binding targets connected in series and linked to the binding sequence.

In some embodiments, the method further comprises: f) contacting, after step e), the target nucleic acid molecule with a third plurality of readout probes. Each readout probe comprises: a readout binding sequence that specifically binds to a first additional readout binding target of the one or more additional readout binding targets of a bridge probe in the second plurality of bridge probes, and a signal moiety linked to the readout binding sequence via a cleavable linker.

In these embodiments, the signal moiety is capable of emitting a third detectable visual signal upon binding of each readout probe from the third plurality of readout probes to the first additional readout binding target of the one or more additional readout binding targets.

In some embodiments, the method further comprises: f1) imaging the target nucleic acid molecule after step f) so that interactions between the third plurality of readout probes and the first additional readout binding target of the one or more additional readout binding targets of a bridge probe in the second plurality of bridge probes are detected by the presence of the third detectable visual signal; and f2) applying a cleaving agent to cleave the linker, thereby eliminating the signal moiety from each readout probe in the third plurality of readout probes.

In some embodiments, the method further comprises: g) contacting, after step f), the target nucleic acid molecule with a fourth plurality of readout probes. Each readout probe comprises: a readout binding sequence that specifically binds to a second additional readout binding target of the one or more additional readout binding targets of a bridge probe in the second plurality of bridge probes, and a signal moiety linked to the readout binding sequence via a cleavable linker.

In these embodiments, the signal moiety is capable of emitting a fourth detectable visual signal upon binding of each readout probe from the fourth plurality of readout probes to the second additional readout binding target of the one or more additional readout binding targets.

In some embodiments, the method further comprises: h1) imaging the target nucleic acid molecule after step g) so that interactions between the fourth plurality of readout probes and the second additional readout binding target of the one or more additional readout binding targets of a bridge probe in the second plurality of bridge probes are detected by the presence of the fourth detectable visual signal; and h2) applying a cleaving agent to cleave the linker, thereby eliminating the signal moiety from each readout probe in the fourth plurality of readout probes.

In some embodiments, the target nucleic acid molecule is an mRNA or a DNA. In some embodiments, the target nucleic acid molecule is within an intact mammalian cell. In some embodiments, the intact mammalian cell is a human cell.

In these embodiments, the cleavable linker is selected from the group consisting of an enzyme cleavable linker, a nucleophile/base sensitive linker, reduction sensitive linker, a photo-cleavable linker, an electrophile/acid sensitive linker, a metal-assisted cleavable linker, and an oxidation sensitive linker. In these embodiments, the cleavable linker is a disulfide bond or a nucleic acid restriction site. In these embodiments, the one or more readout binding targets comprises three or more readout binding targets.

In these embodiments where a second overhang is present, the additional one or more readout binding targets comprises three or more readout binding targets.

In one aspect, disclosed herein is a composition that comprises a plurality of primary probes and a first plurality of readout probes. In these embodiments, each primary probe comprises: a primary binding sequence that binds to a complementary target sequence in a target nucleic acid molecule, and a first overhang sequence connected to one end of the primary binding sequence, wherein the first overhang sequence comprises one or more readout binding targets connected in series. Also in these embodiments, each readout probe comprises: a readout binding sequence that specifically binds to a first readout binding target of the one or more readout binding targets in a first overhang sequence, and a signal moiety linked to the readout binding sequence via a cleavable linker. In these embodiments, the signal moiety is capable of emitting a first detectable visual signal upon binding of each readout probe from the first plurality of readout probes to the first readout binding target of one of the one or more readout binding targets.

In some embodiments, the composition further comprises: a second plurality of readout probes, where each readout probe comprises: a readout binding sequence that specifically binds to a second readout binding target of the one or more readout binding targets in a first overhang sequence, and a signal moiety linked to the readout binding sequence via a cleavable linker. In these embodiments, the signal moiety is capable of emitting a second detectable visual signal upon binding of each readout probe from the second plurality of readout probes to the second readout binding target of the one or more readout binding targets.

In some embodiments, a primary probe further comprises: a second overhang sequence, linked to the other end of the primary binding sequence, where the second overhang sequence comprises one or more additional readout binding targets connected in series.

In some embodiments, the composition further comprises a third plurality of readout probes, where each readout probe comprises: a readout binding sequence that specifically binds to a first additional readout binding target of the one or more additional readout binding targets in a second overhang sequence, and a signal moiety linked to the readout binding sequence via a cleavable linker. In these embodiments, the signal moiety is capable of emitting a third detectable visual signal upon binding of each readout probe from the third plurality of readout probes to the first additional readout binding target of the one or more additional readout binding targets.

In some embodiments, the composition further comprises a fourth plurality of readout probes, where each readout probe comprises: a readout binding sequence that specifically binds to a second additional readout binding target of the one or more additional readout binding targets in a second overhang sequence, and a signal moiety linked to the readout binding sequence via a cleavable linker. In these embodiments, the signal moiety is capable of emitting a fourth detectable visual signal upon binding of each readout probe from the fourth plurality of readout probes to the second additional readout binding target of the one or more additional readout binding targets.

In any embodiments disclosed herein, the cleavable linker is selected from the group consisting of an enzyme cleavable linker, a nucleophile/base sensitive linker, reduction sensitive linker, a photo-cleavable linker, an electrophile/acid sensitive linker, a metal-assisted cleavable linker, and an oxidation sensitive linker.

In any embodiments disclosed herein, the cleavable linker is a disulfide bond or a nucleic acid restriction site.

In any embodiments disclosed herein, the one or more readout binding targets comprises three or more readout binding targets.

In embodiments where a second overhang sequence is present, the additional one or more readout binding targets comprises three or more readout binding targets.

In some embodiments, the target nucleic acid molecule is an mRNA or a DNA. In some embodiments, the target nucleic acid molecule is within an intact mammalian cell. In some embodiments, the intact mammalian cell is a human cell.

In one aspect, disclosed herein is a sequential hybridization method utilizing with a plurality of primary probes and a first plurality of readout probes. The method comprises the steps of: a) contacting a target nucleic acid molecule with a plurality of primary probes. Each primary probe comprises: a primary binding sequence that binds to a complementary target sequence within the target nucleic acid molecule, and a first overhang sequence connected to one end of the primary binding sequence, wherein the first overhang sequence comprises one or more readout binding targets connected in series; and b) contacting, after step a) the target nucleic acid molecule with a first plurality of readout probes. Each readout probe comprises: a readout binding sequence that specifically binds to a first readout binding target of the one or more readout binding targets of a primary probe of the plurality of primary probes, and a signal moiety linked to the readout binding sequence via a cleavable linker.

In these embodiments, the signal moiety is capable of emitting a first detectable visual signal upon binding of each readout probe from the first plurality of readout probes to the first readout binding target of one of the one or more readout binding targets.

In some embodiments, the method further comprises the steps of: b1) imaging the target nucleic acid molecule after step b) so that interactions between the first plurality of readout probes and the first readout binding target of the one or more readout binding targets of a primary bridge probe are detected by the presence of the first detectable visual signal; and b2) applying a cleaving agent to cleave the linker, thereby eliminating the signal moiety from each readout probe in the first plurality of readout probes.

In some embodiments, the method further comprises the steps of: c) contacting, after step b), the target nucleic acid molecule with a second plurality of readout probes. Each readout probe comprises: a readout binding sequence that specifically binds to a second readout binding target of the one or more readout binding targets of a primary probe, and a signal moiety linked to the readout binding sequence via a cleavable linker.

In these embodiments, the signal moiety is capable of emitting a second detectable visual signal upon binding of each readout probe from the second plurality of readout probes to the second readout binding target of the one or more readout binding targets.

In some embodiments, the method further comprises the steps of: c1) imaging the target nucleic acid molecule after step c) so that interactions between the second plurality of readout probes and the second readout binding target of the one or more readout binding targets of a primary probe are detected by the presence of the second detectable visual signal; and c2) applying a cleaving agent to cleave the linker, thereby eliminating the signal moiety from each readout probe in the second plurality of readout probes.

In some embodiments, each primary probe in the plurality of primary probes further comprises: a second overhang sequence connected to the other end of the primary binding sequence, wherein the second overhang sequence comprises one or more additional readout binding targets connected in series.

In some embodiments, the method further comprises the steps of: d) contacting, after step c), the target nucleic acid molecule with a third plurality of readout probes. Each readout probe comprises: a readout binding sequence that specifically binds to a first additional readout binding target of the one or more additional readout binding targets of a primary probe, and a signal moiety linked to the readout binding sequence via a cleavable linker.

In these embodiments, the signal moiety is capable of emitting a third detectable visual signal upon binding of each readout probe from the third plurality of readout probes to the first additional readout binding target of the one or more additional readout binding targets.

In some embodiments, the method further comprises the steps of: d1) imaging the target nucleic acid molecule after step d) so that interactions between the second plurality of readout probes and the second readout binding target of the one or more readout binding targets of a primary probe are detected by the presence of the second detectable visual signal; and d2) applying a cleaving agent to cleave the linker, thereby eliminating the signal moiety from each readout probe in the second plurality of readout probes.

In some embodiments, the method further comprises the steps of: e) contacting, after step d), the target nucleic acid molecule with a fourth plurality of readout probes. Each readout probe comprises: a readout binding sequence that specifically binds to a second additional readout binding target of the one or more additional readout binding targets of a primary probe, and a signal moiety linked to the readout binding sequence via a cleavable linker, In these embodiments, the signal moiety is capable of emitting a fourth detectable visual signal upon binding of each readout probe from the fourth plurality of readout probes to the second additional readout binding target of the one or more additional readout binding targets.

In some embodiments, the method further comprises the steps of: e1) imaging the mRNA after step d) so that interactions between the fourth plurality of readout probes and the second additional readout binding target of the one or more additional readout binding targets of a primary probe are detected by the presence of the fourth detectable visual signal; and e2) applying a cleaving agent to cleave the linker, thereby eliminating the signal moiety from each readout probe in the fourth plurality of readout probes.

In some embodiments, the target nucleic acid molecule is an mRNA or a DNA. In some embodiments, the target nucleic acid molecule is within an intact mammalian cell. In some embodiments, the intact mammalian cell is a human cell.

In some embodiments, the cleavable linker is selected from the group consisting of an enzyme cleavable linker, a nucleophile/base sensitive linker, reduction sensitive linker, a photo-cleavable linker, an electrophile/acid sensitive linker, a metal-assisted cleavable linker, and an oxidation sensitive linker. In some embodiments, the cleavable linker is a disulfide bond or a nucleic acid restriction site.

In some embodiments, the one or more readout binding targets comprises three or more readout binding targets.

In some embodiments where the second overhang sequence is present, the additional one or more readout binding targets comprises three or more readout binding targets.

In one aspect, disclosed herein is a composition comprising a first plurality of nucleic acid detection probes and an extendible signal motif formed by a first plurality populations of extender probes {EP₁, EP₂, ..., EPₙ}. In some embodiments, each nucleic acid detection probe in the first plurality of nucleic acid detection probes comprises: a binding region comprising a binding sequence that binds to a first target sequence; and an initiator sequence linked to the binding region with a cleavable linker. In some embodiments, each population of extender probes is represented by EP₁, EP₂, ..., EPₙ, respectively, where each extender probe in EP₁ comprises: a binding sequence that binds to all or a part of the initiator sequence; one or more target sequences for extender probes in EP₂ and subsequent populations of extender probes, and a signal moiety capable of emitting a first detectable signal. In some embodiments, each probe in EP₂ and subsequent populations of extender probes comprises: a binding sequence that binds to all or a part of the previous extender sequence; one or more target sequences for probes in subsequent populations of extender probes; and a signal moiety capable of emitting the first detectable signal.

In some embodiments, the first target sequence is within a primary probe that directly binds to a target nucleic acid molecule. In some embodiments, the first target sequence is within a secondary probe that binds to a primary probe that directly binds to a target nucleic acid molecule. In some embodiments, the first target sequence is within a tertiary probe that binds to a secondary probe that binds to a primary probe that directly binds to a target nucleic acid molecule.

In some embodiments, the target nucleic acid molecule is an mRNA or a DNA. In some embodiments, the target nucleic acid molecule is within an intact mammalian cell. In some embodiments, the intact mammalian cell is a human cell.

In some embodiments, the cleavable linker is selected from the group consisting of an enzyme cleavable linker, a nucleophile/base sensitive linker, reduction sensitive linker, a photo-cleavable linker, an electrophile/acid sensitive linker, a metal-assisted cleavable linker, and an oxidation sensitive linker. In some embodiments, the cleavable linker is a disulfide bond or a nucleic acid restriction site.

In some embodiments, each extender probe of the plurality of extender probes comprises a binding sequence that is complementary to all or a part of the initiator sequence in the nucleic acid detection probe, wherein each extender probe forms a hairpin structure, and wherein the presence of the initiator sequence causes the hairpin structure to unfold and initiates a hybridization chain reaction.

In some embodiments, the composition further comprises a second plurality of nucleic acid detection probes and an extendible signal motif formed by a second plurality populations of extender probes {EP_{1'}, EP_{2'}, ..., EP_{n'}}. In some embodiments, each nucleic acid detection probe in the second plurality of nucleic acid detection probes comprises: a binding region comprising a binding sequence that binds to a second target sequence; and an initiator sequence linked to the binding region with a cleavable linker. In some embodiments, each population of extender probes is represented by EP_{1'}, EP_{2'}, ..., EP_{n'}, respectively, wherein each extender probe in EP_{1'} comprises: a binding sequence that binds to all or a part of the initiator sequence; one or more target sequences for extender probes in EP_{2'} and subsequent populations of extender probes; and a signal moiety capable of emitting a second detectable signal. In some embodiments, each probe in EP_{2'} and subsequent populations of extender probes comprises: a binding sequence that binds to all or a part of the previous extender sequence; one or more target sequences for probes in subsequent populations of extender probes; and a signal moiety capable of emitting the second detectable signal.

In one aspect, disclosed herein is a sequential hybridization method. The method comprises the steps of: a) contacting a target nucleic acid molecule with a first plurality of nucleic acid detection probes and b) contacting, after step a) the target nucleic acid molecule with a first plurality populations of extender probes {EP₁, EP₂, ..., EPₙ}. In some embodiments, each nucleic acid detection probe in the first plurality of nucleic acid detection probes comprises: a binding region comprising a binding sequence that binds to a first target sequence; and an initiator sequence linked to the binding region with a cleavable linker. In some embodiments, each population of extender probes is represented by EP₁, EP₂, ..., EPₙ, respectively, where each extender probe in EP₁ comprises: a binding sequence that binds to all or a part of the initiator sequence; one or more target sequences for extender probes in EP₂ and subsequent populations of extender probes; and a signal moiety capable of emitting a first detectable signal. In some embodiments, each probe in EP₂ and subsequent populations of extender probes comprises: a binding sequence that binds to all or a part of the previous extender sequence; one or more target sequences for probes in subsequent populations of extender probes; and a signal moiety capable of emitting the first detectable signal.

In some embodiments, the method further comprises: b1) imaging the target nucleic acid molecule after step b) so that interactions between the first plurality of nucleic acid detection probes and first target sequences are detected by the presence of the first detectable visual signal; and b2) applying a cleaving agent to cleave the linker, thereby eliminating the extendible signal motif.

In some embodiments, the method further comprises: c) contacting an target nucleic acid molecule with a second plurality of nucleic acid detection probes. In some embodiment, each nucleic acid detection probe in the second plurality of nucleic acid detection probes comprises: a binding region comprising a binding sequence that binds to a second target sequence; and an initiator sequence linked to the binding region with a cleavable linker.

In some embodiments, the method further comprises: d) contacting, after step c) the target nucleic acid molecule with a second plurality populations of extender probes {EP_{1'}, EP_{2'}, ..., EP_{n'}}, where each population of extender probes is represented by EP_{1'}, EP_{2'}, ..., and EP_{n'}, respectively. In some embodiments, each extender probe in EP_{1'} comprises: a binding sequence that binds to all or a part of the initiator sequence; one or more target sequences for extender probes in EP₂, and subsequent populations of extender probes; and a signal moiety capable of emitting a second detectable signal. In some embodiments, each probe in EP₂, and subsequent populations of extender probes comprises: a binding sequence that binds to all or a part of the previous extender sequence; one or more target sequences for probes in subsequent populations of extender probes; and a signal moiety capable of emitting the second detectable signal.

In some embodiments, the method further comprises: d1) imaging the target nucleic acid molecule after step d) so that interactions between the second plurality of nucleic acid detection probes and second target sequences are detected by the presence of the second detectable visual signal; and d2) applying a cleaving agent to cleave the linker, thereby eliminating the extendible signal motif.

In some embodiments, the second target sequence is within a primary probe that directly binds to a target nucleic acid molecule. In some embodiments, the second target sequence is within a secondary probe that binds to a primary probe that directly binds to a target nucleic acid molecule. In some embodiments, the second target sequence is within a tertiary probe that binds to a secondary probe that binds to a primary probe that directly binds to a target nucleic acid molecule.

Also provided is a sequential hybridization method including the steps of:
a) contacting a target molecule with a plurality of primary antibodies, wherein each primary antibody contains one or more binding targets connected in series and linked to the primary antibody;
b) contacting the target molecule with a first plurality of readout probes, wherein each readout probe includes a signal moiety, and wherein each readout probe interacts with a first binding target of the one or more binding targets of a primary antibody of the plurality of primary antibodies,
   wherein the signal moiety is capable of emitting a first detectable visual signal upon the interaction of each readout probe from the first plurality of readout probes to the first binding target of a primary antibody of the plurality of primary antibodies;
c) imaging the target molecule after step b) so that the interactions between the first plurality of readout probes and the plurality of primary antibodies are detected by the presence of the first detectable visual signal;
d) contacting the target molecule, the plurality of primary antibodies and the first plurality of readout probes with a solution containing a denaturing agent, wherein contact of the solution with the target molecule, the plurality of primary antibodies, and the first plurality of readout probes does not disrupt the interaction between the plurality of primary antibodies and the target molecule;
e) contacting the target molecule and the plurality of primary antibodies with a second plurality of readout probes, wherein each readout probe comprises a signal moiety, and wherein each readout probe interacts with a second binding target of a primary antibody of the plurality of primary antibodies,
   wherein the signal moiety is capable of emitting a second detectable visual signal upon the interaction of each readout probe with the second binding target of a primary antibody of the plurality of primary antibodies; and
f) imaging the target nucleic acid molecule after step e) so that interactions between the second plurality of readout probes and the plurality of primary antibodies are detected by the presence of the second detectable visual signal.

In some embodiments, the method further includes the steps of:
g) contacting the target molecule, the plurality of primary antibodies, and the second plurality of readout probes with a solution comprising a denaturing agent, wherein contact of the solution with the target molecule, the plurality of primary antibodies, and the second plurality of readout probes does not disrupt the interaction between the plurality of primary antibodies and the target molecule;
h) contacting the target molecule and the plurality of primary antibodies with a third plurality of readout probes, wherein each readout probe comprises a signal moiety, and wherein each readout probe interacts with a third binding target of a primary antibody of the plurality of primary antibodies,
wherein the signal moiety is capable of emitting a third detectable visual signal upon the interaction of each readout probe from the third plurality of readout probes with the third binding target of a primary antibody of the plurality of primary antibodies; and
i) imaging the target nucleic acid molecule after step h) so that interactions between the third plurality of readout probes and the plurality of primary antibodies are detected by the presence of the third detectable visual signal.

In some embodiments, each readout probe in any plurality of readout probes interacts with its binding target by hybridizing to its binding target in a primary antibody of the plurality of primary antibodies. In some embodiments, each readout probe in any plurality of readout probes interacts with its binding target by hybridizing to a bridge probe that comprises: (i) a sequence that is complementary to the one or more binding targets of a primary antibody of the plurality of primary antibodies, and (ii) a sequence to which the readout probe binds.

In some embodiments, the target molecule is an RNA, a DNA, or a protein.

In some embodiments, the target molecule is within an intact cell. The intact cell can be a prokaryotic cell, a eukaryotic cell, a mammalian cell, or a human cell.

Also provided is a sequential hybridization method including the steps of:
a) contacting a target nucleic acid molecule with a plurality of primary probes, wherein each primary probe comprises: (i) a primary binding sequence that binds to a complementary target sequence within the target nucleic acid molecule, and (ii) a first overhang sequence connected to one end of the primary binding sequence comprising one or more binding targets connected in series and linked to the primary binding sequence;
b) contacting the target nucleic acid molecule with a first plurality of readout probes, wherein each readout probe comprises a signal moiety, and wherein each readout probe interacts with a first binding target of the one or more binding targets of a primary probe of the plurality of primary probes,
   wherein the signal moiety is capable of emitting a first detectable visual signal upon the interaction of each readout probe from the first plurality of readout probes with the first binding target of the one or more binding targets of a primary probe of the plurality of primary probes;
c) imaging the target nucleic acid molecule after step b) so that the interactions between the first plurality of readout probes and the plurality of primary probes are detected by the presence of a first detectable visual signal;
d) contacting the target nucleic acid molecule, the plurality of primary probes, and the first plurality of readout probes with a solution comprising a denaturing agent, wherein contact of the solution with the target nucleic acid molecule, the plurality of primary probes, and the first plurality of readout probes does not disrupt the interaction between the plurality of primary probes and the target nucleic acid molecule;
e) contacting the target nucleic acid molecule with a second plurality of readout probes, wherein each readout probe comprises a signal moiety, and wherein each readout probe interacts with a second binding target of the one or more binding targets of a primary probe,
   wherein the signal moiety is capable of emitting a second detectable visual signal upon the interaction of each readout probe from the second plurality of readout probes with the second binding target of the one or more binding targets of a primary probe of the plurality of primary probes; and
f) imaging the target nucleic acid molecule after step e) so that the interactions between the second plurality of readout probes and the plurality of primary probes are detected by the presence of the second detectable visual signal.

In some embodiments, the method further includes the steps of:
g) contacting the target nucleic acid molecule, the plurality of primary bridge probes, and the second plurality of readout probes with the solution containing a denaturing agent, wherein contact of the solution with the target nucleic acid molecule, the plurality of primary probes, and the second plurality of readout probes does not disrupt the interaction between the plurality of primary probes and the target nucleic acid molecule;
h) contacting the target nucleic acid molecule with a third plurality of readout probes, wherein each readout probe includes a signal moiety, and wherein each readout probe interacts with a third binding target of the one or more binding targets of a primary probe,
wherein the signal moiety is capable of emitting a third detectable visual signal upon the interaction of each readout probe from the third plurality of readout probes with the third binding target of the one or more binding targets of a primary probe of the plurality of primary probes; and
i) imaging the target nucleic acid molecule after step h) so that the interactions between the third plurality of readout probes and the plurality of primary probes are detected by the presence of the third detectable visual signal.

In some embodiments, each primary probe in the plurality of primary probes further includes: a second overhang sequence connected to the other end of the primary binding sequence containing one or more additional binding targets connected in series and linked to the primary binding sequence.

In some embodiments, the method further includes the steps of:
c1) contacting, after step c), the target nucleic acid molecule with a fourth plurality of readout probes, wherein each readout probe comprises a signal moiety, and wherein each readout probe interacts with a first additional binding target of the second overhang sequence of a primary probe in the plurality of primary probes; and
c2) imaging the target nucleic acid molecule after step c1) so that interactions between the fourth plurality of readout probes and the second overhang sequence of a primary probe in the plurality of primary probes are detected by the presence of the fourth detectable visual signal;
wherein steps c1) to c2) take place prior to step d) of the method.

In some embodiments, the method further includes the steps of:
e1) contacting, after step e), the target nucleic acid molecule with a fifth plurality of readout probes, wherein each readout probe comprises a signal moiety, and wherein each readout probe interacts with a second additional binding target of the second overhang sequence of a primary probe in the plurality of primary probes; and
e2) imaging the target nucleic acid molecule after step e1) so that interactions between the fifth plurality of readout probes and the second overhang sequence of a primary probe in the plurality of primary probes are detected by the presence of the fifth detectable visual signal.

In some examples of the foregoing methods, each readout probe in any plurality of readout probes interacts with its binding target by hybridizing to its binding target in a primary probe of the plurality of primary probes. In some examples of the foregoing methods, each readout probe in any plurality of readout probes interacts with its binding target by hybridizing to a bridge probe that comprises: (i) a sequence that is complementary to all or part of the first overhang sequence of a primary probe of the plurality of primary probes, and (ii) a sequence to which the readout probe binds.
one or more additional binding targets of the second overhang sequence interact with a readout probe from a fourth plurality of readout probes or with a readout probe form a fifith plurality of readout probes. In some embodiments, the interaction between from the readout probe from a fourth plurality of readout probes or a fifth plurality of readout probes

In some embodiments, the target nucleic acid molecule is an RNA or a DNA molecule.

In some embodiments, the target nucleic acid molecule is within an intact cell. The intact cell can be a prokaryotic cell, a eukaryotic cell, a mammalian cell, or a human cell.

In some embodiments, the one or more binding targets comprises three or more binding targets. In some embodiments, the additional one or more binding targets comprises three or more readout binding targets.

In some embodiments, the denaturing agent is formamide.

In some embodiments, the readout probes are less than 17 nucleotides in length. In some embodiments, the readout probes are between 10 and 17 nucleotides in length. In some embodiments, the readout probes are less than 10 nucleotides in length. In some embodiments, the readout probes are between 5 and 10 nucleotides in length.

In some embodiments, the target nucleic acid molecule is an mRNA or a DNA. In some embodiments, the target nucleic acid molecule is within an intact mammalian cell. In some embodiments, the intact mammalian cell is a human cell.

In some embodiments, the cleavable linker is selected from the group consisting of an enzyme cleavable linker, a nucleophile/base sensitive linker, reduction sensitive linker, a photo-cleavable linker, an electrophile/acid sensitive linker, a metal-assisted cleavable linker, and an oxidation sensitive linker. In some embodiments, the cleavable linker is a disulfide bond or a nucleic acid restriction site.

In some embodiments, each extender probe of the plurality of extender probes comprises a binding sequence that is complementary to all or a part of the initiator sequence in the nucleic acid detection probe, where each extender probe forms a hairpin structure, and where the presence of the initiator sequence causes the hairpin structure to unfold and initiates a hybridization chain reaction.

The compositions and methods disclosed herein can be used in sequential hybridizations to identify any suitable cellular targets within an intact cell or in an *in vitro* setting. In some embodiments, the cellular targets can be mRNAs or DNAs. In some embodiments, the cellular targets can be proteins. For example, the initial target-binding primary probe can be an antibody conjugated with nucleic acid sequence for subsequent bindings.

One of skill in the art would understand that embodiments disclosed herein can be applied or combined in any aspect when applicable.

### DEFINITIONS

*Animal:* As used herein, the term "animal" refers to any member of the animal kingdom. In some embodiments, "animal" refers to humans, at any stage of development. In some embodiments, "animal" refers to non-human animals, at any stage of development. In certain embodiments, the non-human animal is a mammal (*e*.*g*., a rodent, a mouse, a rat, a rabbit, a monkey, a dog, a cat, a sheep, cattle, a primate, and/or a pig). In some embodiments, animals include, but are not limited to, mammals, birds, reptiles, amphibians, fish, and/or worms. In some embodiments, an animal may be a transgenic animal, a genetically-engineered animal, and/or a clone.

*Approximately:* As used herein, the terms "approximately" or "about" in reference to a number are generally taken to include numbers that fall within a range of 5%, 10%, 15%, or 20% in either direction (greater than or less than) of the number unless otherwise stated or otherwise evident from the context (except where such number would be less than 0% or exceed 100% of a possible value). In some embodiments, use of the term "about" in reference to dosages means ± 5 mg/kg/day.

*Homology:* "Homology" or "identity" or "similarity" refers to sequence similarity between two nucleic acid molecules. Homology and identity can each be determined by comparing a position in each sequence which can be aligned for purposes of comparison. When an equivalent position in the compared sequences is occupied by the same base, then the molecules are identical at that position; when the equivalent site occupied by the same or a similar nucleic acid residue (e.g., similar in steric and/or electronic nature), then the molecules can be referred to as homologous (similar) at that position. Expression as a percentage of homology/similarity or identity refers to a function of the number of identical or similar nucleic acids at positions shared by the compared sequences. A sequence which is "unrelated" or "non- homologous" shares less than 40% identity, less than 35% identity, less than 30% identity, or less than 25% identity with a sequence described herein. In comparing two sequences, the absence of residues (amino acids or nucleic acids) or presence of extra residues also decreases the identity and homology/similarity.

In some embodiments, the term "homology" describes a mathematically based comparison of sequence similarities which is used to identify genes with similar functions or motifs. The nucleic acid sequences described herein can be used as a "query sequence" to perform a search against public databases, for example, to identify other family members, related sequences or homologs. In some embodiments, such searches can be performed using the NBLAST and XBLAST programs (version 2.0) of Altschul, et al. (1990) J. Mol. Biol. 215:403-410. In some embodiments, BLAST nucleotide searches can be performed with the NBLAST program, score=100, word length =12 to obtain nucleotide sequences homologous to nucleic acid molecules of the invention. In some embodiments, to obtain gapped alignments for comparison purposes, Gapped BLAST can be utilized as described in Altschul et al., (1997) Nucleic Acids Res. 25(17):3389-3402. When utilizing BLAST and Gapped BLAST programs, the default parameters of the respective programs (e.g., XBLAST and BLAST) can be used (See www.ncbi.nlm.nih.gov).

*Identity:* As used herein, "identity" means the percentage of identical nucleotide residues at corresponding positions in two or more sequences when the sequences are aligned to maximize sequence matching, i.e., taking into account gaps and insertions. Identity can be readily calculated by known methods, including but not limited to those described in (Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; and Carillo, H., and Lipman, D., SIAM J. Applied Math., 48: 1073 (1988). Methods to determine identity are designed to give the largest match between the sequences tested. Moreover, methods to determine identity are codified in publicly available computer programs. Computer program methods to determine identity between two sequences include, but are not limited to, the GCG program package (Devereux, J., et al., Nucleic Acids Research 12(1): 387 (1984)), BLASTP, BLASTN, and FASTA (Altschul, S. F. et al., J. Molec. Biol. 215: 403-410 (1990) and Altschul et al. Nuc. Acids Res. 25: 3389-3402 (1997)). The BLAST X program is publicly available from NCBI and other sources (BLAST Manual, Altschul, S., et al., NCBI NLM NIH Bethesda, Md. 20894; Altschul, S., et al., J. Mol. Biol. 215: 403-410 (1990). The well-known Smith Waterman algorithm can also be used to determine identity.

*In vitro:* As used herein, the term "*in vitro*" refers to events that occur in an artificial environment, *e.g.,* in a test tube or reaction vessel, in cell culture, *etc*., rather than within an organism (*e*.*g*., animal, plant, and/or microbe).

*In vivo*: As used herein, the term "*in vivo*" refers to events that occur within an organism (*e*.*g*., animal, plant, and/or microbe).

*Oligonucleotide:* the term "oligonucleotide" refers to a polymer or oligomer of nucleotide monomers, containing any combination of nucleobases, modified nucleobases, sugars, modified sugars, phosphate bridges, or modified bridges. Oligonucleotides as disclosed herein can be of various lengths. In particular embodiments, oligonucleotides can range from about 2 to about 200 nucleotides in length. In various related embodiments, oligonucleotides, single-stranded, doublestranded, and triple-stranded, can range in length from about 4 to about 10 nucleotides, from about 10 to about 50 nucleotides, from about 20 to about 50 nucleotides, from about 15 to about 30 nucleotides, from about 20 to about 30 nucleotides in length. In some embodiments, the oligonucleotide is from about 9 to about 39 nucleotides in length. In some embodiments, the oligonucleotide is at least 4 nucleotides in length. In some embodiments, the oligonucleotide is at least 5 nucleotides in length. In some embodiments, the oligonucleotide is at least 6 nucleotides in length. In some embodiments, the oligonucleotide is at least 7 nucleotides in length. In some embodiments, the oligonucleotide is at least 8 nucleotides in length. In some embodiments, the oligonucleotide is at least 9 nucleotides in length. In some embodiments, the oligonucleotide is at least 10 nucleotides in length. In some embodiments, the oligonucleotide is at least 11 nucleotides in length. In some embodiments, the oligonucleotide is at least 12 nucleotides in length. In some embodiments, the oligonucleotide is at least 15 nucleotides in length. In some embodiments, the oligonucleotide is at least 20 nucleotides in length. In some embodiments, the oligonucleotide is at least 25 nucleotides in length. In some embodiments, the oligonucleotide is at least 30 nucleotides in length. In some embodiments, the oligonucleotide is a duplex of complementary strands of at least 18 nucleotides in length. In some embodiments, the oligonucleotide is a duplex of complementary strands of at least 21 nucleotides in length.

*Predetermined:* By predetermined is meant deliberately selected, for example as opposed to randomly occurring or achieved. A composition that may contain certain individual oligonucleotides because they happen to have been generated through a process that cannot be controlled to intentionally generate the particular oligonucleotides is not a "predetermined" composition. In some embodiments, a predetermined composition is one that can be intentionally reproduced (e.g., through repetition of a controlled process).

*Sample:* As used herein, the term "sample" refers to a biological sample obtained or derived from a source of interest, as described herein. In some embodiments, a source of interest comprises an organism, such as an animal or human. In some embodiments, a biological sample comprises biological tissue or fluid. In some embodiments, a biological sample is or comprises bone marrow; blood; blood cells; ascites; tissue or fine needle biopsy samples; cell- containing body fluids; free floating nucleic acids; sputum; saliva; urine; cerebrospinal fluid, peritoneal fluid; pleural fluid; feces; lymph; gynecological fluids; skin swabs; vaginal swabs; oral swabs; nasal swabs; washings or lavages such as a ductal lavages or broncheoalveolar lavages; aspirates; scrapings; bone marrow specimens; tissue biopsy specimens; surgical specimens; feces, other body fluids, secretions, and/or excretions; and/or cells therefrom, *etc.* In some embodiments, a biological sample is or comprises cells obtained from an individual. In some embodiments, a sample is a "primary sample" obtained directly from a source of interest by any appropriate means. For example, in some embodiments, a primary biological sample is obtained by methods selected from the group consisting of biopsy (*e*.*g*., fine needle aspiration or tissue biopsy), surgery, collection of body fluid (*e*.*g*., blood, lymph, feces *etc*.), *etc.* In some embodiments, as will be clear from context, the term "sample" refers to a preparation that is obtained by processing (e.g., by removing one or more components of and/or by adding one or more agents to) a primary sample. For example, filtering using a semi-permeable membrane. Such a "processed sample" may comprise, for example nucleic acids or proteins extracted from a sample or obtained by subjecting a primary sample to techniques such as amplification or reverse transcription of mRNA, isolation and/or purification of certain components, etc.

*Subject:* As used herein, the term "subject" or "test subject" refers to any organism to which a provided compound or composition is administered in accordance with the methods disclosed herein, *e*.*g*., for experimental, diagnostic, prophylactic, and/or therapeutic purposes. Typical subjects include animals (*e*.*g*., mammals such as mice, rats, rabbits, non-human primates, and humans; insects; worms; *etc*.) and plants. In some embodiments, a subject may be suffering from, and/or susceptible to a disease, disorder, and/or condition.

*Substantially:* As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and/or chemical phenomena.

*Suffering from*: An individual who is "suffering from" a disease, disorder, and/or condition has been diagnosed with and/or displays one or more symptoms of a disease, disorder, and/or condition.

*Susceptible to*: An individual who is "susceptible to" a disease, disorder, and/or condition is one who has a higher risk of developing the disease, disorder, and/or condition than does a member of the general public. In some embodiments, an individual who is susceptible to a disease, disorder and/or condition may not have been diagnosed with the disease, disorder, and/or condition. In some embodiments, an individual who is susceptible to a disease, disorder, and/or condition may exhibit symptoms of the disease, disorder, and/or condition. In some embodiments, an individual who is susceptible to a disease, disorder, and/or condition may not exhibit symptoms of the disease, disorder, and/or condition. In some embodiments, an individual who is susceptible to a disease, disorder, and/or condition will develop the disease, disorder, and/or condition. In some embodiments, an individual who is susceptible to a disease, disorder, and/or condition will not develop the disease, disorder, and/or condition.

*Treat:* As used herein, the term "treat," "treatment," or "treating" refers to any method used to partially or completely alleviate, ameliorate, relieve, inhibit, prevent, delay onset of, reduce severity of, and/or reduce incidence of one or more symptoms or features of a disease, disorder, and/or condition. Treatment may be administered to a subject who does not exhibit signs of a disease, disorder, and/or condition. In some embodiments, treatment may be administered to a subject who exhibits only early signs of the disease, disorder, and/or condition, for example for the purpose of decreasing the risk of developing pathology associated with the disease, disorder, and/or condition.

*Wild-type:* As used herein, the term "wild-type" has its art-understood meaning that refers to an entity having a structure and/or activity as found in nature in a "normal" (as contrasted with mutant, diseased, altered, etc.) state or context. Those of ordinary skill in the art will appreciate that wild type genes and polypeptides often exist in multiple different forms (e.g., alleles).

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

Those of skill in the art will understand that the drawings, described below, are for illustrative purposes only. The drawings are not intended to limit the scope of the present teachings in any way.
**FIG. 1** represents a schematic of methodologies provided by the present disclosure.

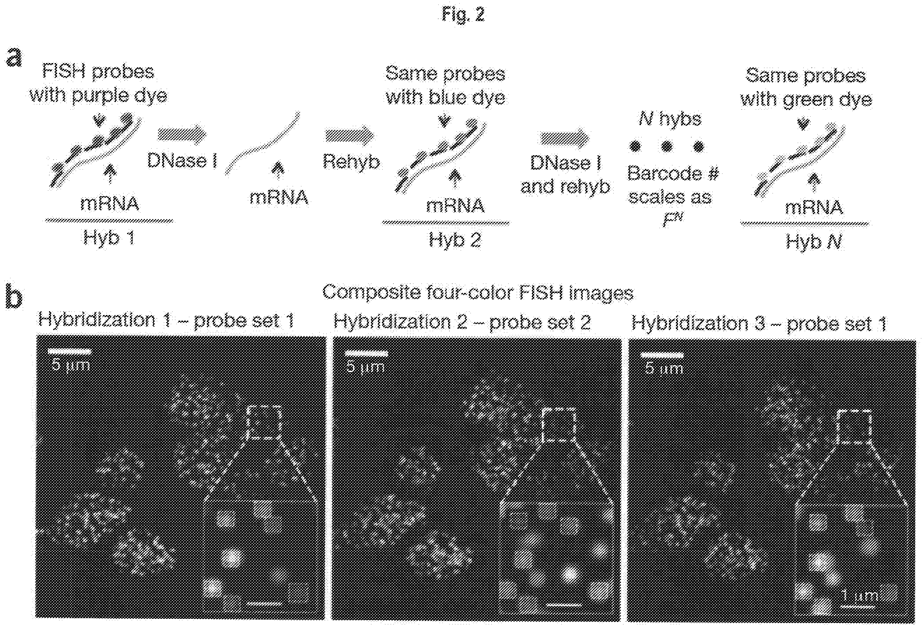

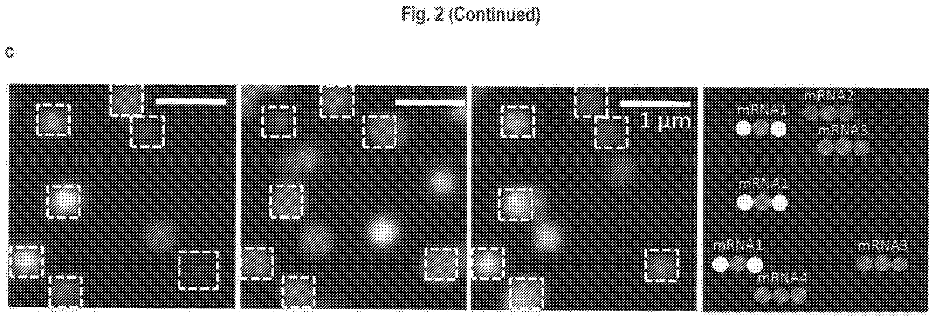

**FIG. 2****.** Exemplary sequential barcoding of provided methods. (a) Schematic of sequential barcoding. In each round of hybridization, multiple probes (e.g., 24)were hybridized on each transcript, imaged and then stripped by DNase I treatment. The same probe sequences could be used in different rounds of hybridization, but probes were coupled to different fluorophores. (b) Composite four-color FISH Data from 3 rounds of hybridizations on multiple yeast cells. Twelve genes were encoded by 2 rounds of hybridization, with the third hybridization using the same probes as hybridization 1. The boxed regions were magnified in the bottom right corner of each image. The matching spots were shown and barcodes were extracted. Spots without co-localization, without the intention to be limited by theory, could be due to nonspecific binding of probes in the cell as well as mis-hybridization. The number of each barcode were quantified to provide the abundances of the corresponding transcripts in single cells. (c) Exemplary barcodes. mRNA 1: Yellow-Blue-Yellow; mRNA 2: Green-Purple- Green; mRNA 3: Purple-Blue-Purple; and mRNA 4: Blue-Purple-Blue.
**FIG. 3****.** Schematic of sequential hybridization and barcoding. (a) Schematic of sequential hybridization and barcoding. (b) Schematic of the FISH images of the cell. In each round of hybridization, the same spots were detected, but the dye associated with the transcript changes. The identity of an mRNA was encoded in the temporal sequence of dyes hybridized.

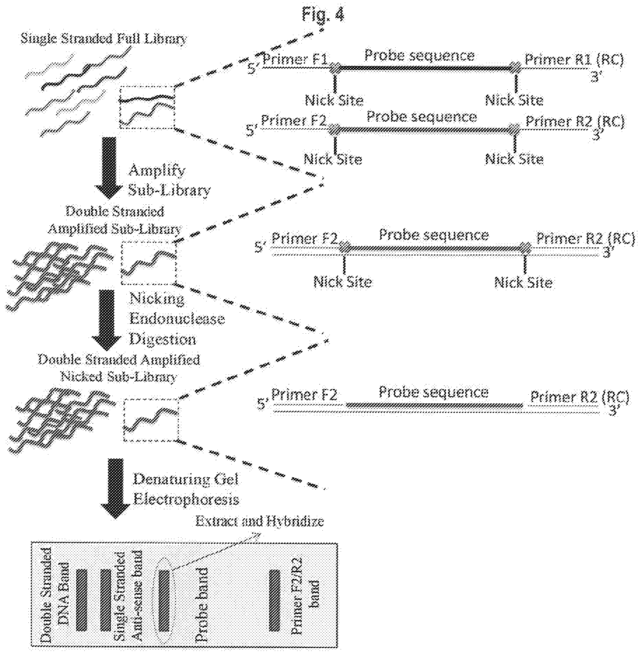

**FIG. 4****.** Exemplary oligonucleotide preparation. The original oligonucleotide (as exemplified in this Figure, probe) library contains several probe sub-libraries. Each sub- library has a specific set of primers that can be used to amplify the sub-library using PCR. Once the desired sub-library is amplified, the product is incubated with a nicking enzyme. The enzyme cleaves the phosphodiester bond on the probe strand at its recognition site. Denaturing the resulting product and running it on a denaturing gel allows the desired probe sequence to be released. The probe band can then be cut out of the gel and extracted. The extracted product can be used for hybridization.
**FIG. 5** illustrates an exemplary reaction scheme for synthesizing DNA probes conjugated to dye through cleavable disulfide linker.
**FIG. 6A** is a schematic illustrating an exemplary embodiment of a sequential barcoding method using gene specific primary probes, secondary bridge probes and tertiary readout probes.
**FIG. 6B** illustrates an exemplary embodiment of a sequential barcoding method using primary probes with two overhang sequences.
**FIG. 7A** illustrates an exemplary hybridization chain reaction (HCR) that is carried out according to prior art methods.
**FIG. 7B** illustrates an exemplary readout probe.
**FIG. 7C** illustrates an exemplary hybridization chain reaction based on readout probes with cleavable linkers.
**FIG. 8** is a schematic of an exemplary re-hybridization scheme for targeting nucleic acid molecules of interest.
**FIG. 9** is a schematic of an exemplary re-hybridization scheme for targeting protein molecules of interest.
**FIG. 10** is a representative set of confocal images illustrating a sequential hybridization with removal of readout probes as described herein.
**FIG. 11** is a representative set of confocal images illustrating a sequential hybridization protocol using oligonucleotide-conjugated antibodies for detecting target molecules and with removal of readout probes between rounds of hybridization as described herein.

### DETAILED DESCRIPTION OF CERTAIN EMBODIMENTS

Embodiments relate to new methods, compositions and/or kits for profiling nucleic acids (*e.g*., transcripts and/or DNA loci) in cells.

In some embodiments, provided herein are methods for profiling nucleic acids (e.g., transcripts and/or DNA loci) in cells. In some embodiments, provide methods profile multiple targets in single cells. Provided methods can, among other things, profile a large number of targets (transcripts, DNA loci or combinations thereof), with a limited number of detectable labels through sequential barcoding.

FIG. 1 depicts methodologies in accordance with embodiments disclosed herein. As depicted in FIG. 1, provided herein are methodologies in which multiple rounds of hybridization (contacting steps) with labeled probes detects target molecules (e.g., mRNAs) present in cells. For example, as depicted in FIG. 1, sets of probes that hybridize with nucleic acid targets in cells are provided, wherein probes (i.e., detectably labeled oligonucleotides that hybridize with different targets) are labeled within a single set and, furthermore, at least one probe is differently labeled in different sets.

In some embodiments, for example, as represented in FIG. 1, provided herein are methods comprising steps of:
(a) performing a first contacting step that involves contacting a cell comprising a plurality of transcripts and DNA loci with a first plurality of detectably labeled oligonucleotides, each of which targets a transcript or DNA locus and is labeled with a detectable moiety, so that the composition comprises at least:
   (i) a first oligonucleotide targeting a first transcript or DNA locus and labeled with a first detectable moiety; and
   (ii) a second oligonucleotide targeting a second transcript or DNA locus and labeled with a second detectable moiety;
(b) imaging the cell after the first contacting step so that hybridization by oligonucleotides of the first plurality with their targets is detected;
(c) performing a second contacting step that involves contacting the cell with a second plurality of detectably labeled oligonucleotides, which second plurality includes oligonucleotides targeting overlapping transcripts and/or DNA loci that are targeted by the first plurality, so that the second plurality comprises at least:
   (i) a third oligonucleotide, optionally identical in sequence to the first oligonucleotide, targeting the first transcript or DNA locus; and
   (ii) a fourth oligonucleotide, optionally identical in sequence to the second oligonucleotide, targeting the second transcript or DNA locus,

   wherein the second plurality differs from the first plurality in that at least one of the oligonucleotides present in the second plurality is labeled with a different detectable moiety than the corresponding oligonucleotide targeting the same transcript or DNA locus in the first plurality, so that, in the second plurality:
      (iii) the third oligonucleotide is labeled with the first detectable moiety, the second detectable moiety or a third detectable moiety; and
      (iv) the fourth oligonucleotide is labeled with the first detectable moiety, the second detectable moiety, the third detectable moiety, or a fourth detectable moiety,
   wherein either the third oligonucleotide is labeled with a different detectable moiety than was the first oligonucleotide, or the fourth oligonucleotide is labeled with a different detectable moiety than was the second oligonucleotide, or both;
(d) imaging the cell after the second contacting step so that hybridization by oligonucleotides of the second plurality with their targets is detected; and
(e) optionally repeating the contacting and imaging steps, each time with a new plurality of detectably labeled oligonucleotides comprising oligonucleotides that target overlapping transcripts or DNA loci targeted by the first and second pluralities, wherein each utilized plurality differs from each other utilized plurality, due to at least one difference in detectable moiety labeling of oligonucleotides targeting the same transcript or DNA locus.

In the foregoing embodiments, the methods further comprise steps of:
(f) performing a contacting step that involves contacting a cell comprising a plurality of nucleic acids with a plurality of intermediate oligonucleotides, each of which:
   (i) targets a nucleic acid and is optionally labeled with a detectable moiety; and
   (ii) comprises an overhang sequence after hybridization with the target; and
(g) optionally imaging the cell so that interaction between the intermediate oligonucleotides with their targets is detected.

In some embodiments, step (f) and optionally step (g) are performed before step (a). In some embodiments, step (f) is performed step (a). In some embodiments, a removing step preserves intermediate oligonucleotides.

Also provided herein is a sequential hybridization method comprising the steps of:
a) contacting a target molecule with a plurality of primary antibodies, wherein each primary antibody comprises one or more binding targets connected in series and linked to the primary antibody;
b) contacting the target molecule with a first plurality of readout probes, wherein each readout probe comprises a signal moiety, and wherein each readout probe interacts with a first binding target of the one or more binding targets of a primary antibody of the plurality of primary antibodies,
   wherein the signal moiety is capable of emitting a first detectable visual signal upon the interaction of each readout probe from the first plurality of readout probes to the first binding target of a primary antibody of the plurality of primary antibodies;
c) imaging the target molecule after step b) so that the interactions between the first plurality of readout probes and the plurality of primary antibodies are detected by the presence of the first detectable visual signal;
d) contacting the target molecule, the plurality of primary antibodies and the first plurality of readout probes with a solution comprising a denaturing agent, wherein contact of the solution with the target molecule, the plurality of primary antibodies, and the first plurality of readout probes does not disrupt the interaction between the plurality of primary antibodies and the target molecule;
e) contacting the target molecule and the plurality of primary antibodies with a second plurality of readout probes, wherein each readout probe comprises a signal moiety, and wherein each readout probe interacts with a second binding target of a primary antibody of the plurality of primary antibodies,
   wherein the signal moiety is capable of emitting a second detectable visual signal upon the interaction of each readout probe with the second binding target of a primary antibody of the plurality of primary antibodies; and
f) imaging the target nucleic acid molecule after step e) so that interactions between the second plurality of readout probes and the plurality of primary antibodies are detected by the presence of the second detectable visual signal.

In the foregoing embodiments, the target molecule can be a nucleic acid or a protein. For example, in some embodiments, the target molecule is a DNA sequence. In some embodiments, the target molecule is an RNA sequence. In some embodiments, the target molecule is an RNA transcipt. In some embodiments, the target molecule is protein.

In addition, provided herein is a sequential hybridization method comprising the steps of:
a) contacting a target nucleic acid molecule with a plurality of primary probes, wherein each primary probe comprises: (i) a primary binding sequence that binds to a complementary target sequence within the target nucleic acid molecule, and (ii) a first overhang sequence connected to one end of the primary binding sequence comprising one or more binding targets connected in series and linked to the primary binding sequence;
b) contacting the target nucleic acid molecule with a first plurality of readout probes, wherein each readout probe comprises a signal moiety, and wherein each readout probe interacts with a first binding target of the one or more binding targets of a primary probe of the plurality of primary probes,
   wherein the signal moiety is capable of emitting a first detectable visual signal upon the interaction of each readout probe from the first plurality of readout probes with the first binding target of the one or more binding targets of a primary probe of the plurality of primary probes;
c) imaging the target nucleic acid molecule after step b) so that the interactions between the first plurality of readout probes and the plurality of primary probes are detected by the presence of a first detectable visual signal;
d) contacting the target nucleic acid molecule, the plurality of primary probes, and the first plurality of readout probes with a solution comprising a denaturing agent, wherein contact of the solution with the target nucleic acid molecule, the plurality of primary probes, and the first plurality of readout probes does not disrupt the interaction between the plurality of primary probes and the target nucleic acid molecule;
e) contacting the target nucleic acid molecule with a second plurality of readout probes, wherein each readout probe comprises a signal moiety, and wherein each readout probe interacts with a second binding target of the one or more binding targets of a primary probe,
   wherein the signal moiety is capable of emitting a second detectable visual signal upon the interaction of each readout probe from the second plurality of readout probes with the second binding target of the one or more binding targets of a primary probe of the plurality of primary probes; and
f) imaging the target nucleic acid molecule after step e) so that the interactions between the second plurality of readout probes and the plurality of primary probes are detected by the presence of the second detectable visual signal.

In the foregoing embodiments, the target nucleic acid molecule can a DNA sequence or an RNA sequence, including, for example, an RNA transcipt.

In embodiments disclosed herein, a detectably labeled oligonucleotide is labeled with a detectable moiety. In some embodiments, a detectably labeled oligonucleotide comprises one detectable moiety. In some embodiments, a detectably labeled oligonucleotide comprises two or more detectable moieties. In some embodiments, a detectably labeled oligonucleotide has one detectable moiety. In some embodiments, a detectably labeled oligonucleotide has two or more detectable moiety.

In embodiments disclosed herein, a probe having a signal moiety is labeled with or linked to a signal moiety. In some embodiments, a probe having a signal moiety comprises one signal moiety. In some embodiments, a probe having a signal moiety comprises two or more signal moieties. In some embodiments, a probe having a signal moiety has one signal moiety. In some embodiments, a probe having a signal moiety has two or more signal moieties.

In some embodiments, a detectable moiety or a signal moiety is or comprises a fluorophore. Exemplary detectably labeled oligonucleotides or probes having a signal moiety can be labeled with fluorophores and include, but are not limited to, probes for fluorescence *in situ* hybridization (FISH). Widely known and practiced by persons having ordinary skill in the art, FISH is used to, among other things, to detect and localize the presence or absence of specific DNA sequences or RNA targets. Methods for designing and preparing detectably labeled oligonucleotides labeled are widely known in the art, including but not limited to those described in, for example, U.S. Patent Application Publication No. 2012-0142014. Due to limitations such as fluorophore availability, FISH, however, can only be used to profile a limited number of targets in a given experiment. Through sequential barcoding to multiplex different targets, the methods disclosed herein can profile a large number of targets, up to *F^{N},* wherein *F* is the number of types of detectable moieties (in the case of FISH, fluorophores) and *N* is the number of contacting steps (in the case of FISH, hybridization). For example, when *F* is four and *N* is 8, almost the entire transcriptome (4⁸ = 65,536) can be profiled. In some embodiments, *F* is at least 2. In some embodiments, *F* is 3. In some embodiments, *F* is 4. In some embodiments, *F* is 5. In some embodiments, *F* is 6. In some embodiments, *F* is 7. In some embodiments, *F* is 8. In some embodiments, *F* is 9. In some embodiments, *F* is 10. In some embodiments, *F* is 11. In some embodiments, *F* is 12. In some embodiments, *F* is 13. In some embodiments, *F* is 14. In some embodiments, *F* is 15. In some embodiments, *F* is greater than 15. In some embodiments, *Nis* 2. In some embodiments, *N* is greater than 2. In some embodiments, *N* is 3. In some embodiments, *N* is greater than 3. In some embodiments, *N* is 4. In some embodiments, *N* is greater than 4. In some embodiments, *N* is 5. In some embodiments, *N* is greater than 5. In some embodiments, *N* is 6. In some embodiments, *N* is greater than 6. In some embodiments, *N* is 7. In some embodiments, *N* is greater than 7. In some embodiments, *N* is 8. In some embodiments, *N* is greater than 8. In some embodiments, *N* is 9. In some embodiments, *N* is greater than 9. In some embodiments, *N* is 10. In some embodiments, *N* is greater than 10. In some embodiments, a plurality of detectably labeled oligonucleotides target at least 100 targets.

In a contacting step, a detectably labeled oligonucleotide or probe having a signal moiety can be labeled prior to, concurrent with or subsequent to its binding to its target. In some embodiments, a detectably labeled oligonucleotide or probe having a signal moiety, such as a fluorophore-labeled oligonucleotide, is labeled prior to its binding to its target. In some embodiments, a detectably labeled oligonucleotide or probe having a signal moiety is labeled concurrent with its binding to its target. In some embodiments, a detectably labeled oligonucleotide or probe having a signal moiety is labeled subsequent to its binding to its target. In some embodiments, a detectably labeled oligonucleotide or probe having a signal moiety is labeled subsequent to hybridization through orthogonal amplification with hybridization chain reactions (HCR) (Choi, HM., Nat Biotechnol. 2010 Nov;28(11):1208-12). In some embodiments, a detectably labeled oligonucleotide or probe having a signal moiety comprises a moiety, *e.g.,* a nucleic acid sequence, that one or more moieties that can provide signals in an imaging step can be directly or indirectly linked to the oligonucleotide.

In some embodiments, the same type of labels can be attached to different probes or oligonucleotides for different targets. In some embodiments, probes or oligonucleotides for the same target have the same label in a plurality of detectably labeled probes or oligonucleotides used in a contacting step (a set of detectably labeled oligonucleotides). Each target, after rounds of contacting and imaging, has its own unique combination of labels (sequential barcoding), so that information, *e*.*g*., quantitative and/or spatial information, can be obtained for a target. For example, when fluorophores are used to label detectably labeled oligonucleotides or a probe having a signal moiety, after *N* steps, a target would have a sequential barcode of *F₁F₂...F_{N}*, wherein *Fₙ* is the color of fluorophore used for the target in the n-th imaging. One target can be differentiated from another by a difference in their barcodes (*e*.*g*., RedRedBlueRed compared to RedRedRedBlue).

In some embodiments, the labels disclosed herein are or comprise one or more fluorescent dyes, including but not limited to fluorescein, rhodamine, Alexa Fluors, DyLight fluors, ATTO Dyes, or any analogs or derivatives thereof.

In some embodiments, the labels disclosed herein include, but are not limited to, fluorescein and chemical derivatives of fluorescein; Eosin; Carboxyfluorescein; Fluorescein isothiocyanate (FITC); Fluorescein amidite (FAM); Erythrosine; Rose Bengal; fluorescein secreted from the bacterium *Pseudomonas aeruginosa*; Methylene blue; Laser dyes; Rhodamine dyes (*e*.*g*., Rhodamine, Rhodamine 6G, Rhodamine B, Rhodamine 123, Auramine O, Sulforhodamine 101, Sulforhodamine B, and Texas Red).

In some embodiments, the labels disclosed herein include, but are not limited to, ATTO dyes; Acridine dyes (*e*.*g*., Acridine orange, Acridine yellow); Alexa Fluor; 7-Amino actinomycin D; 8-Anilinonaphthalene-1-sulfonate; Auramine-rhodamine stain; Benzanthrone; 5,12-Bis(phenylethynyl)naphthacene; 9,10-Bis(phenylethynyl)anthracene; Blacklight paint; Brainbow; Calcein; Carboxyfluorescein; Carboxyfluorescein diacetate succinimidyl ester; Carboxyfluorescein succinimidyl ester; 1-Chloro-9,10-bis(phenylethynyl)anthracene; 2-Chloro- 9,10-bis(phenylethynyl)anthracene; 2-Chloro-9,10-diphenylanthracene; Coumarin; Cyanine dyes (e.g., Cyanine such as Cy3 and Cy5, DiOC6, SYBR Green I); DAPI, Dark quencher, DyLight Fluor, Fluo-4, FluoProbes; Fluorone dyes (e.g., Calcein, Carboxyfluorescein, Carboxyfluorescein diacetate succinimidyl ester, Carboxyfluorescein succinimidyl ester, Eosin, Eosin B, Eosin Y, Erythrosine, Fluorescein, Fluorescein isothiocyanate, Fluorescein amidite, Indian yellow, Merbromin); Fluoro-Jade stain; Fura-2; Fura-2-acetoxymethyl ester; Green fluorescent protein, Hoechst stain, Indian yellow, Indo-1, Lucifer yellow, Luciferin, Merocyanine, Optical brightener, Oxazin dyes (e.g., Cresyl violet, Nile blue, Nile red); Perylene; Phenanthridine dyes (Ethidium bromide and Propidium iodide); Phloxine, Phycobilin, Phycoerythrin, Phycoerythrobilin, Pyranine, Rhodamine, Rhodamine 123, Rhodamine 6G, RiboGreen, RoGFP, Rubrene, SYBR Green I, (E)-Stilbene, (Z)-Stilbene, Sulforhodamine 101, Sulforhodamine B, Synapto-pHluorin, Tetraphenyl butadiene, Tetrasodium tris(bathophenanthroline disulfonate)ruthenium(II), Texas Red, TSQ, Umbelliferone, or Yellow fluorescent protein.

In some embodiments, labels of the present invention include but are not limited to Alexa Fluor family of fluorescent dyes (Molecular Probes, Oregon). Alexa Fluor dyes are widely used as cell and tissue labels in fluorescence microscopy and cell biology. The excitation and emission spectra of the Alexa Fluor series cover the visible spectrum and extend into the infrared. The individual members of the family are numbered according roughly to their excitation maxima (in nm). Certain Alexa Fluor dyes are synthesized through sulfonation of coumarin, rhodamine, xanthene (such as fluorescein), and cyanine dyes. In some embodiments, sulfonation makes Alexa Fluor dyes negatively charged and hydrophilic. In some embodiments, Alexa Fluor dyes are more stable, brighter, and less pH-sensitive than common dyes (e.g. fluorescein, rhodamine) of comparable excitation and emission, and to some extent the newer cyanine series. Exemplary Alexa Fluor dyes include but are not limited to Alexa-350, Alexa- 405, Alexa-430, Alexa-488, Alexa-500, Alexa-514, Alexa-532, Alexa-546, Alexa-555, Alexa-568, Alexa-594, Alexa-610, Alexa-633, Alexa-647, Alexa-660, Alexa-680, Alexa-700, or Alexa-750.

In some embodiments, the labels can comprise one or more of the DyLight Fluor family of fluorescent dyes (Dyomics and Thermo Fisher Scientific). Exemplary DyLight Fluor family dyes include but are not limited to DyLight-350, DyLight-405, DyLight- 488, DyLight-549, DyLight-594, DyLight-633, DyLight-649, DyLight-680, DyLight-750, or DyLight-800.

In some embodiments, a detectable or signal moiety is or comprises a nanomaterial. In some embodiments, a detectable or signal moiety is or compresses a nanoparticle. In some embodiments, a detectable or signal moiety is or comprises a quantum dot. In some embodiments, a detectable or signal moiety is a quantum dot. In some embodiments, a detectable or signal moiety comprises a quantum dot. In some embodiments, a detectable or signal moiety is or comprises a gold nanoparticle. In some embodiments, a detectable or signal moiety is a gold nanoparticle. In some embodiments, a detectable or signal moiety comprises a gold nanoparticle.

One of skill in the art understands that, in some embodiments, selection of label or signal moiety for a particular probe or oligonucleotide in a particular cycle may be determined based on a variety of factors, including, for example, size, types of signals generated, manners attached to or incorporated into a probe, properties of the cellular constituents including their locations within the cell, properties of the cells, types of interactions being analyzed, and etc.

For example, in some embodiments, probes are labeled with either Cy3 or Cy5 that has been synthesized to carry an N-hydroxysuccinimidyl ester (NHS-ester) reactive group. Since NHS-esters react readily with aliphatic amine groups, nucleotides can be modified with aminoalkyl groups. This can be done through incorporating aminoalkyl-modified nucleotides during synthesis reactions. In some embodiments, a label is used in every 60 bases to avoid quenching effects.

A detectably labeled oligonucleotide or probe having a signal moiety can hybridize with a target, *e.g.,* a transcript or DNA locus. In some embodiments, a target is or comprises a transcript. In some embodiments, a target is a transcript. In some embodiments, a transcript is an RNA. In some embodiments, a transcript is an mRNA. In some embodiments, a transcript is tRNA. In some embodiments, a transcript is rRNA. In some embodiments, a transcript is snRNA. In some embodiments, an RNA is a non-coding RNA. Exemplary non-coding RNA types are widely known in the art, including but not limited to long non-coding RNA (lncRNA), microRNA (miRNA), short interfering RNA (siRNA), piwi-interacting RNA (piRNA), small nucleolar RNA (snoRNA) and other short RNAs. In some embodiments, an RNA is lncRNA. In some embodiments, an RNA is miRNA. In some embodiments, an RNA is piRNA. In some embodiments, an RNA is snoRNA.

In some embodiments, a target is or comprises a DNA locus. In some embodiments, when a target is a DNA locus, a detectably labeled oligonucleotide optionally comprises one or more RNA nucleotide or RNA segments. A detectably labeled oligonucleotide comprises RNA sequences can be selectively removed, for example, through RNA-specific enzymatic digestion, after imaging without degrading the DNA target. Exemplary enzymes that specifically degrade RNA but not DNA include but are not limited to various RNase, such as RNase A and RNase H.

In some embodiments, a detectably labeled oligonucleotide or probe having a signal moiety directly hybridizes to its target, *e*.*g*., a transcript or DNA locus. In some embodiments, a detectably labeled oligonucleotide or probe having a signal moiety specifically interacts with (recognizes) its target through binding or hybridization to one or more intermediate, *e*.*g*., an oligonucleotide, that is bound, hybridized, or otherwise specifically linked to the target. In some embodiments, an intermediate oligonucleotide is hybridized against its target with an overhang such that a second oligonucleotide with complementary sequence (also referred to as a "bridge oligonucleotide," "bridge probe," or a "readout probe") can bind to it. For example, in some embodiments, an intermediate oligonucleotide (also referred herein as a "primary probe") is hybridized against a target molecule, wherein the intermediate oligonucleotide includes at least one overhang sequence such that a readout probe, which includes (i) a sequence that is complementary to a portion of the overhang sequence of the intermediate oligonucleotide and (ii) a detectable or signal moiety, can bind to the intermediate oligonucleotide. In some embodiments, the at least one overhang sequence of the intermediate oligonucleotide is complementary to a sequence of a bridge probe, and the bridge probe includes a sequence that is complementary to that of a readout probe, wherein the readout probe includes (i) a sequence that is complementary to a portion of the bridge probe and (ii) a detectable or signal moiety. The readout probe then interacts with the intermediate oligonucleotide through binding with the bridge probe, which is linked or bound to the intermediate oligonucleotide.

In some embodiments, an intermediate targets a nucleic acid and is optionally labeled with a detectable or signal moiety, and comprises an overhang sequence after hybridization with the target. In some embodiments, an intermediate comprises a sequence that hybridizes to a target, an overhang sequence, and optionally a detectable or signal moiety. In some embodiments, an intermediate comprises a sequence that hybridizes to a target and an overhang sequence. In some embodiments, an intermediate does not have a detectable or signal moiety. In some embodiments, a second oligonucleotide is a detectably labeled oligonucleotide. In some embodiments, a second detectably labeled oligonucleotide is labeled with a dye. In some embodiments, a detectably labeled oligonucleotide is labeled with an HCR polymer. In some embodiments, intermediate oligonucleotides bound to targets are preserved through multiple contacting, removing and/or imaging steps; sequential barcodes are provided through combinations of detectable labels that are linked to intermediate oligonucleotides through bridge probes in the contacting and imaging steps. For example, when detectably labeled oligonucleotides are used as readout probes, barcodes are provided by detectably labeled oligonucleotides that hybridize with intermediate oligonucleotides through their overhang sequences. After an imaging step, readout oligonucleotides are optionally removed as described herein. In some embodiments, the readout probes interact directly with the intermediate oligonucleotides. In some embodiments, the readout probes interact with a bridge probe, which interacts or is hybridized to the intermeidate oligonucleotides.

In some embodiments, one intermediate oligonucleotide is employed for a target. In some embodiments, two or more intermediate oligonucleotides are employed for a target. In some embodiments, three or more intermediate oligonucleotides are employed for a target. In some embodiments, four or more intermediate oligonucleotides are employed for a target. In some embodiments, five or more intermediate oligonucleotides are employed for a target. In some embodiments, six or more intermediate oligonucleotides are employed for a target. In some embodiments, seven or more intermediate oligonucleotides are employed for a target. In some embodiments, eight or more intermediate oligonucleotides are employed for a target. In some embodiments, nine or more intermediate oligonucleotides are employed for a target. In some embodiments, 10 or more intermediate oligonucleotides are employed for a target. In some embodiments, 11 or more intermediate oligonucleotides are employed for a target. In some embodiments, 12 or more intermediate oligonucleotides are employed for a target. In some embodiments, 13 or more intermediate oligonucleotides are employed for a target. In some embodiments, 14 or more intermediate oligonucleotides are employed for a target. In some embodiments, 15 or more intermediate oligonucleotides are employed for a target. In some embodiments, 16 or more intermediate oligonucleotides are employed for a target. In some embodiments, 17 or more intermediate oligonucleotides are employed for a target. In some embodiments, 18 or more intermediate oligonucleotides are employed for a target. In some embodiments, 19 or more intermediate oligonucleotides are employed for a target. In some embodiments, 20 or more intermediate oligonucleotides are employed for a target. In some embodiments, 21 or more intermediate oligonucleotides are employed for a target. In some embodiments, 22 or more intermediate oligonucleotides are employed for a target. In some embodiments, 23 or more intermediate oligonucleotides are employed for a target. In some embodiments, 24 or more intermediate oligonucleotides are employed for a target. In some embodiments, 25 or more intermediate oligonucleotides are employed for a target. In some embodiments, 30 or more intermediate oligonucleotides are employed for a target. In some embodiments, 40 or more intermediate oligonucleotides are employed for a target. In some embodiments, 50 or more intermediate oligonucleotides are employed for a target.

In some embodiments, each intermediate oligonucleotide hybridizes with a different sequence of a target. In some embodiments, each intermediate oligonucleotide of a target comprises the same overhang sequence. In some embodiments, each detectably labeled oligonucleotide for a target comprises the same sequence complimentary to the same overhang sequence shared by all intermediate oligonucleotides of the target. In some embodiments, an intermediate oligonucleotide comprises a sequence complimentary to a target, and a sequence complimentary to a detectably labeled oligonucleotide.

In some embodiments, provided technologies are used to profile different transcripts formed as a result of splicing variation, RNA editing, oligonucleotide modification, or a combination thereof. In some embodiments, a target is an RNA splicing variant. In some embodiments, provided technologies profile one or more splicing variants of a gene, *e*.*g*., locations and quantities of one or more splicing variant of a gene. In some embodiments, provided methods or compositions profile different splicing variants. In some embodiments, an exon that contains one or more variants is targeted and barcoded by sequential hybridization and barcoding. In some embodiments, a splicing variant contains one or more distinguishable sequences resulted from splicing, and such sequences are targeted. In some embodiments, by targeting exons and/or distinguishable sequences, provided technologies can profile one or more specific splicing variants, or an entire splicing repertoire of an mRNA. As widely known in the art, mRNA splicing are important to numerous biological processes and diseases, for example, neurological diseases like autism or Down syndrome. Molecules responsible for cell-to-cell adhesion and synpatogenesis are spliced and their defects are known to generate miswiring in the brain and cause diseases.

In some embodiments, detectably labeled oligonucleotides target sequence modifications caused by sequence editing, chemical modifications and/or combinations thereof. In some embodiments, a modified nucleic acid target, optionally after a conversion process, hybridizes with one or more different complementary sequences compared to an un-modified target, and is profiled using one or more oligonucleotides that selectively hybridizes with the modified nucleic acid. In some embodiments, a target is an RNA through by RNA editing (Brennicke, A., A. Marchfelder, et al. (1999). "RNA editing". FEMS Microbiol Rev 23 (3): 297- 316). In some embodiments, provided technologies profiles different RNA variants formed by RNA editing. In some embodiments, provided technologies profile modified oligonucleotide. In some embodiments, provided technologies profiles methylated RNA (Song CX, Yi C, He C. Mapping recently identified nucleotide variants in the genome and transcriptome. Nat Biotechnol. 2012 Nov;30(11):1107-16). In some embodiments, provided technologies profile methylated DNA. In some embodiments, a target is single-nucleotide polymorphism (SNP).

In some embodiments, by profiling a target, provided technologies provide, among other things, quantitative and/or positioning information of a target, in some cases, in single cells, a tissue, an organ, or an organism. In some embodiments, profiling of transcripts can be used to qualitatively and/or quantitatively define the spatial-temporal patterns of gene expression within cells, tissues, organs or organisms.

In some embodiments, each detectably labeled oligonucleotide in a set has a different target, *e*.*g*., a transcript, a DNA locus, or a protein. In some embodiments, two or more detectably labeled oligonucleotides in a set have the same target. In some embodiments, two or more detectably labeled oligonucleotides target the same transcript. In some embodiments, two or more detectably labeled oligonucleotides target the same DNA locus. In some embodiments, about 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 50, 60, 70, 80, 90 or 100 detectably labeled oligonucleotides the same target. In some embodiments, two or more detectably labeled oligonucleotides target the same target. In some embodiments, five or more detectably labeled oligonucleotides target the same target. In some embodiments, 10 or more detectably labeled oligonucleotides target the same target. In some embodiments, 15 or more detectably labeled oligonucleotides target the same target. In some embodiments, 20 or more detectably labeled oligonucleotides target the same target. In some embodiments, 25 or more detectably labeled oligonucleotides target the same target. In some embodiments, 30 or more detectably labeled oligonucleotides target the same target. In some embodiments, 35 or more detectably labeled oligonucleotides target the same target. In some embodiments, 40 or more detectably labeled oligonucleotides target the same target. In some embodiments, 45 or more detectably labeled oligonucleotides target the same target. In some embodiments, 50 or more detectably labeled oligonucleotides target the same target. In some embodiments, 60 or more detectably labeled oligonucleotides target the same target. In some embodiments, 70 or more detectably labeled oligonucleotides target the same target. In some embodiments, 80 or more detectably labeled oligonucleotides target the same target. In some embodiments, 90 or more detectably labeled oligonucleotides target the same target. In some embodiments, 100 or more detectably labeled oligonucleotides target the same target. In some embodiments, about 1-10 detectably labeled oligonucleotides target the same target. In some embodiments, about 5-15 detectably labeled oligonucleotides target the same target. In some embodiments, about 10-20 detectably labeled oligonucleotides target the same target. In some embodiments, about 15-25 detectably labeled oligonucleotides target the same target. In some embodiments, about 20-30 detectably labeled oligonucleotides target the same target. In some embodiments, about 25-35 detectably labeled oligonucleotides target the same target. In some embodiments, about 30-40 detectably labeled oligonucleotides target the same target. In some embodiments, about 35-45 detectably labeled oligonucleotides target the same target. In some embodiments, about 40-50 detectably labeled oligonucleotides target the same target. In some embodiments, about 45-55 detectably labeled oligonucleotides target the same target. In some embodiments, about 50-70 detectably labeled oligonucleotides target the same target. In some embodiments, about 60-80 detectably labeled oligonucleotides target the same target. In some embodiments, about 70-90 detectably labeled oligonucleotides target the same target. In some embodiments, about 80-100 detectably labeled oligonucleotides target the same target.

In some embodiments, using multiple detectably labeled oligonucleotides for the same target increases signal intensity. In some embodiments, each detectably labeled oligonucleotide in a set targeting the same target interacts with a different portion of a target.

In some embodiments, all detectably labeled oligonucleotides for a target in a set have the same detectable moieties. In some embodiments, all detectably labeled oligonucleotides are labeled in the same way. In some embodiments, all the detectably labeled oligonucleotides for a target have the same fluorophore.

In some embodiments, detectably labeled oligonucleotides for a target are positioned within a targeted region of a target. A targeted region can have various lengths. In some embodiments, a targeted region is about 20 bp in length. In some embodiments, a targeted region is about 30 bp in length. In some embodiments, a targeted region is about 40 bp in length. In some embodiments, a targeted region is about 50 bp in length. In some embodiments, a targeted region is about 60 bp in length. In some embodiments, a targeted region is about 80 bp in length. In some embodiments, a targeted region is about 100 bp in length. In some embodiments, a targeted region is about 150 bp in length. In some embodiments, a targeted region is about 200 bp in length. In some embodiments, a targeted region is about 250 bp in length. In some embodiments, a targeted region is about 300 bp in length. In some embodiments, a targeted region is about 350 bp in length. In some embodiments, a targeted region is about 400 bp in length. In some embodiments, a targeted region is about 450 bp in length. In some embodiments, a targeted region is about 500 bp in length. In some embodiments, a targeted region is about 600 bp in length. In some embodiments, a targeted region is about 700 bp in length. In some embodiments, a targeted region is about 800 bp in length. In some embodiments, a targeted region is about 900 bp in length. In some embodiments, a targeted region is about 1,000 bp in length. In some embodiments, detectably labeled oligonucleotides for a target are positioned in proximity to each other on the target.

As understood by a person having ordinary skill in the art, different technologies can be used for the imaging steps. Exemplary methods include but are not limited to epi-fluorescence microscopy, confocal microscopy, the different types of super-resolution microscopy (PALM/STORM, SSIM/GSD/STED), and light sheet microscopy (SPIM and etc).

Exemplary super resolution technologies include but are not limited to I⁵M and 4Pi-microscopy, Stimulated Emission Depletion microscopy (STEDM), Ground State Depletion microscopy (GSDM), Spatially Structured Illumination microscopy (SSIM), Photo-Activated Localization Microscopy (PALM), Reversible Saturable Optically Linear Fluorescent Transition (RESOLFT), Total Internal Reflection Fluorescence Microscope (TIRFM), Fluorescence-PALM (FPALM), Stochastical Optical Reconstruction Microscopy (STORM), Fluorescence Imaging with One-Nanometer Accuracy (FIONA), and combinations thereof. For examples: Chi, 2009 "Super-resolution microscopy: breaking the limits, Nature Methods 6(1):15-18; Blow 2008, "New ways to see a smaller world," Nature 456:825-828; Hell, et al., 2007, "Far-Field Optical Nanoscopy," Science 316: 1153; R. Heintzmann and G. Ficz, 2006, "Breaking the resolution limit in light microscopy," Briefings in Functional Genomics and Proteomics 5(4):289-301; Garini et al., 2005, "From micro to nano: recent advances in high-resolution microscopy," Current Opinion in Biotechnology 16:3-12; and Bewersdorf et al., 2006, "Comparison of I5M and 4Pi-microscopy," 222(2):105-117; and Wells, 2004, "Man the Nanoscopes," JCB 164(3):337-340.

In some embodiments, electron microscopes (EM) are used.

In some embodiments, an imaging step detects a target. In some embodiments, an imaging step localizes a target. In some embodiments, an imaging step provides three-dimensional spatial information of a target. In some embodiments, an imaging step quantifies a target. By using multiple contacting and imaging steps, provided methods are capable of providing spatial and/or quantitative information for a large number of targets in surprisingly high throughput. For example, when using *F* detectably different types of labels, spatial and/or quantitative information of up to *F^{N}* targets can be obtained after *N* contacting and imaging steps.

In some embodiments, provided methods comprise additional steps before or after a contacting and/or an imaging step. In some embodiments, provided methods comprise a step of removing a plurality of detectably labeled oligonucleotides after each imaging step. In some embodiments, a step of removing comprises degrading the detectably labeled oligonucleotides. In some embodiments, a step of removing does not significantly degrade a target, so that a target can be used for the next contacting and/or imaging step(s) if desired. In some embodiments, a step of removing comprises contacting the plurality of detectably labeled oligonucleotides with an enzyme that digests a detectably labeled oligonucleotide. In some embodiments, a step of removing comprises contacting the plurality of detectably labeled oligonucleotides with a DNase or RNase. For example, in some embodiments, a detectably labeled oligonucleotide comprises a DNA sequence, and a DNase is used for its degradation; in some other embodiments, a detectably labeled oligonucleotide comprises an RNA sequence, and an RNase is used for its degradation. In some embodiments, a step of removing comprises degrading a detectable moiety. In some embodiments, a step of removing comprises photobleaching. In some embodiments, a step of removing comprises contacting the plurality of detectably labeled oligonucleotides with a denaturing agent to disrupt the interaction between the detectably labeled oligonucleotides and the intermediate probe or the target nucleic acid. Denaturing agents and compositions are disclosed herein.

In some embodiments, targets of one set of detectably labeled oligonucleotides are also targets of another set. In some embodiments, targets of one set of detectably labeled oligonucleotides overlap with those of another set. In some embodiments, the overlap is more than 10%. In some embodiments, the overlap is more than 20%. In some embodiments, the overlap is more than 30%. In some embodiments, the overlap is more than 40%. In some embodiments, the overlap is more than 50%. In some embodiments, the overlap is more than 60%. In some embodiments, the overlap is more than 70%. In some embodiments, the overlap is more than 80%. In some embodiments, the overlap is more than 90%. In some embodiments, the overlap is more than 91%. In some embodiments, the overlap is more than 92%. In some embodiments, the overlap is more than 93%. In some embodiments, the overlap is more than 94%. In some embodiments, the overlap is more than 90%. In some embodiments, the overlap is more than 95%. In some embodiments, the overlap is more than 96%. In some embodiments, the overlap is more than 97%. In some embodiments, the overlap is more than 98%. In some embodiments, the overlap is more than 99%. In some embodiments, the overlap is more than 99.5%. In some embodiments, the overlap is more than 99.6%. In some embodiments, the overlap is more than 99.7%. In some embodiments, the overlap is more than 99.8%. In some embodiments, the overlap is more than 99.9%. In some embodiments, the overlap is 100%. In some embodiments, targets of one set of detectably labeled oligonucleotides are the same as targets of another set. In some embodiments, each set of detectably labeled oligonucleotides targets the same targets.

In some embodiments, a third detectably labeled oligonucleotide in a second contacting step targeting the first transcript or DNA locus (the first target) optionally has an identical sequence to the first detectably labeled oligonucleotide targeting the first transcript or DNA locus. In some embodiments, the sequences are identical. In some embodiments, the sequences are different. Similarly, in some embodiments, a fourth detectably labeled oligonucleotide in a second contacting step targeting the second transcript or DNA locus (the first target) optionally has an identical sequence to the second detectably labeled oligonucleotide targeting the first transcript or DNA locus. In some embodiments, the sequences are identical. In some embodiments, the sequences are different.

In some embodiments, the second plurality differs from the first plurality in that at least one of the oligonucleotides present in the second plurality is labeled with a different detectable moiety than the corresponding oligonucleotide targeting the same transcript or DNA locus in the first plurality. In some embodiments, each plurality of detectably labeled oligonucleotides is different from another, in that at least one of the oligonucleotides present in a plurality is labeled with a different detectable moiety than the corresponding oligonucleotide targeting the same transcript or DNA locus in another plurality.

In some embodiments, a detectably labeled oligonucleotide has the structure of [S]-[L], wherein [S] is an oligonucleotide sequence, [L] is a detectable moiety or a combination of detectable moieties. In some embodiments, [L] comprises multiple units of detectable labels, *e.g.,* fluorophores, each of which independently associates with one or more nucleotidic moieties of an oligonucleotide sequence, *e.g.,* [S]. In some embodiments, each detectable label attached to the same detectably labeled oligonucleotide provides the same detectable signal. In some embodiments, all detectable labels attached to the same oligonucleotide sequence are the same.

In some embodiments, oligonucleotides targeting the same target have the same set of sequences among two or more sets of detectably labeled oligonucleotides, *i.e.,* the differences, if any, among the detectably labeled oligonucleotides are within the detectable moieties, not the sequences. For example, in one set of detectably labeled oligonucleotides, the detectably labeled oligonucleotides targeting a first target all have the same detectable moiety, or combination of detect moieties [L]₁:
[S]₁-[L]₁, [S]₂-[L]₁, ... , [S]ₙ-[L]₁, wherein n is the number of detectably labeled oligonucleotides for a target, *e.g.,* an integer of 3-50.

In another set of detectably labeled oligonucleotides, wherein oligonucleotides targeting the same target are differently labeled, the oligonucleotides targeting the same target are having the same set of oligonucleotide sequences ([S]₁, [S]₂, ... , [S]ₙ) yet a different [L]₂:
[S]₁-[L]₂, [S]₂-[L]₂, ... , [S]ₙ-[L]₂, wherein [L]₁ is detectably different than [L]₂.

For example, a two-step, two-label, 4-target (*F^{N}* = 2² = 4) process, wherein all detectably labeled oligonucleotides targeting the same target in each set independently have the same detectable moiety, is provided below:
Step 1. Contacting the targets with the first plurality (P1) of detectably labeled oligonucleotides:
   Target T1: [S]_{P1-T1-1}[L]₁, [S]_{P1-T1-2}[L]₁, [S]_{P1-T1-3}[L]₁, ..., [S]_{P1-T1-P1T1}[L]₁, wherein P1T1 is the number of detectably labeled oligonucleotides targeting T1 in the first plurality, and [L]₁ is the first detectable label;
   Target T2: [S]_{P1-T2-1}[L]₁, [S]_{P1-T2-2}[L]₁, [S]_{P1-T2-3}[L]₁, ..., [S]_{P1-T2-P1T2}[L]₁, wherein P1T2 is the number of detectably labeled oligonucleotides targeting T2 in the first plurality;
   Target T3: [S]_{P1-T3-1}[L]₂, [S]_{P1-T3-2}[L]₂, [S]_{P1-T3-3}[L]₂, ..., [S]_{P1-T3-P1T3}[L]₂, wherein P1T3 is the number of detectably labeled oligonucleotides targeting T3 in the first plurality, and [L]₂ is a detectably different label than [L]₁;
   Target T4: [S]_{P1-T4-1}[L]₂, [S]_{P1-T4-2}[L]₂, [S]_{P1-T4-3}[L]₂, ..., [S]_{P1-T4-P1T4}[L]₂, wherein P1T4 is the number of detectably labeled oligonucleotides targeting T4 in the first plurality.
Step 2: Imaging;
Step 3: Removing P1 from the targets;
Step 4: Contacting the targets with the second plurality (P2) of detectably labeled oligonucleotides:
   Target T1: [S]_{P2-T1-1}[L]₁, [S]_{P2-T1-2}[L]₁, [S]_{P2-T1-3}[L]₁, ..., [S]_{P2-T1-P2Y1}[L]₁, wherein P2T1 is the number of detectably labeled oligonucleotides targeting T1 in the second plurality;
   Target T2: [S]_{P2-T2-1}[L]₂, [S]_{P2-T2-2}[L]₂, [S]_{P2-T2-3}[L]₂, ..., [S]_{P2-T2}-_{P2T2}[L]₂, wherein P2T2 is the number of detectably labeled oligonucleotides targeting T2 in the second plurality;
   Target T3: [S]_{P2-T3-1}[L]₁, [S]_{P2-T3-2}[L]₁, [S]_{P2-T3-3}[L]₁, ..., [S]_{P2-T3-P2T3}[L]₁, wherein P2T3 is the number of detectably labeled oligonucleotides targeting T3 in the second plurality;
   Target T4: [S]_{P2-T4-1}[L]₂, [S]_{P2-T4-2}[L]₂, [S]_{P2-T4-3}[L]₂, ..., [S]_{P2-T4-P2T4}[L]₂, wherein P2T4 is the number of detectably labeled oligonucleotides targeting T4 in the second plurality.
Step 5: Imaging.

After the two imaging steps, each target has its own unique sequential barcode:
T1: [L]₁[L]₁;
T2: [L]₁[L]₂;
T3: [L]₂[L]₁; and
T4: [L]₂[L]₂.
In some embodiments, additional barcodes, T1--, T2--, --T1, --T2 can also be used, whereinindicates no signal for that step.

In the exemplified process above, each of P1T1, P1T2, P1T3, P1T4, P2T1, P2T2, P2T3 and P2T4 is independently a natural number (an integer greater than 0). In some embodiments, P1T1 = P2T1. In some embodiments, P1T2 = P2T2. In some embodiments, P1T3 = P2T3. In some embodiments, P1T4 = P2T4. In some embodiments, one detectably labeled oligonucleotide is used for a target. In some embodiments, two or more detectably labeled oligonucleotides are used for a target.

In some embodiments, detectably labeled oligonucleotides targeting the same target have the same set of sequences in each plurality.For example, for target T1 in the example above, each of [S]_{P1-T1-1} to [S]_{P1-T1-P1T1} independently has the same sequence as one of [S]_{P2-T1-1} to [S]_{P2-T1-P2T1}, and each of [S]_{P2-T1-1} to [S]_{P2-T1-P2T1} independently has the same sequence as one of [S]_{P1-T1-1} to [S]_{P1-T1-P1T1}. In some embodiments, detectably labeled oligonucleotides targeting the same target have different sets of sequences in each plurality.

In some embodiments, the methods provided herein optionally comprise a step of removing a plurality of detectably labeled oligonucleotides after an imaging step. In some embodiments, provided methods comprise a removing step after an imaging step. In some embodiments, provided methods comprise a removing step after each imaging step but the last imaging step. In some embodiments, provided methods comprise a removing step after each imaging step.

A removing step in the methods disclosed herein can serve one or more of a variety of purposes. In some embodiments, a removing step removes a plurality of detectably labeled oligonucleotides from targets so that targets are available for interacting with another plurality of detectably labeled oligonucleotides. In some embodiments, a removing step removes a plurality of detectably labeled oligonucleotides so that detectable moieties of one plurality of detectably labeled oligonucleotides do not interfere with detection of another plurality of detectably labeled oligonucleotides bound to targets. In some embodiments, a removing step removes at least 80% detectably labeled oligonucleotides. In some embodiments, a removing step removes at least 85% detectably labeled oligonucleotides. In some embodiments, a removing step removes at least 90% detectably labeled oligonucleotides. In some embodiments, a removing step removes at least 91% detectably labeled oligonucleotides. In some embodiments, a removing step removes at least 92% detectably labeled oligonucleotides. In some embodiments, a removing step removes at least 93% detectably labeled oligonucleotides. In some embodiments, a removing step removes at least 94% detectably labeled oligonucleotides. In some embodiments, a removing step removes at least 95% detectably labeled oligonucleotides. In some embodiments, a removing step removes at least 96% detectably labeled oligonucleotides. In some embodiments, a removing step removes at least 97% detectably labeled oligonucleotides. In some embodiments, a removing step removes at least 98% detectably labeled oligonucleotides. In some embodiments, a removing step removes at least 99% detectably labeled oligonucleotides. In some embodiments, a removing step removes at least 99.1% detectably labeled oligonucleotides. In some embodiments, a removing step removes at least 99.2% detectably labeled oligonucleotides. In some embodiments, a removing step removes at least 99.3% detectably labeled oligonucleotides. In some embodiments, a removing step removes at least 99.4% detectably labeled oligonucleotides. In some embodiments, a removing step removes at least 99.5% detectably labeled oligonucleotides. In some embodiments, a removing step removes at least 80% of the detectable signal. In some embodiments, a removing step removes at least 85% of the detectable signal. In some embodiments, a removing step removes at least 90% of the detectable signal. In some embodiments, a removing step removes at least 91% of the detectable signal. In some embodiments, a removing step removes at least 92% of the detectable signal. In some embodiments, a removing step removes at least 93% of the detectable signal. In some embodiments, a removing step removes at least 94% of the detectable signal. In some embodiments, a removing step removes at least 95% of the detectable signal. In some embodiments, a removing step removes at least 96% of the detectable signal. In some embodiments, a removing step removes at least 97% of the detectable signal. In some embodiments, a removing step removes at least 98% of the detectable signal. In some embodiments, a removing step removes at least 99% of the detectable signal. In some embodiments, a removing step removes at least 99.5% of the detectable signal. In some embodiments, a removing step removes 100% of the detectable signal. In some embodiments, after a removing step no signal can be detected by an imaging step.

A removing step optionally preserves targets (*e.g*., transcripts or DNA loci) for further use, for example, further detection or quantification by additional contacting and/or imaging steps. In some embodiments, a removing step preserves at least 80% targets. Percentage of preserved targets can be measured, for example, by comparing data collected before and after a removing step, optionally using the same contacting and imaging protocols. In some embodiments, a removing step preserves at least 85% targets. In some embodiments, a removing step preserves at least 90% targets. In some embodiments, a removing step preserves at least 91% targets. In some embodiments, a removing step preserves at least 92% targets. In some embodiments, a removing step preserves at least 93% targets. In some embodiments, a removing step preserves at least 94% targets. In some embodiments, a removing step preserves at least 95% targets. In some embodiments, a removing step preserves at least 96% targets. In some embodiments, a removing step preserves at least 97% targets. In some embodiments, a removing step preserves at least 98% targets. In some embodiments, a removing step preserves at least 99% targets.

Methods for removing detectably labeled oligonucleotides can include those known in the art. In some embodiments, a removing step comprising degrading a detectably labeled oligonucleotide. In some embodiments, a detectably labeled oligonucleotide is removed by enzymatic digestion. In some embodiments, a removing step comprising contacting a plurality of detectably labeled oligonucleotides with an enzyme that digests a detectably labeled oligonucleotide.

Suitable enzymes are widely used in the art. For example, depending on the type(s) of detectably labeled oligonucleotides and/or targets, either DNase or RNase can be used. In some embodiments, a detectably labeled oligonucleotide comprising a DNA sequence for detecting/quantifying a RNA target is digested by a DNase, *e.g*., DNase I. In some embodiments, a detectably labeled oligonucleotide comprising an RNA sequence for detecting/quantifying a DNA target is digested by a RNase. In some embodiments, a detectably labeled RNA oligonucleotide is used to target a DNA loci.

In some embodiments, a detectably labeled oligonucleotide interacts with its target through binding or hybridization to one or more intermediates, such as an oligonucleotide, that is bound, hybridized, or otherwise linked to the target. In some embodiments, a detectably labeled oligonucleotide interacts with a target through hybridization with an intermediate oligonucleotide hybridized to a target, wherein the intermediate oligonucleotide comprises a sequence complimentary to the target, and a sequence complementary to the detectably labeled oligonucleotide (overhang). In some embodiments, a removing step removes detectably labeled oligonucleotides, optionally keeping intermediate oligonucleotides intact. In some embodiments, a removing step removes detectably labeled oligonucleotides and keeps intermediate oligonucleotides intact. In some embodiments, detectably labeled oligonucleotides differ from intermediates in a chemical or enzymatic perspective, so that detectably labeled oligonucleotides can be selectively removed.

In some embodiments, a removing step comprises contacting the target molecule, the one or more intermediates, and the detectably labeled oligonucleotide with a solution comprising formamide, wherein the formamide is present in the solution at a concentration of about 40%, 41%, 42%, 43%, 44%, 45%, 46%, 47%, 48%, 49%, 50%, 51%, 52%, 53%, 54%, or 55% (v/v).

In some embodiments, a removing step comprises contacting the target molecule, the one one or more intermediates, and the detectably labeled oligonucleotide with a solution comprising urea, wherein urea is present in the solution at a concentration of about 2*M* to 5*M.* In some embodiments, the urea is present in the solution at a concentration of about 2*M* to 4*M.* In some embodiments, the urea is present in the solution at a concentration of about 2*M* to 3*M*. In some embodiments, the urea is present in the solution at a concentration of about 5*M,* 4.5*M*, 4*M,* 3.5*M,* 3*M,* 2.5*M,* or 2*M*.

In some embodiments, the removing step comprising contact between the target molecule, the one or more intermediates, and the detectably labeled oligonucleotide with a solution comprising formamide selectively disrupts the interaction between the detectably labeled oligonucleotide and the one or more intermediates. For example, the removing step can selectively disrupt the interaction between the detectably labeled oligonucleotide and the one or more intermediates without affecting the interaction between the target molecule and the one or more intermediates. In such embodiments, the one of more intermediates are able to remain bound to the target molecule while the detectable labeled oligonucleotide is detached, unbound, and/or removed from its interaction with the one or more intermediates.

In some embodiments, intermediate DNA oligonucleotides are used to hybridize against DNA loci, with an overhang sequence (*e.g.,* 20 nt) such that a readout probe comprising: (i) a nucleic acid sequence that includes a sequence complementary to the overhang sequence, and (ii) a detectable signal, can bind. In some embodiments, the readout probe comprises a nucleic acid sequence that is about 17 nucleotides or less in length. In some embodiments, the readout probe comprises a nucleic acid sequence that is about 17 nucleotides in length. In some embodiments, the readout probe comprises a nucleic acid sequence that is less than about 17 nucleotides or less in length. In some embodiments, the readout probe comprises a nucleic acid sequence that is between about 10 and 17 nucleotides in length. In some embodiments, the readout probe comprises a nucleic acid sequence that is between about 11 and 17 nucleotides in length. In some embodiments, the readout probe comprises a nucleic acid sequence that is between about 12 and 17 nucleotides in length. In some embodiments, the readout probe comprises a nucleic acid sequence that is between about 13 and 17 nucleotides in length. In some embodiments, the readout probe comprises a nucleic acid sequence that is between about 14 and 17 nucleotides in length. In some embodiments, the readout probe comprises a nucleic acid sequence that is between about 15 and 17 nucleotides in length. In some embodiments, the readout probe comprises a nucleic acid sequence that is less than about 10 nucleotides in length. In some embodiments, the readout probe comprises a nucleic acid sequence that is between about 5 and 10 nucleotides in length. In some embodiments, the readout probe comprises a nucleic acid sequence that is between about 6 and 9 nucleotides in length. In some embodiments, the readout probe comprises a nucleic acid sequence that is between about 7 to 8 nucleotides in length. In some embodiments, the readout probe comprises a nucleic acid sequence that is about 17, 16, 15, 14, 13, 12, 11, 10, 9, 8, 7, 6, or 5 nucleotides in length.

In some embodiments, intermediate DNA oligonucleotides are used to hybridize against DNA loci, with an overhang (*e.g.,* 20 nt) such that a bridge oligonucleotide comprising an RNA sequence and with complementary sequence (*e.g*., RNA bridge probe) can bind. An RNA bridge probe can be labeled directly with a dye or a HCR polymer (which can also be DNA). After imaging, RNase can be used to digest away the RNA bridge probes, while leaving the DNA probe intact hybridized on the DNA loci. Such a method provides multiple advantages. For example, subsequent contacting steps only involve RNA bridge probes hybridizing against DNA oligonucleotides with overhangs, and avoid getting double stranded DNA to melt and hybridize with DNA oligonucleotides, which can be a difficult process. Further, the overhang can be made to be the same for all DNA oligonucleotides (*e.g*., 20-40) targeting the same gene, so that only one type of RNA bridge probe is needed per gene per round of hybridization. To switch colors on different hybridization (contacting steps), one can change RNA bridge probes with a different label or different HCR polymer. DNA bridge probes that can be specifically removed, *e.g*., with a specific enzyme restriction site like EcoRI on the bridge or the HCR hairpins, can also be used. Incubating the cells with the appropriate nuclease can digest away all detectable moieties without affecting the DNA loci and/or the probe hybridized on them.

In some embodiments, detectably labeled oligonucleotides comprises 5' phosphorylation and can be degraded by Lambda exonuclease, while intermediate oligonucleotides are not 5'-phosphoralated and cannot be degraded by Lambda exonuclease.

In some embodiments, a detectably labeled oligonucleotide comprises uracil. In some embodiments, detectably labeled oligonucleotides contain uracil, and can be degraded by USER^{™} enzyme (New England BioLabs, Ipswich, Massachusetts, MA, US), while intermediate oligonucleotides contain no uracil and cannot be degraded by USER^{™} enzyme. In some embodiments, an oligonucleotide hybridized against an overhang of an intermediate oligonucleotide has a recessed 3'-end when hybridized against the overhang. Detectably labeled oligonucleotides with recessed 3'-end when hybridized against intermediate oligonucleotides can be selectively digested by Exonuclease III. Intermediate oligonucleotides which do not have recessed 3'-ends, or whose 3'-ends are in RNA-DNA duplexes, can be kept intact due to the much weaker activities of exonuclease III toward them.

In some embodiments, when an enzyme is involved, a removing step is performed at a temperature that produces optimal results. In some embodiments, a removing step is performed at about 37 °C. In some embodiments, a removing step is performed at room temperature. In some embodiments, digestion with Lambda exonuclease is conducted at about 37 °C. In some embodiments, digestion with USER^{™} enzyme is conducted at about 37 °C. In some embodiments, digestion with USER^{™} enzyme is conducted at room temperature. In some embodiments, digestion with Exonuclease III is conducted at about 37 °C. In some embodiments, digestion with Exonuclease III is conducted at room temperature.

In some embodiments, use of an intermediate oligonucleotide and an overhang sequence for detectably labeled oligonucleotide binding provides a variety of advantages. In some embodiments, kinetics of hybridization between an overhang sequence and a detectably labeled oligonucleotide is faster than that between an intermediate oligonucleotide and a target. In some embodiments, all intermediate oligonucleotides for a target comprise the same overhang sequence, and all detectably labeled oligonucleotides for a target comprises the same complimentary sequence for binding to the same overhang sequence. In some embodiments, hybridization between a set of detectably labeled oligonucleotides and a set of intermediate oligonucleotides is up to about 20-40 times faster than that between a set of an intermediate oligonucleotides and a set of targets. In some embodiments, hybridization between detectably labeled oligonucleotides and intermediate oligonucleotides can be done in 30 minutes, compared to, in some cases, up to about 12 hours for hybridization between intermediate oligonucleotides and targets.

In some embodiments, strand displacement is used in a removing step to remove a detectably labeled oligonucleotide. In some embodiments, heat is used to dissociate a detectably labeled oligonucleotide in a removing step.

In some embodiments, a removing step comprises photobleaching. In some embodiments, photobleaching destroys a dye, such as a fluorophore, of a detectably labeled oligonucleotide.

In some embodiments, a first and a second sets of detectably labeled oligonucleotides target different sequences of each target, and a removing step after a first imaging step is optional. For example, one strategy is to target the same RNA with different DNA probes (detectably labeled DNA oligonucleotides), such that the first plurality of probes can target one set of sequences on the RNA, and the second plurality of probes target a different set of sequences on the same RNA. On the first hybridization (contacting), the first plurality of probes is used. They can then be imaged and optionally photobleached or digested by DNase, or other methods of destroying either the oligos or the dyes. The second set of probes can be hybridized and imaged without interferences from the first set of probes.

In some embodiments, provide methods optionally comprise HCR, light sheet microscopy, CLARITY, or combinations thereof. In some embodiments, provided methods allow direct profiling of targets in a tissue, an organ or an organism. In some embodiments, an organ is a brain. In some embodiments, provided methods allow direct imaging of transcripts in intact brains or tissues. In some embodiments, provided methods further comprise HCR. In some embodiments, provided methods further comprise light sheet microscopy. In some embodiments, provided methods further comprise CLARITY.

The methods disclosed herein offer many advantages over methods used in the prior art. For example, in some embodiments, provided methods provide high-throughput at reasonable cost. In some embodiments, provided methods provide direct probing of target without transformation or amplification of a target. In some embodiments, provided methods enable quick scale up without the requirement of a large number of detectable labels. In some embodiments, provided methods can apply multiple labels to the same target and therefore increase signal intensity. In some embodiments, provided methods provide a combination of the advantages.

In some embodiments, provided herein are compositions comprising a plurality of detectably labeled oligonucleotides, for, *e.g.,* use in provided methods. Exemplary compositions include but are not limited to those described in exemplary method embodiments herein.

In some embodiments,provided herein are compositions comprising a plurality of detectably labeled oligonucleotides, each of which targets a nucleic acid and is labeled with a detectable moiety, so that the composition comprises at least:
(i) a first oligonucleotide targeting a first nucleic acid and labeled with a first detectable moiety; and
(ii) a second oligonucleotide targeting a second nucleic acid and labeled with a second detectable moiety.

In some embodiments,provided herein are compositions comprising a plurality of detectably labeled oligonucleotides, each of which targets a transcript or DNA locus and is labeled with a detectable moiety, so that the composition comprises at least:
(i) a first oligonucleotide targeting a first transcript or DNA locus and labeled with a first detectable moiety; and
(ii) a second oligonucleotide targeting a second transcript or DNA locus and labeled with a second detectable moiety.

In some embodiments,provided herein are kits comprising a plurality of detectably labeled oligonucleotides, each of which targets a transcript or DNA locus and is labeled with a detectable moiety, so that the kit comprises at least:
(i) a first oligonucleotide targeting a first transcript or DNA locus and labeled with a first detectable moiety;
(ii) a second oligonucleotide targeting a second transcript or DNA locus and labeled with a second detectable moiety.
(iii) a third oligonucleotide, optionally identical in sequence to the first oligonucleotide, targeting the first transcript or DNA locus and labeled with the first, the second or a third detectable moiety; and
(iv) a fourth oligonucleotide, optionally identical in sequence to the second oligonucleotide, targeting the second transcript or DNA locus, and labeled with the first, the second, the third or a fourth detectable moiety,
wherein either the third oligonucleotide is labeled with a different detectable moiety than the first oligonucleotide, or the fourth oligonucleotide is labeled with a different detectable moiety than the second oligonucleotide, or both.

In some embodiments, detectably labeled oligonucleotides targeting the same target (transcript or DNA locus) in a composition are labeled with moieties providing the same detectable signal, or detectable signals that cannot be differentiated in an imaging step. In some embodiments, detectably labeled oligonucleotides targeting the same target in a composition are labeled with the same detectable moiety.

In some embodiments, a detectable moiety is or comprises a fluorophore. In some embodiments, a detectable moiety is a fluorophore. Exemplary fluorophores are widely known and used in the art, for example but not limited to fluorescein, rhodamine, Alexa Fluors, DyLight fluors, ATTO Dyes, or any analogs or derivatives thereof.

In some embodiments, a first and a second detectably labeled oligonucleotides target different target. In some embodiments, a first and a second detectably labeled oligonucleotides target the same target. In some embodiments, detectably labeled oligonucleotides in a composition or a kit targets two or more targets, *e.g*., transcripts and/or DNA loci. In some embodiments, detectably labeled oligonucleotides in a composition or a kit targets two or more transcripts. In some embodiments, detectably labeled oligonucleotides in a composition or a kit targets two or more DNA loci. In some embodiments, detectably labeled oligonucleotides in a composition or kit targets at least 4 targets. In some embodiments, detectably labeled oligonucleotides in a composition or kit targets at least 9 targets. In some embodiments, detectably labeled oligonucleotides in a composition or kit targets at least 16 targets. In some embodiments, detectably labeled oligonucleotides in a composition or kit targets at least 25 targets. In some embodiments, detectably labeled oligonucleotides in a composition or kit targets at least 36 targets. In some embodiments, detectably labeled oligonucleotides in a composition or kit targets at least 50 targets. In some embodiments, detectably labeled oligonucleotides in a composition or kit targets at least 100 targets. In some embodiments, detectably labeled oligonucleotides in a composition or kit targets at least 200 targets. In some embodiments, detectably labeled oligonucleotides in a composition or kit targets at least 500 targets. In some embodiments, detectably labeled oligonucleotides in a composition or kit targets at least 1,000 targets. In some embodiments, detectably labeled oligonucleotides in a composition or kit targets at least 5,000 targets. In some embodiments, detectably labeled oligonucleotides in a composition or kit targets at least 10,000 targets. In some embodiments, detectably labeled oligonucleotides in a composition or kit targets at least 50,000 targets. In some embodiments, detectably labeled oligonucleotides in a composition or kit targets at least 100,000 targets. In some embodiments, detectably labeled oligonucleotides in a composition or kit targets at least 1,000,000 targets.

In some embodiments, a first and a second oligonucleotides have different oligonucleotide sequences. In some embodiments, a first and a second detectable moieties are different. In some embodiments, a first and a second detectable moieties are the same.

In some embodiments, a first and a second oligonucleotides share less than 5% sequence identity. In some embodiments, a first and a second oligonucleotides share less than 10% sequence identity. In some embodiments, a first and a second oligonucleotides share less than 20% sequence identity. In some embodiments, a first and a second oligonucleotides share less than 30% sequence identity. In some embodiments, a first and a second oligonucleotides share less than 40% sequence identity. In some embodiments, a first and a second oligonucleotides share less than 50% sequence identity. In some embodiments, a first and a second oligonucleotides share less than 60% sequence identity. In some embodiments, a first and a second oligonucleotides share less than 65% sequence identity. In some embodiments, a first and a second oligonucleotides share less than 68% sequence identity. In some embodiments, a first and a second oligonucleotides share less than 70% sequence identity. In some embodiments, a first and a second oligonucleotides share less than 80% sequence identity. In some embodiments, a first and a second oligonucleotides share less than 90% sequence identity.

In some embodiments, each oligonucleotide shares less than 5% sequence identity with any other oligonucleotide. In some embodiments, each oligonucleotide shares less than 10% sequence identity with any other oligonucleotide. In some embodiments, each oligonucleotide shares less than 20% sequence identity with any other oligonucleotide. In some embodiments, each oligonucleotide shares less than 30% sequence identity with any other oligonucleotide. In some embodiments, each oligonucleotide shares less than 40% sequence identity with any other oligonucleotide. In some embodiments, each oligonucleotide shares less than 50% sequence identity with any other oligonucleotide. In some embodiments, each oligonucleotide shares less than 55% sequence identity with any other oligonucleotide. In some embodiments, each oligonucleotide shares less than 60% sequence identity with any other oligonucleotide. In some embodiments, each oligonucleotide shares less than 65% sequence identity with any other oligonucleotide. In some embodiments, each oligonucleotide shares less than 68% sequence identity with any other oligonucleotide. In some embodiments, each oligonucleotide shares less than 70% sequence identity with any other oligonucleotide. In some embodiments, each oligonucleotide shares less than 80% sequence identity with any other oligonucleotide. In some embodiments, each oligonucleotide shares less than 90% sequence identity with any other oligonucleotide.

In some embodiments, a composition or kit comprises two or more detectably labeled oligonucleotides targeting the same target. In some embodiments, 5, 10, 20, 30, 40, 50 or more detectably labeled oligonucleotides target the same target.

Detectably labeled oligonucleotides can be of various suitable lengths. In someembodiments, a detectably labeled oligonucleotide is at least 15 base pairs in length. In some embodiments, a detectably labeled oligonucleotide is at least 16 base pairs in length. In some embodiments, a detectably labeled oligonucleotide is at least 17 base pairs in length. In some embodiments, a detectably labeled oligonucleotide is at least 18 base pairs in length. In some embodiments, a detectably labeled oligonucleotide is at least 19 base pairs in length. In some embodiments, a detectably labeled oligonucleotide is at least 20 base pairs in length. In some embodiments, a detectably labeled oligonucleotide is at least 21 base pairs in length. In some embodiments, a detectably labeled oligonucleotide is at least 22 base pairs in length. In some embodiments, a detectably labeled oligonucleotide is at least 23 base pairs in length. In some embodiments, a detectably labeled oligonucleotide is at least 24 base pairs in length. In some embodiments, a detectably labeled oligonucleotide is at least 25 base pairs in length. In some embodiments, a detectably labeled oligonucleotide is at least 26 base pairs in length. In some embodiments, a detectably labeled oligonucleotide is at least 27 base pairs in length. In some embodiments, a detectably labeled oligonucleotide is at least 28 base pairs in length. In some embodiments, a detectably labeled oligonucleotide is at least 29 base pairs in length. In some embodiments, a detectably labeled oligonucleotide is at least 30 base pairs in length. In some embodiments, a detectably labeled oligonucleotide is at least 35 base pairs in length. In some embodiments, a detectably labeled oligonucleotide is at least 40 base pairs in length. In some embodiments, a detectably labeled oligonucleotide is at least 50 base pairs in length. In some embodiments, a detectably labeled oligonucleotide is about 15-25 base pairs in length. In some embodiments, a detectably labeled oligonucleotide is about 20-30 base pairs in length. In some embodiments, a detectably labeled oligonucleotide is about 25-35 base pairs in length. In some embodiments, a detectably labeled oligonucleotide is about 30-40 base pairs in length. In some embodiments, a detectably labeled oligonucleotide is about 35-45 base pairs in length. In some embodiments, a detectably labeled oligonucleotide is about 40-50 base pairs in length. In some embodiments, a detectably labeled oligonucleotide is about 15-30 base pairs in length. In some embodiments, a detectably labeled oligonucleotide is about 20-30 base pairs in length. In some embodiments, a detectably labeled oligonucleotide is about 15-35 base pairs in length. In some embodiments, a detectably labeled oligonucleotide is about 20-35 base pairs in length.

In some embodiments, a plurality of detectably labeled oligonucleotides contains two detectable moieties. In some embodiments, a plurality of detectably labeled oligonucleotides contains three detectable moieties. In some embodiments, a plurality of detectably labeled oligonucleotides contains four detectable moieties. In some embodiments, a plurality of detectably labeled oligonucleotides contains five detectable moieties. In some embodiments, a plurality of detectably labeled oligonucleotides contains six detectable moieties. In some embodiments, a plurality of detectably labeled oligonucleotides contains seven detectable moieties. In some embodiments, a plurality of detectably labeled oligonucleotides contains eight detectable moieties. In some embodiments, a plurality of detectably labeled oligonucleotides contains nine detectable moieties. In some embodiments, a plurality of detectably labeled oligonucleotides contains ten detectable moieties.

In some embodiments, a plurality of detectably labeled oligonucleotides comprises at least two detectable moieties. In some embodiments, a plurality of detectably labeled oligonucleotides comprises at least three detectable moieties. In some embodiments, a plurality of detectably labeled oligonucleotides comprises at least four detectable moieties. In some embodiments, a plurality of detectably labeled oligonucleotides comprises at least five detectable moieties. In some embodiments, a plurality of detectably labeled oligonucleotides comprises at least six detectable moieties. In some embodiments, a plurality of detectably labeled oligonucleotides comprises at least seven detectable moieties. In some embodiments, a plurality of detectably labeled oligonucleotides comprises at least eight detectable moieties. In some embodiments, a plurality of detectably labeled oligonucleotides comprises at least nine detectable moieties. In some embodiments, a plurality of detectably labeled oligonucleotides comprises at least ten detectable moieties.

In some embodiments, a composition further comprises:
(iii) a third oligonucleotide, optionally identical in sequence to the first oligonucleotide, targeting the first transcript or DNA locus; and
(iv) a fourth oligonucleotide, optionally identical in sequence to the second oligonucleotide , targeting the second transcript or DNA locus
wherein either the third oligonucleotide is labeled with a different detectable moiety than the first oligonucleotide, or the fourth oligonucleotide is labeled with a different detectable moiety than the second oligonucleotide, or both.

In some embodiments, a third oligonucleotide is identical in sequence to a first oligonucleotide. In some embodiments, a third oligonucleotide comprises a sequence overlapping with a first oligonucleotide. In some embodiments, a third oligonucleotide has less than 50% sequence identity with a first oligonucleotide. In some embodiments, a third oligonucleotide has less than 40% sequence identity with a first oligonucleotide. In some embodiments, a third oligonucleotide has less than 30% sequence identity with a first oligonucleotide. In some embodiments, a third oligonucleotide has less than 20% sequence identity with a first oligonucleotide. In some embodiments, a third oligonucleotide has less than 10% sequence identity with a first oligonucleotide. In some embodiments, a third oligonucleotide has less than 5% sequence identity with a first oligonucleotide.

In some embodiments, a fourth oligonucleotide is identical in sequence to a second oligonucleotide. In some embodiments, a fourth oligonucleotide comprises a sequence overlapping with a second oligonucleotide. In some embodiments, a fourth oligonucleotide has less than 50% sequence identity with a second oligonucleotide. In some embodiments, a fourth oligonucleotide has less than 40% sequence identity with a second oligonucleotide. In some embodiments, a fourth oligonucleotide has less than 30% sequence identity with a second oligonucleotide. In some embodiments, a fourth oligonucleotide has less than 20% sequence identity with a second oligonucleotide. In some embodiments, a fourth oligonucleotide has less than 10% sequence identity with a second oligonucleotide. In some embodiments, a fourth oligonucleotide has less than 5% sequence identity with a second oligonucleotide.

In some embodiments, a third oligonucleotide is labeled with a different detectable moiety than the first oligonucleotide. In some embodiments, a fourth oligonucleotide is labeled with a different detectable moiety than the second oligonucleotide.

In some embodiments, amount of a detectably labeled oligonucleotide in a plurality, composition, kit or method is pre-determined. In some embodiments, amounts of 5% detectably labeled oligonucleotides in a plurality, composition, kit or method are pre-determined. In some embodiments, amounts of 10% detectably labeled oligonucleotides in a plurality, composition, kit or method are pre-determined. In some embodiments, amounts of 20% detectably labeled oligonucleotides in a plurality, composition, kit or method are pre-determined. In some embodiments, amounts of 30% detectably labeled oligonucleotides in a plurality, composition, kit or method are pre-determined. In some embodiments, amounts of 40% detectably labeled oligonucleotides in a plurality, composition, kit or method are pre-determined. In some embodiments, amounts of 50% detectably labeled oligonucleotides in a plurality, composition, kit or method are pre-determined. In some embodiments, amounts of 60% detectably labeled oligonucleotides in a plurality, composition, kit or method are pre-determined. In some embodiments, amounts of 70% detectably labeled oligonucleotides in a plurality, composition, kit or method are pre-determined. In some embodiments, amounts of 80% detectably labeled oligonucleotides in a plurality, composition, kit or method are pre-determined. In some embodiments, amounts of 90% detectably labeled oligonucleotides in a plurality, composition, kit or method are pre-determined.

In some embodiments, amounts of at least 5 detectably labeled oligonucleotides in a plurality, composition, kit or method are pre-determined. In some embodiments, amounts of at least 10 detectably labeled oligonucleotides in a plurality, composition, kit or method are pre- determined. In some embodiments, amounts of at least 20 detectably labeled oligonucleotides in a plurality, composition, kit or method are pre-determined. In some embodiments, amounts of at least 30 detectably labeled oligonucleotides in a plurality, composition, kit or method are pre- determined. In some embodiments, amounts of at least 40 detectably labeled oligonucleotides in a plurality, composition, kit or method are pre-determined. In some embodiments, amounts of at least 50 detectably labeled oligonucleotides in a plurality, composition, kit or method are pre- determined. In some embodiments, amounts of at least 60 detectably labeled oligonucleotides in a plurality, composition, kit or method are pre-determined. In some embodiments, amounts of at least 70 detectably labeled oligonucleotides in a plurality, composition, kit or method are pre- determined. In some embodiments, amounts of at least 80 detectably labeled oligonucleotides in a plurality, composition, kit or method are pre-determined. In some embodiments, amounts of at least 90 detectably labeled oligonucleotides in a plurality, composition, kit or method are pre- determined. In some embodiments, amounts of at least each detectably labeled oligonucleotides in a plurality, composition, kit or method is pre-determined.

In some embodiments, two or more detectably labeled oligonucleotides are provided for one target. In some embodiments, total amount of all detectably labeled oligonucleotides for a target is pre-determined. In some embodiments, total amount of all detectably labeled oligonucleotides for a target is pre-determined, wherein the amount of each of the detectably labeled oligonucleotide for the target is independently and optionally pre-determined. In some embodiments, total amount of all detectably labeled oligonucleotides for each of a plurality of targets is independently pre-determined. In some embodiments, a plurality of targets has at least two targets. In some embodiments, a plurality of targets has at least five targets. In some embodiments, a plurality of targets has at least 10 targets. In some embodiments, a plurality of targets has at least 50 targets. In some embodiments, a plurality of targets has at least 100 targets. In some embodiments, a plurality of targets has at least 500 targets. In some embodiments, a plurality of targets has at least 1,000 targets.

In some embodiments, a target of a plurality, composition, kit or method is pre-determined. In some embodiments, at least 10 targets of a plurality, composition, kit or method are pre-determined. In some embodiments, at least 50 targets of a plurality, composition, kit or method are pre-determined. In some embodiments, at least 100 targets of a plurality, composition, kit or method are pre-determined. In some embodiments, at least 1,000 targets of a plurality, composition, kit or method are pre-determined. In some embodiments, up to *F^{N}* targets of a plurality, composition, kit or method are pre-determined, wherein F is the number of detectable moieties in a pluralities, and *N* is the number of imaging steps.

Methods for synthesizing detectably labeled oligonucleotides are widely known and practiced in the art, for example, see Lubeck, E. & Cai, L. Nat. Methods 9, 743-48 (2012). Oligonucleotides are also commercially available from various vendors. In some embodiments, the methods disclosed herein can be used for preparing detectably labeled oligonucleotides. In some embodiments, the methods disclosed herein can be used for preparing intermediate oligonucleotides. In some embodiments, the methods disclosed herein can be used for preparing bridge oligonucleotides.

In some embodiments, provided herein are methods for preparing a target nucleic acid having a first sequence, comprising steps of:
1) providing a first nucleic acid comprising the first sequence, wherein the first sequence is flanked by nicking endonuclease sites at both ends;
2) amplifying the first nucleic acid or part of the first nucleic acid to provide a second nucleic acid comprising the first sequence and the flanking nicking endonuclease sites; and
3) contacting the second nucleic acid with one or more nicking endonuclease corresponding to the flanking nicking endonuclease sites.

In some embodiments, a target nucleic acid having a first sequence is single-stranded. In some embodiments, an amplifying step comprises polymerase chain reaction (PCR). In some embodiments, provided methods further comprise a step of denaturing, wherein doublestranded second nucleic acid is denatured and the two strands become single-stranded. In some embodiments, provided methods further comprise isolating the nucleic acid having a first sequence. In some embodiments, a second nucleic acid is optionally modified before contacting with nicking endonucleases. In some embodiments, provided methods further comprise labeling a nucleic acid having a first sequence.

In some embodiments, the two flanking endonuclease sites are the same. In some embodiments, one nicking endonuclease corresponding to the same nicking endonuclease sites is used. In some embodiments, the two flanking endonuclease sites are different. In some embodiments, two nicking endonucleases, each of which independently corresponds to a nicking endonuclease site, are used.

In some embodiments, oligonucleotides of provided technologies are generated from oligonucleotide pools. In some embodiments, such pools are available commercially. An initial DNA oligonucleotide pool in some embodiments consists of up to 12,000 or more different single stranded sequences organized into subsets. Each sequence is designed such that nicking endonuclease sites and a forward and reverse primer sequence flank a desired sequence (*e.g*., a probe sequence). The forward and reverse primer sequences specify to which subset with the desired sequence belongs. The primer pair can be used to amplify the subset using polymerase chain reaction (PCR). The product of the PCR reaction is isolated and digested by the nicking endonucleases. The incubation time with the nicking enzyme varies based on the amount of enzyme used and the amount of DNA recovered. In some embodiments, about 10 units of enzyme digest about 1 µg of DNA in about 1 hour. The sample is then purified and reconstituted in a buffer, *e.g.,* 2x loading buffer (96% formamide/20mM EDTA) and water to make a final loading buffer (48% formamide/10mM EDTA), and denatured, e.g., by heating to 95 °C to completely denature the DNA. The denatured DNA is purified and the desired product isolated. In some embodiments, purification and/or isolation comprise electrophoresis. An exemplary process is illustrated in Figure 25.

In some embodiments, provided herein is a method for preparing a target nucleic acid having a first sequence, comprising steps of:
1) providing a first nucleic acid comprising the first sequence or its complimentary sequence, wherein the first sequence or its complementary sequence is flanked by at least one restriction site;
2) amplifying the first nucleic acid or part of the first nucleic acid to provide a second nucleic acid comprising the first sequence and the at least one flanking restriction site; and
3) contacting the second nucleic acid with a restriction enzyme corresponding to the at least one flanking restriction site to provide a third nucleic acid comprising a recessed end;
4) contacting the third nucleic acid with a nuclease to selectively digest the strand comprising the complementary sequence, if any, while keeping the strand comprising the first sequence.

In some embodiments, the first sequence or its complementary sequence is independently flanked by a restriction site at each end.

In some embodiments, provided herein is a method for preparing a target nucleic acid having a first sequence, comprising steps of:
1) providing a first nucleic acid comprising the first sequence or its complimentary sequence, wherein the first sequence or its complementary sequence is flanked by restriction sites at both ends;
2) amplifying the first nucleic acid or part of the first nucleic acid to provide a second nucleic acid comprising the first sequence and the flanking restriction sites; and
3) contacting the second nucleic acid with restriction enzymes corresponding to the flanking restriction sites to provide a third nucleic acid comprising a recessed end;
4) contacting the third nucleic acid with a nuclease to selectively digest the strand comprising the complementary sequence, if any, while keeping the strand comprising the first sequence.

In some embodiments, a target nucleic acid having a first sequence is single-stranded. In some embodiments, an amplifying step comprises PCR. In some embodiments, provided methods further comprise isolating the nucleic acid having a first sequence. In some embodiments, a second nucleic acid is optionally modified before contacting with restriction enzymes. In some embodiments, a third nucleic acid is optionally modified before contacting with a nuclease. In some embodiments, a nuclease is exonuclease III, which preferentially degrade a strand with 3'-recessed ends, and can preserve a strand with a 5' recessed ends. In some embodiments, a restriction enzyme creates a 5'-recessed end. In some embodiments, a restriction enzyme creates a 3'-recessed end. In some embodiments, the complementary sequence has a 3' recessed end after restriction digestion. In some embodiments, the strand comprising the complementary sequence has a 3' recessed end after restriction digestion, and the strand comprising a first sequence has a 5' recessed end after restriction digestion. In some embodiments, provided methods further comprise labeling a nucleic acid having a first sequence.

In some embodiments, single stranded oligonucleotides, *e.g*., probes for seqFISH or intermediate oligonucleotides, can be generated using nuclease digestion, such as exoIII nuclease digestion. Instead of two nick sites on the amplification (*e.g.*, PCR) products, two restriction sites can be used flanking the probe and/or adaptor sequence. In some embodiments, one restriction site leaves a 3' recessed end while the other leaves a 5' recessed ends. For example, EcoRI and BamHI leave 5' recessed ends, while BmtI and PacI leave 3' recessed ends. Such restriction enzymes are widely known and used in the art. Exonuclease III degrades the 3' recessed ends preferentially, and preserve the strand with the 5' recessed ends. This provides another mechanism to generate single stranded probes from oligonucleotide pools using PCR and restriction nucleases.

In some embodiments, a provided target nucleic acid is DNA. In some embodiments, a target nucleic acid has the same sequence a first sequence. In some embodiments, a target nucleic acid is an intermediate oligonucleotide, comprising a first sequence that hybridizes to a target, *e.g.,* a transcript or a DNA locus, and a second sequence that hybridizes to a second oligonucleotide, *e.g*., a detectably labeled oligonucleotide. In some embodiments, a target nucleic acid is an intermediate oligonucleotide, comprising a first sequence that hybridizes to a target, and a second sequence that hybridizes with a detectably labeled oligonucleotide labeled by HCR. In some embodiments, a target nucleic acid is a bridge probe.

In some embodiments, provided methods are used for diagnosis of a disease, wherein the disease is related to an abnormal number of a transcript or a DNA locus. In some embodiments, provided methods are used for selecting subjects for a treatment. In some embodiments, provided methods are used for monitoring a treatment regimen. In some embodiments, a cell in provide methods is from a subject. In some embodiments, a cell in provide methods is a mammalian cell. In some embodiments, a cell in provide methods is a human cell. In some embodiments, a cell in provide methods is from a subject. In some embodiments, a cell in provide methods is from an animal. In some embodiments, a cell in provide methods is from a human subject. In some embodiments, a cell in provide methods is isolated from a human subject. In some embodiments, a cell in provide methods is from a diseased tissue, or a tissue that is susceptible to a disease. Being capable of detecting and quantifying a number of targets at the same time, provided methods provides significant advantages for diagnosis, treatment monitoring and patient stratification.

In some embodiments, provided technologies optionally comprises profiling proteins, neural activities, and/or structural arrangements. In some embodiments, provided methods comprise profiling proteins in the same sample. In some embodiments, provided methods comprise profiling neural activities in the same sample. In some embodiments, provided method comprise profiling structural arrangement.

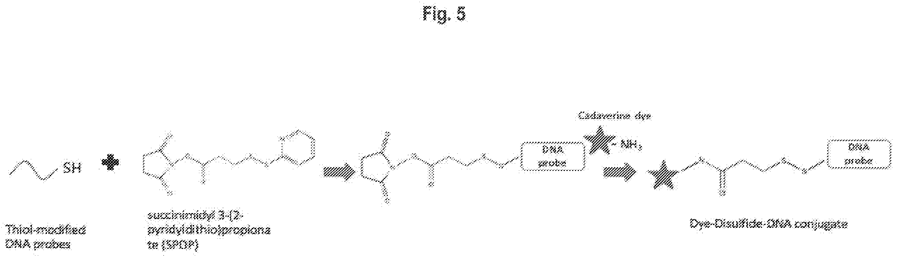

In one aspect, disclosed herein are readout probes with cleavable linkers. FIG. 5 depicts exemplary chemical reactions for synthesizing a readout probe with a disulfide linker.

In one aspect, sequential barcoding FISH (seqFISH) is performed by using nucleic acid readout probes that are conjugated with a signal moiety via a cleavable linker. Any suitable cleavable linkers can be used, including but not limited to an enzyme cleavable linker, a nucleophile/base sensitive linker, reduction sensitive linker, a photo-cleavable linker, an electrophile/acid sensitive linker, a metal-assisted cleavable linker, or an oxidation sensitive linker. Exemplary linkers can be found in Leriche et al., 2012, "Cleavable linkers in chemical biology," Bioorganic & Medicinal Chemistry 20:571-582, which is hereby incorporated herein in its entirety.

In some embodiments, the cleavable linker is a disulfide linkage. In some embodiments, the cleavable linker is a nucleic acid restriction site. In some embodiments, the cleavable linker is a protease cleavage site.

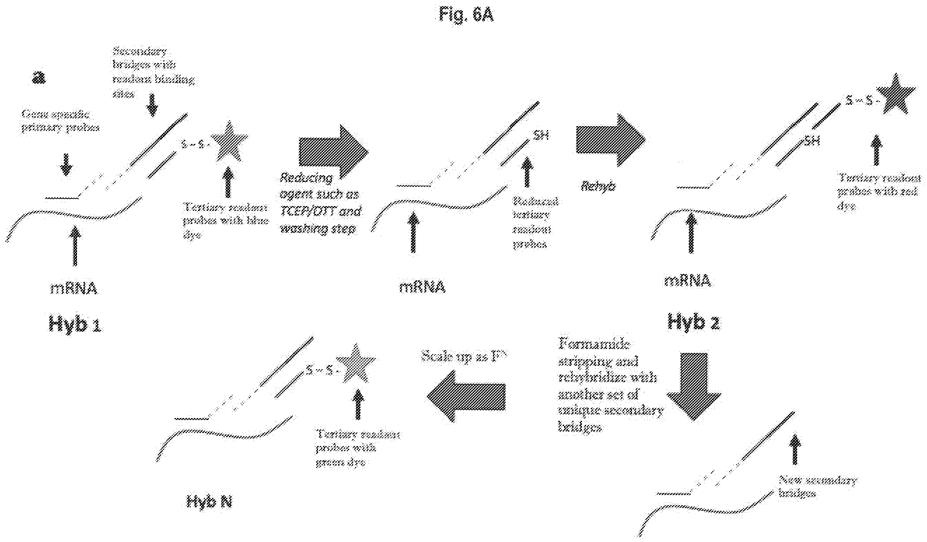

An exemplary system utilizing nucleic acid readout probes is shown in FIG. 6A. As depicted, a gene specific primary probe binds to a target site, *e.g.,* in an mRNA molecule under an *in situ* or *in vitro* setting. In the exemplary embodiment illustrated in FIG. 6A, sequential barcoding is carried out using gene-specific primary probes, secondary bridge probes and tertiary readout probes. For example, sequential barcoding FISH (seqFISH) is carried out with DNA readout probes conjugated with dyes through disulfide linkage. The method involves hybridization of gene-specific primary probes, followed by secondary bridge probes with readout binding sites, and a unique tertiary readout probes with disulfide-linked dye. Once imaged, a reducing agent such as TCEP/DTT can be used to eliminate the fluorescent signals. Subsequent hybridization provides fluorescent signals whose signals are not interfered with by fluorescent signals from prior rounds of hybridization. The secondary bridge probes can be stripped off by a removal step as disclosed herein (*e.g.,* a formamide solution), and replaced by a new set of secondary bridge probes. Besides a binding sequence, the primary probe further includes an overhang sequence at one end of the binding sequence. In some embodiments, a second overhang sequence is included at the other end of the binding sequence.

In some embodiments, an overhang sequence includes one or more target sequences to which one or more nucleic acid readout probes bind. In some embodiments, each target sequence uniquely interacts with a set of readout probes with specific readout binding sequences. As disclosed herein, an overhang sequence may include two target sequences, three target sequences, five or fewer target sequences, seven or fewer target sequences, or ten or fewer target sequences. In some embodiments, an overhang sequence may include ten or more target sequences. Similar arrangements can be implemented where there are two overhang sequences.

In some embodiments, an overhang sequence binds to a bridge probe that provides target sequences for one or more readout probes to bind, as depicted in FIG. 6A. A bridge probe can be interchangeably called an intermediate bridge probe or a secondary bridge probe. A bridge probe includes a binding sequence that binds to all or a portion of an overhang sequence in a primary probe. In some embodiments, a bridge probe further includes one or more readout binding targets that are connection in series and linked to the binding sequence.

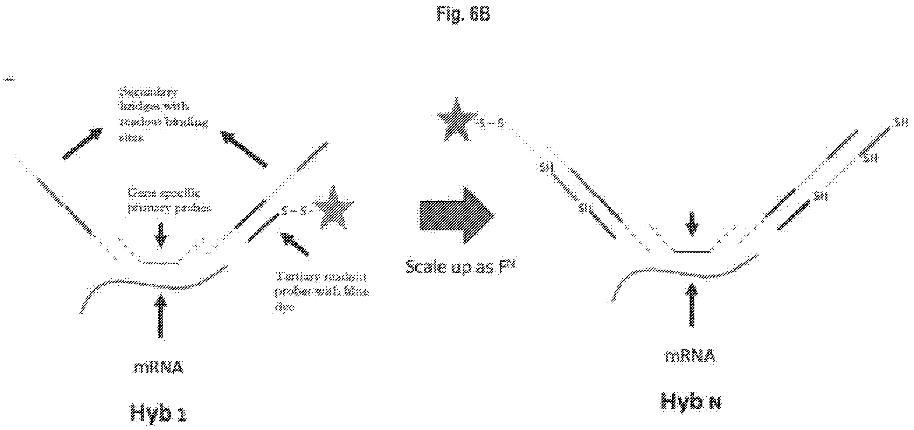

In some embodiments, as depicted in FIG. 6B, two bridge probes can bind to the same primary probe via two overhang sequences. For example, in a primary probe having two overhang sequences, each overhang sequence can bind to a secondary bridge probe comprising unique tertiary readout probe binding sites. In this illustration, each secondary bridge probe comprises three (3) unique tertiary readout probe binding sites. However, a secondary bridge probe can comprise any number of unique tertiary readout probe binding sites, e.g., from one up to ten or more readout probe binding sites. For example, a secondary bridge probe can comprise 1, 2, 3, 4, 5, 6, 7, 8, 9, 10, 11, 12, 13, 14, 15, 16, 17, 18, 19, or 20 or more tertiary readout probe binding sites, In the example illustrated in FIG. 6B, four (4) different colors of fluorophore are employed. By employing four different fluorophore colors, one can scale up the number of barcodes to 46 = 4096 barcodes with this design.

As disclosed herein, a bridge probe may include two readout binding targets, three readout binding targets, five or fewer readout binding targets, seven or fewer readout binding targets, or ten or fewer readout binding targets. In some embodiments, an overhang sequence may include ten or more readout binding targets. Similar arrangements can be implemented where there are two bridge probes bound to overhang sequences.

Exemplary rehybridization schemes utilizing the readout probes are illustrated in FIGs. 6A and 6B. For example, the first round of rehybridization (hyb1) begins with the hybridization of gene specific primary probes to the target mRNA. Each gene specific primary probes contains one or more "overhang" sequences to which the secondary bridge probes can hybridize. The secondary bridges contain two or more tertiary readouts binding sites which is the key to efficient and quick rehybridization. In the first hybridization, unique tertiary readout probes conjugated with blue dye are hybridized to their unique binding sites on the secondary bridge probe. Once imaged, the sample is treated with reducing agent such as TCEP or DTT to cleave off the disulfide-linked dyes. Then, the sample is washed with wash buffers. During the second round of hybridization, a second set of unique tertiary readout probes with red dye is hybridized to its unique binding site on the secondary bridge. After two rounds of hybridizations, a particular mRNA is then barcoded with a color barcode of red and blue. Additional rounds of hybridization can be applied to create more sophisticated barcoding sequences. Technically, the scaling factor of seqFISH with this rehybridization method depends on the number of available secondary bridges with its number of unique tertiary probes binding sites. For example, by incorporating 2 secondary bridges with total 8 unique tertiary readout binding sites (N=8) , and with 4 fluorophores (F=4), one can generate up over 64,000 unique barcodes (F^{N} = 4⁸ = 65,536). Moreover, in embodiments where bridge probes are used, it is possible to strip off the secondary bridges with high concentration of formamide, and flow in another unique set of secondary bridges to continue the scaling process, which further increases the upper limit of the scaling factor.

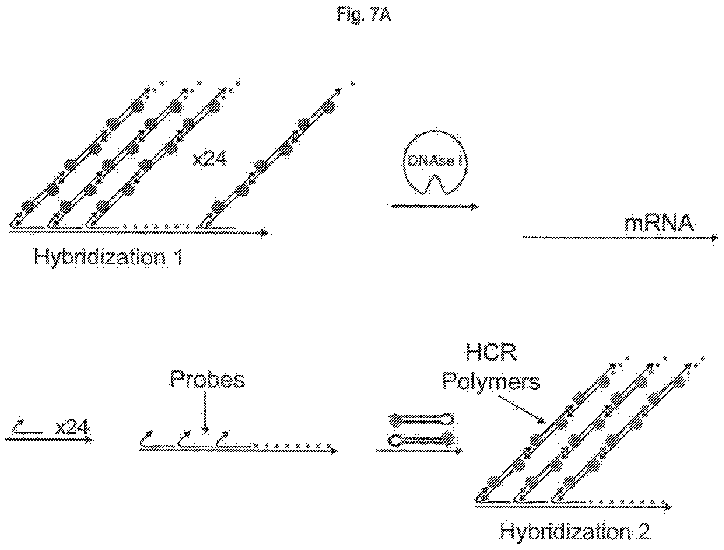

In one aspect, disclosed herein are methods and systems for amplifying visual signals during each round of hybridization during sequential hybridization reactions, based on hybridization chain reaction (HCR). An exemplary embodiment of HCR is illustrated in FIG. 7A. During hybridization round 1, probes with overhang initiator sequences are added to a nucleic acid target molecule such as an mRNA or a DNA. Also added are hairpin nucleic acid probes bearing sequences complementary to those of the initiator sequences. The presence of initiator sequences cause unfolding of the hairpin nucleic acid probes and result in chain reactions that lead to self-assembled extended HCR polymers. Because each hairpin nucleic acid probe bears a signal, self-assembled extended HCR polymers result in amplification of signals and better detection of target sites.

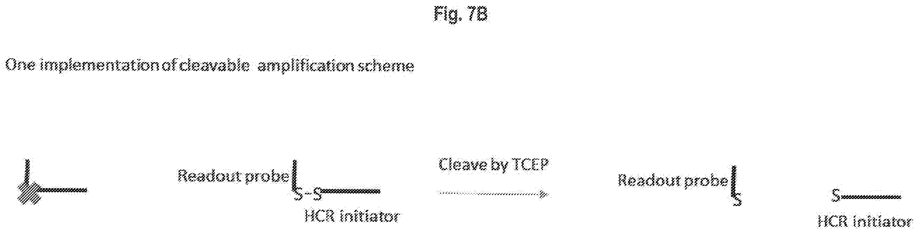

FIG. 7B illustrates an exemplary readout probe embedded with a cleavable linker. Here, the cleavable linker is a disulfide bond. At one end of the cleavable linker, a readout probe as disclosed herein includes a binding sequence that allows it to bind to a specific nucleic acid target. In some embodiments, the nucleic acid target is an mRNA or a DNA. In some embodiments, the nucleic acid target is within an intact cell or as part of cell extract. In some embodiments, the nucleic acid target is within a primary binding probe that directly binds to a target site in an mRNA. In some embodiments, the nucleic acid target is within a secondary binding probe that binds to a primary binding probe that directly binds to a target site in an mRNA. In some embodiments, the nucleic acid target is within a tertiary or quaternary binding probe. One of skill in the art can apply the principle to any level of binding and interaction.

At the other end of the cleavable linker, a readout probe as disclosed herein further includes an HCR initiator sequence. When exposed to hairpin nucleic acids bearing partial or complete complementary sequences, the initiator sequence can trigger a chair reaction that allows a signal motif formed by multiple extender probes. Each extender probe includes a signal moiety. Aggregation of multiple extender probes enhances signal detection.

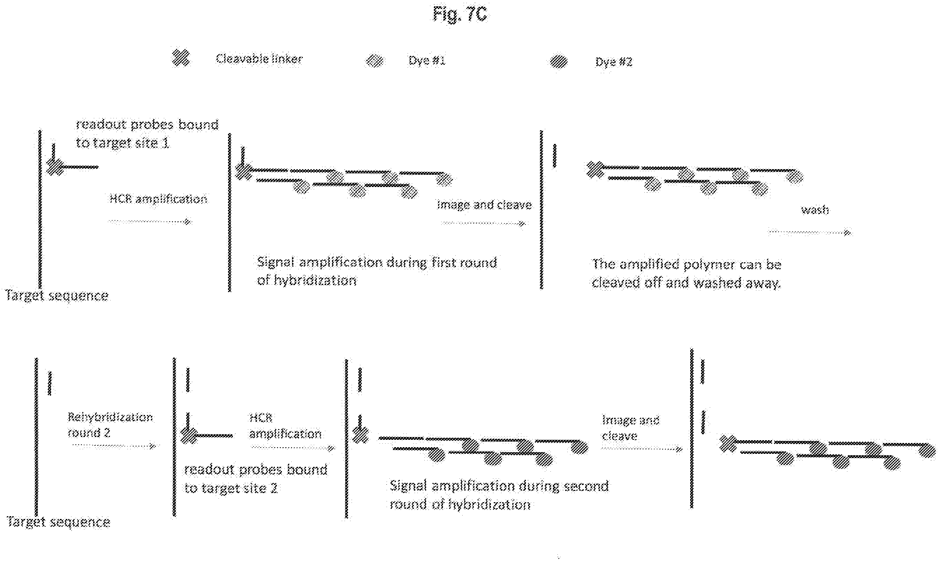

An exemplary scheme for forming a signal motif with multiple extender probes during a sequential hybridization process is illustrated in FIG. 7C. During the first round of hybridization, nucleic acid detection probes with embedded cleavable linkers binds to a first target site within a nucleic acid target sequence. In some embodiments, extender probes are added after the initial binding of nucleic acid detection probes to the first target sequences. In some embodiments, extender probes form an aggregate before the aggregated polymer is added to the reaction mix and binds to the imitator sequence in the nucleic acid detection probes.

In some embodiments, extender probes are standard hairpin probes each including a sequence that is partly or completely complementary to the initiator sequence in the readout probes. In these embodiments, extender probes are very similar or identical to each other. The size of the resulting extendible signal motif may be controlled by the concentration or absolute quantity of the extender probes added.

In some embodiments, extender probes including different types of nucleic acid sequences can be used to achieve controlled signal amplification. For example, the signal can be amplified five times if five populations of extender probes are used: {EP₁, EP₂, EP₃, EP₄, and EP₅}. The first population of extender probes includes a binding sequence that binds to all or a part of the initiator sequence. The second population of extender probes includes a binding sequence that binds to a region in the first population of extender sequence. The third population of extender probes includes a binding sequence that binds to a region in the second population of extender sequence. The fourth population of extender probes includes a binding sequence that binds to a region in the third population of extender sequence. The fifth population of extender probes includes a binding sequence that binds to a region in the fourth population of extender sequence. In such embodiments of linear amplification, the size of the resulting extendible signal motif can be controlled by the number of populations of extender probes that are provided.

In some embodiments, an extender probe may include multiple binding sites for binding subsequent extender probes. For example, besides binding to the initiator sequence, EP₁ may include two or more binding sites for EP₂, thus allowing further amplification of the signal. This form of amplification may occur at any level. For example, in the example above, multiple binding sites for subsequent or downstream extender probes can be implemented in any one or combinations of EP₁, EP₂, EP₃, or EP₄. For example, extender probes from EP₂, EP₃, or EP₄ can all bind to target sites in EP₁, which in turn binds to the initiator sequence.

In some embodiments, the amplification occurs at multiple levels. Generally, when m populations of extender probes are present, multiple binding sites for subsequent or downstream extender probes cam be implemented in any one or combinations of EP₁, EP₂, ..., or EPₘ₋₁. Additionally, when multiple binding sites are present, they can be connected in series or arranged in a non-linear fashion (e.g., in a branched or circular arrangement). Depending on the number and configuration of the binding sites, the resulting extendible signal motif can be a stick, a ball, a net or in any other applicable form.

One of skill in the art would understand that any suitable number of populations of extender probes can be added to achieve an optimal signal to noise ratio for the best imaging effects. For example, the extender probes can include five or fewer, seven or few, 10 or fewer, 15 or fewer, 20 or fewer, 25 or fewer, 30 or fewer, 40 or fewer, 50 or fewer populations.

In some embodiments, the extender probes are mixed together prior to being mixed with the readout probes having the initiator sequence. In some embodiments, the extender probes are sequentially added to the readout probes having the initiator sequence where the readout probes are already bound to its nucleic acid targets.

As shown in FIG. 7C, after imaging analysis, a cleaving agent can be applied to sever the linker between the binding sequence and the imitator sequence in a readout probe. The amplified polymers can then be cleaved off and washed away.

During a second round of rehybridization, new nucleic acid detection probes are applied. The new nucleic acid detection probes include a different binding sequence that binds to a second and different target site in the nucleic acid target sequence. The new nucleic acid detection probes also include a cleavable linker and an initiator sequence. The initiator sequence can be the same as or different from the initiator sequence from the previous set of nucleic acid detection probes.

The new extender probes are used, as described hereinabove, to form amplified polymers to enhance signal detection. After imaging analysis, the new set of amplified polymers can be cleaved off and washed away. By using extender probes bearing a different type of visual signals, barcodes can be established for nucleic acid targets. Depending on the availability of target sites within a nucleic acid target, multiple rounds of hybridizations can be performed to create more complex barcodes. For example, there can be three rounds of hybridizations, four rounds of hybridizations, five rounds of hybridizations, seven or fewer rounds of hybridizations, 10 or fewer rounds of hybridizations, 12 or fewer rounds of hybridizations, 15 or fewer rounds of hybridizations, 20 or fewer rounds of hybridizations, 30 or fewer rounds of hybridizations, 40 or fewer rounds of hybridizations, or 50 or fewer rounds of hybridizations.

The compositions and methods disclosed herein can be used in sequential hybridizations to identify any suitable cellular targets within an intact cell or in an *in vitro* setting. In some embodiments, the cellular targets can be mRNAs or DNAs. In some embodiments, the cellular targets can be proteins. For example, the initial target-binding primary probe can be an antibody conjugated with nucleic acid sequence for subsequent bindings.

The methods disclosed herein are applicable for a wide variety of samples. For example, HCR-seqFISH worked in brain slices and that SPIMs can robustly detect single mRNAs in CLARITY brain slices. In some embodiments, provided technologies are useful for profiling targets in mouse models of neurodegenerative diseases, or human brains. No other technology prior to the methods and compositions disclosed herein can deliver the same quality and quantity of data.

### EXAMPLES

The foregoing has been a description of certain non-limiting embodiments of the invention. Accordingly, it is to be understood that the embodiments of the invention herein described are merely illustrative of the application of the principles of the invention. Reference herein to details of the illustrated embodiments is not intended to limit the scope of the claims. Additional examples are described, e.g., in U.S. Patent Publication No. 2016-0369329.

### EXAMPLE 1

### IN SITU PROFILING OF NUCLEIC ACIDS BY SEQUENTIAL HYBRIDIZATION AND BARCODING

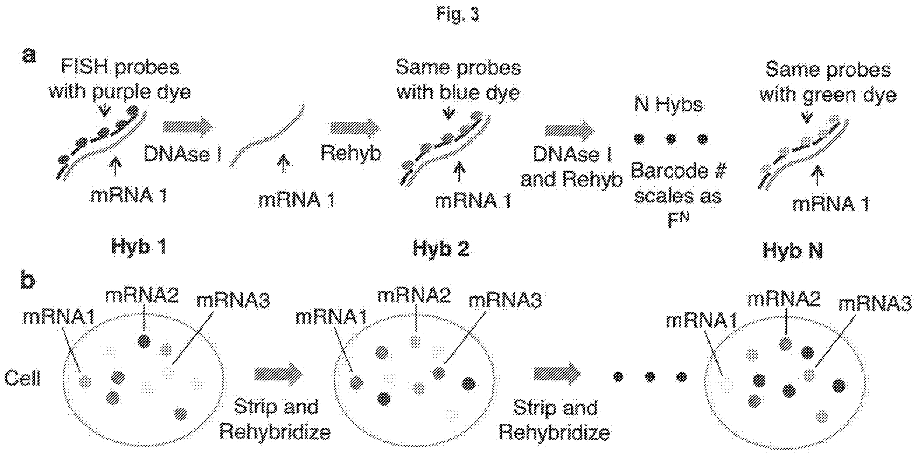

As described in the non-limiting examples herein, nucleic acids in cells, for example, mRNAs, were profiled by provided methods through sequential rounds of contacting, imaging and removing steps (FIGs. 2(a) and 3). As the transcripts are fixed in cells, the corresponding fluorescent spots remain in place during multiple rounds of hybridization, and can be aligned to read out a fluorophore sequence. This sequential barcode is designed to uniquely identify an mRNA.

During each round of hybridization, each transcript was targeted by a set of detectably labeled oligonucleotides, in this case, FISH probes labeled with a single type of fluorophore. The sample was imaged and then treated it with DNase I to remove the FISH probes. In a subsequent round the mRNA was hybridized with FISH probes with the same set of oligonucleotide sequences, but now labeled with a different dye. The number of barcodes available scales as *F^{N}*, where *F* is the number of fluorophores and *N* is the number of hybridization rounds. For example, with 4 dyes, 8 rounds of hybridization can cover almost the entire transcriptome (48=65,536).

In some embodiments, to distinguish different mRNA species, mRNAs are barcoded with detectably labeled oligonucleotides, such as FISH probes using sequential rounds of hybridization. During a round of hybridization, each transcript is targeted by a set of multiple, for example, 24 FISH probes, labeled with a single type of fluorophore. The sample is imaged and the FISH probes are removed by enzymatic digestion. Then the mRNA is hybridized in a subsequent round with the same FISH probes, but now labeled with, in some cases, a different dye. As the transcripts are fixed in cells, the fluorescent spots corresponding to single mRNAs remain in place during multiple rounds of hybridization, and can be aligned to read out a color sequence. Each mRNA species is therefore assigned a unique barcode. The number of each transcript in a given cell can be determined by counting the number of the corresponding barcode. Exemplary processes are illustrated in FIGs. 1, 2, and 3, and practical examples based on the methods disclosed herein are provided, for example, in U.S. Patent Publication No. 2016-0369329.

### EXAMPLE 2

### OLIGONUCLEOTIDE PREPARATION

A set of sequences were amplified by PCR (FIG. 4). The product was isolated, e.g., precipitated using 5 volumes of precipitation buffer (30:1 EtOH : 1M NaOAc) at -20 °C for at least 10 minutes. The precipitation mixture was centrifuged for 10 minutes. The supernatant was discarded and the oligonucleotide pellet was reconstituted in nicking enzyme buffer with the appropriate units of enzyme, based on that about 10 units of enzyme digest about 1 µg of DNA in 1 hour. Once the incubation time had elapsed, the sample was again precipitated and reconstituted in 2x loading buffer (96% formamide/20mM EDTA) and water to make a final loading buffer (48% formamide/10mM EDTA). The sample was heated to 95 °C to completely denature the DNA. The denatured DNA was then loaded into a denaturing acrylamide gel (8M urea 10-12% acrylamide). The gel was run at 250V for 1 hour, or optimized as desired. After electrophoresis, the gel was stained using 1x sybr gold for 15 minutes and then visualized. The appropriate band was cut out, crushed, and incubated in DI water for 2 hours. After incubation, the sample was precipitated again and then purified using a vacuum column. The column was eluted with 30 µL of RNase free water to yield the final product, as shown in Figure 26.

In some embodiments, the methods exemplified herein can use restriction sites instead of nicking endonuclease sites. Similar to the amplification step in FIG. 25, a set of sequences are amplified by PCR, with a BamHI site flanking the 5'-end, and an AatII site flanking the 3'-end. The PCR product is precipitated with 5 volumes of precipitation buffer (30:1 EtOH : 1M NaOAc) at -20 °C for at least 10 minutes and isolated, followed by digestion with BamHI and AatII. The product is again purified, and subjected to exo III digestion. Removal of the digested nucleic acids provides the product oligonucleotides.

### Synthesis of DNA probes-disulfide-dye conjugates

An exemplary scheme for synthesizing readout probes-dye conjugates connected by a disulfide bond. Thiol-modified DNA probes were ordered from Integrated DNA Technologies in their oxidized form. 10nmoles of thiol-modified DNA probes was treated with 10mM TCEP at 37°C for 30 minutes. After reduction step and gel column purified, the DNA probes were mixed with 50 equivalents of 3-(2-Pyridyldithio) propionic acid N- hydroxysuccinimide ester (SPDP) linker in 1x PBS solution containing 10mM EDTA. The mixture was allowed to react at room temperature for at least 2hours. Immediately after the reaction, the mixture was spin column purified and was resuspended in 60uL of 1x PBS containing 100ug of cadaverine dyes. The reaction was allowed to proceed at room temperature for at least 4 hours before subjected to ethanol-precipitation purification and HPLC purified. The concentration of the final product was determined using Nanodrop.

Technically, any heterobifunctional cross-linking reagent that can connect between the dye and thiol-modified DNA probes will work for this rehybridization scheme. DNA probes-disulfide-dye conjugates were synthesized using 3-(2-pyridyldithio) propionyl hydrazide (PDPH) linker and NHS ester dyes which work equally well as former conjugates.

### EXAMPLE 3

### DETECTION OF NUCLEIC ACID TARGET MOLECULES USING SEQUENTIAL HYBRIDIZATION WITH SELECTIVE REMOVAL OF READOUT PROBES BETWEEN HYBRIDIZATION ROUNDS

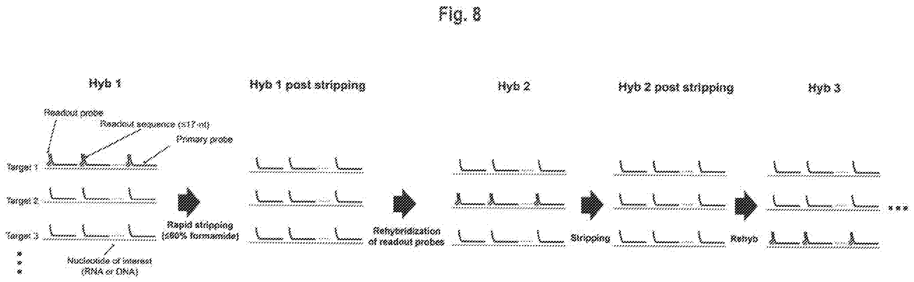

In a exemplary sequential hybridization and barcoding protocol, an efficient method to selectively remove readout probes without affecting the target molecule or disrupting the interaction between the target molecule and the and a plurality of primary nucleic acid probes were incorporated. In this example, sequential hybridization and barcoding were carried out on mouse embryonic stem cells (mESCs) to detect Rlim mRNA. *See, e.g.,* the exemplary process illustrated in FIG. 8.

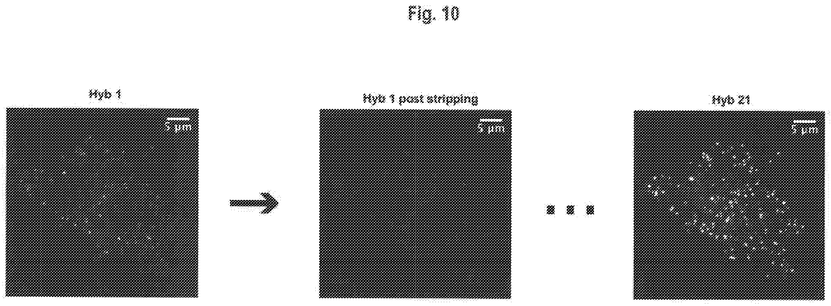

Rlim mRNA transcripts in mouse embryonic stem cells (mESCs) were targeted with a plurality of primary nucleic acid (ssDNA) probes, wherein each primary nucleic acid probe having a unique sequence hybridized to a unique target mRNA molecule. In the first round of hybridization (hyb 1), readout probes of 15 nucleotides in length and labeled with Cy3B were contacted with the target mRNA and bound primary probes. Subsequent to hybridization of the readout probes and imaging, the slide was washed with 50% (v/v) formamide solution at room temperature for 5 minutes. After the formamide wash, in a second round of hybridization (hyb 2), a second set of readout probes of 15 nucleotides in length and labeled with Cy3B were contacted with the fixed cells. After imaging of hyb 2, the wash with formamide was carried out. Each subsequent round of hybridization and imaging was followed with the formamide wash step up to hyb 21, in which the same cells were targeted with the same probes as in hyb 1 after 20 rounds of hybridization, imaging, and washing. FIG. 10 illustrates a set of representative confocal images obtained with the described protocol. Images are shown as maximum intensity projection of z stack fluorescent images with the same contrast levels.

### EXAMPLE 4

### DETECTION OF TARGET MOLECULES WITH ANTIBODIES USING SEQUENTIAL HYBRIDIZATION WITH SELECTIVE REMOVAL OF READOUT PROBES BETWEEN HYBRIDIZATION ROUNDS

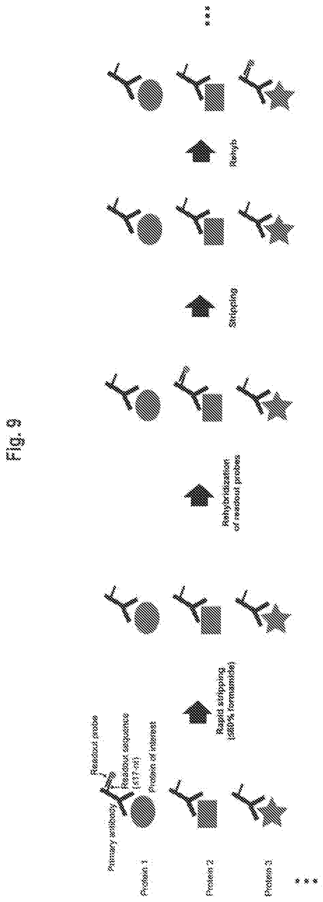

One or more target molecules of interest can be detected in a sample or a cell using a sequential hybridization method as disclosed herein. Using standard protocols, a sample is prepared by fixation and contacted with a primary antibody solution that specifically detects a target molecule in the sample. The primary antibody includes a nucleic acid readout sequence that is 17 nucleotides or less in length. The target molecule with the bound primary antibody can then be detected or barcoded using the sequential hybridization methods disclosed herein, *e.g*., with detectably labeled oligonucleotides, such as readout probes labeled with unique fluorophores. The readout probes include sequences that are complementary to the readout sequence on the primary antibody. Between rounds of hybridization and imaging, the sample is washed with formamide solution (*e.g.* 50% v/v) to selectively remove the readout probes of each hybridization round prior to hybridization with a subsequent set of readout probes in a subsequent hybridization round. The one or more target molecules in the sample can be a protein of interest. *See, e.g.,* the exemplary process illustrated in FIG. 9.

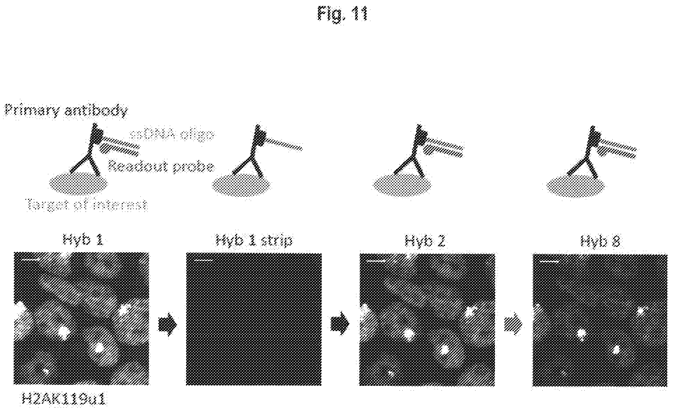

A particular example is illustrated in FIG. 11. In this example, a pool of antibodies was conjugated with oligonucleotides. Antibody 1 ("AB1") was conjugated to oligonucleotide 1, antibody 2 ("AB2") was conjugated to oligonucleotide 2, and so on. The antibody solution was then applied to fixed cells, and the primary antibodies were detected using sequential hybridization methods disclosed herein. Between rounds of hybridization and imaging, the cells were washed with 30% (v/v) formamide solution to selectively remove the readout probes of each hybridization round prior to hybridization with a subsequent set of readout probes in a subsequent hybridization round. The readout probes were 12 nucleotides in length. This example illustrates that sequential hybridization can be carried out not only a single antibody but with a plurality of antibodies for detecting target molecules in cells.

### EQUIVALENTS

Having described some illustrative embodiments of the invention, it should be apparent to those skilled in the art that the foregoing is merely illustrative and not limiting, having been presented by way of example only. Numerous modifications and other illustrative embodiments are within the scope of one of ordinary skill in the art and are contemplated as falling within the scope of the invention. In particular, although many of the examples presented herein involve specific combinations of method acts or system elements, it should be understood that those acts and those elements may be combined in other ways to accomplish the same objectives. Acts, elements, and features discussed only in connection with one embodiment are not intended to be excluded from a similar role in other embodiments. Further, for the one or more means-plus-function limitations recited in the following claims, the means are not intended to be limited to the means disclosed herein for performing the recited function, but are intended to cover in scope any means, known now or later developed, for performing the recited function.

Use of ordinal terms such as "first", "second", "third", *etc.,* in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements. Similarly, use of a), b)*, etc.,* or i), ii), *etc.* does not by itself connote any priority, precedence, or order of steps in the claims. Similarly, the use of these terms in the specification does not by itself connote any required priority, precedence, or order.

The foregoing written specification is considered to be sufficient to enable one skilled in the art to practice the invention. The present invention is not to be limited in scope by examples provided, since the examples are intended as a single illustration of one aspect of the invention and other functionally equivalent embodiments are within the scope of the invention. Various modifications of the invention in addition to those shown and described herein will become apparent to those skilled in the art from the foregoing description and fall within the scope of the appended claims. The advantages and objects of the invention are not necessarily encompassed by each embodiment of the invention.

## Claims

1. A sequential hybridization method, comprising:
a) contacting a target nucleic acid molecule with a plurality of primary probes,
wherein each primary probe comprises:
a primary binding sequence that binds to a complementary target sequence within the target nucleic acid molecule, and
a first overhang sequence connected to one end of the primary binding sequence comprising one or more binding targets connected in series and linked to the primary binding sequence;
b)contacting the target nucleic acid molecule with a first plurality of readout probes, wherein each readout probe comprises a signal moiety, and wherein each readout probe interacts with an overhang sequence connected to a primary probe of the plurality of primary probes,
wherein the signal moiety is capable of emitting a first detectable visual signal upon the interaction of each readout probe from the first plurality of readout probes with the first overhang sequence of the primary probe;
c)imaging the target nucleic acid molecule after step b) so that the interactions between the first plurality of readout probes and the plurality of primary probes are detected by the presence of a first detectable visual signal;
d)contacting the target nucleic acid molecule, the plurality of primary probes, and the first plurality of readout probes with a solution comprising a denaturing agent, wherein the denaturing agent comprises formamide present in the solution at a percent concentration of about 40%, 41%, 42%, 43%, 44%, 45%, 46%, 47%, 48%, 49%, 50%, 51%, 52%, 53%, 54%, or 55% (v/v) or urea present in a solution at a concentration of 2M to 5M wherein contact of the solution with the target nucleic acid molecule, the plurality of primary probes, and the first plurality of readout probes does not disrupt the interaction between the plurality of primary probes and the target nucleic acid molecule but does disrupt the interaction between the plurality of primary probes and the plurality of readout probes allowing removal of the plurality of readout probes;
e) contacting the target nucleic acid molecule with a second plurality of readout probes, wherein each readout probe comprises a signal moiety, and wherein each readout probe interacts with an overhang sequence of the_primary probe,
wherein the signal moiety is capable of emitting a second detectable visual signal upon the interaction of each readout probe from the second plurality of readout probes with the overhang sequence of the primary probe; and
f) imaging the target nucleic acid molecule after step e) so that the interactions between the second plurality of readout probes and the plurality of primary probes are detected by the presence of the second detectable visual signal;
repeating steps d)-f) each repeat with a new plurality of readout probes, so that a target nucleic acid in the sample is described by a barcode, and can be differentiated from another target nucleic acid in the sample by a difference in their barcodes.

2. The method of claim 1, further comprising
g)contacting the target nucleic acid molecule interacting with a primary probe with a bridge probe in a first plurality of bridge probes, wherein the bridge probe comprises a binding sequence that specifically binds to all or a part of the first overhang sequence of a primary probe of the plurality of primary probes, and one or more readout probes connected in series

3. The method of claim 1, wherein each readout probe in any plurality of readout probes interacts with its binding target by hybridizing to a bridge probe that comprises: (i) a sequence that is complementary to all or part of the first overhang sequence of a primary probe of the plurality of primary probes, and (ii) a sequence to which the readout probe binds.

4. The method of any one of claims 1-3, wherein the target nucleic acid molecule is an RNA or a DNA, or wherein the target nucleic acid molecule is within an intact cell, or wherein the intact cell is a prokaryotic cell, or wherein the intact cell is a eukaryotic cell, or wherein the intact cell is a mammalian cell, or wherein the intact cell is a human cell.

5. A sequential hybridization method, comprising:
a) contacting a target molecule with a plurality of primary antibodies, wherein each primary antibody comprises one or more binding targets connected in series and linked to the primary antibody;
b) contacting the primary antibody with a first plurality of readout probes, wherein each readout probe comprises a signal moiety, and wherein each readout probe interacts with a first binding target of the one or more binding targets of a primary antibody of the plurality of primary antibodies,
wherein the signal moiety is capable of emitting a first detectable visual signal upon the interaction of each readout probe from the first plurality of readout probes to the first binding target of a primary antibody of the plurality of primary antibodies;
c) imaging the target molecule after step b) so that the interactions between the first plurality of readout probes and the plurality of primary antibodies are detected by the presence of the first detectable visual signal;
d) contacting the target molecule, the plurality of primary antibodies and the first plurality of readout probes with a solution comprising a denaturing agent, wherein the denaturing agent comprises formamide present in the solution at a percent concentration of about 40%, 41%, 42%, 43%, 44%, 45%, 46%, 47%, 48%, 49%, 50%, 51%, 52%, 53%, 54%, or 55% (v/v) or urea present in a solution at a concentration of 2M to 5M wherein contact of the solution with the target molecule, the plurality of primary antibodies, and the first plurality of readout probes does not disrupt the interaction between the plurality of primary antibodies and the target molecule but does disrupt the interaction between the first plurality of primary antibodies and the first plurality of readout probes allowing removal of the first plurality of readout probes;
e) contacting the plurality of primary antibodies with a second plurality of readout probes, wherein each readout probe comprises a signal moiety, and wherein each readout probe interacts with a second binding target of a primary antibody of the plurality of primary antibodies,
wherein the signal moiety is capable of emitting a second detectable visual signal upon the interaction of each readout probe with the second binding target of a primary antibody of the plurality of primary antibodies;
f) imaging the target molecule after step e) so that interactions between the second plurality of readout probes and the plurality of primary antibodies are detected by the presence of the second detectable visual signal, and
repeating steps d)-f) each repeat with a new plurality of readout probes, so that a target molecule in the sample is described by a barcode, and can be differentiated from another target molecule in the sample by a difference in their barcodes.

6. The method of claim 5, wherein each readout probe in any plurality of readout probes interacts with its binding target by hybridizing to its binding target in a primary antibody of the plurality of primary antibodies.

7. The method of claim 5 or 6, wherein each readout probe in any plurality of readout probes interacts with its binding target by hybridizing to a bridge probe that comprises: (i) a sequence that is complementary to the one or more binding targets of a primary antibody of the plurality of primary antibodies, and (ii) a sequence to which the readout probe binds.

8. The method of any one of claims 5-7, wherein the target molecule is an RNA, a DNA, or a protein, or wherein the target molecule is within an intact cell, or wherein the intact cell is a prokaryotic cell, or wherein the intact cell is a eukaryotic cell, or wherein the intact cell is a mammalian cell, or wherein the intact cell is a human cell.

9. The method of claim 1 or claim 5, wherein the one or more binding targets comprises three or more binding targets, or wherein the additional one or more binding targets comprises three or more readout binding targets.

10. The method of any one of claims 1 to 9, wherein the readout probes are 17 nucleotides in length, or wherein the readout probes are less than 17 nucleotides in length, or wherein the readout probes are between 10 and 17 nucleotides in length, or wherein the readout probes are between 11 and 17 nucleotides in length, or wherein the readout probes are between 12 and 17 nucleotides in length, or wherein the readout probes are between 13 and 17 nucleotides in length, or wherein the readout probes are between 14 and 17 nucleotides in length, or wherein the readout probes are between 15 and 17 nucleotides in length, or wherein the readout probes are less than 10 nucleotides in length, or wherein the readout probes are between 5 and 10 nucleotides in length, or wherein the readout probes are between 6 and 9 nucleotides in length, or wherein the readout probes are 7-8 nucleotides in length.

11. The method of claim 1, wherein each readout probe forms a hairpin structure, and wherein the presence of an initiator sequence causes the hairpin structure to unfold initiating a hybridization chain reaction (HCR).

## Patentansprüche

1. Ein Verfahren zur sequenziellen Hybridisierung, das Folgendes beinhaltet:
a) In-Kontakt-Bringen eines Zielnukleinsäuremoleküls mit einer Vielzahl von primären Sonden, wobei jede primäre Sonde Folgendes beinhaltet:
eine primäre Bindungssequenz, die an eine komplementäre Zielsequenz innerhalb des Zielnukleinsäuremoleküls bindet, und
eine erste Überhangsequenz, die mit einem Ende der primären Bindungssequenz verbunden ist und die ein oder mehrere Bindungsziele beinhaltet, die in Reihe verbunden und mit der primären Bindungssequenz verknüpft sind;
b) In-Kontakt-Bringen des Zielnukleinsäuremoleküls mit einer ersten Vielzahl von Auslesesonden, wobei jede Auslesesonde eine Signalgruppierung beinhaltet und wobei jede Auslesesonde mit einer Überhangsequenz interagiert, die mit einer primären Sonde der Vielzahl von primären Sonden verbunden ist,
wobei die Signalgruppierung in der Lage ist, bei der Interaktion jeder Auslesesonde von der ersten Vielzahl von Auslesesonden mit der ersten Überhangsequenz der primären Sonde ein erstes detektierbares visuelles Signal zu emittieren;
c) Abbilden des Zielnukleinsäuremoleküls nach Schritt b), sodass die Interaktionen zwischen der ersten Vielzahl von Auslesesonden und der Vielzahl von primären Sonden durch das Vorhandensein eines ersten detektierbaren visuellen Signals detektiert werden;
d) In-Kontakt-Bringen des Zielnukleinsäuremoleküls, der Vielzahl von primären Sonden und der ersten Vielzahl von Auslesesonden mit einer Lösung, die ein Denaturierungsmittel beinhaltet, wobei das Denaturierungsmittel Formamid, das in der Lösung in einer prozentualen Konzentration von etwa 40 %, 41 %, 42 %, 43 %, 44 %, 45 %, 46 %, 47 %, 48 %, 49 %, 50 %, 51 %, 52 %, 53 %, 54 % oder 55 % (v/v) vorhanden ist, oder Harnstoff, der in einer Lösung in einer Konzentration von 2 M bis 5 M vorhanden ist, beinhaltet, wobei der Kontakt der Lösung mit dem Zielnukleinsäuremolekül, der Vielzahl von primären Sonden und der ersten Vielzahl von Auslesesonden die Interaktion zwischen der Vielzahl von primären Sonden und dem Zielnukleinsäuremolekül nicht stört, aber die Interaktion zwischen der Vielzahl von primären Sonden und der Vielzahl von Auslesesonden stört, was die Entfernung der Vielzahl von Auslesesonden ermöglicht;
e) In-Kontakt-Bringen des Zielnukleinsäuremoleküls mit einer zweiten Vielzahl von Auslesesonden, wobei jede Auslesesonde eine Signalgruppierung beinhaltet und wobei jede Auslesesonde mit einer Überhangsequenz der primären Sonde interagiert,
wobei die Signalgruppierung in der Lage ist, bei der Interaktion jeder Auslesesonde von der zweiten Vielzahl von Auslesesonden mit der Überhangsequenz der primären Sonde ein zweites detektierbares visuelles Signal zu emittieren; und
f) Abbilden des Zielnukleinsäuremoleküls nach Schritt e), sodass die Interaktionen zwischen der zweiten Vielzahl von Auslesesonden und der Vielzahl von primären Sonden durch das Vorhandensein des zweiten detektierbaren visuellen Signals detektiert werden;
Wiederholen der Schritte d)-f) mit einer neuen Vielzahl von Auslesesonden je Wiederholung, sodass eine Zielnukleinsäure in der Probe durch einen Barcode beschrieben wird und von einer anderen Zielnukleinsäure in der Probe durch einen Unterschied in ihren Barcodes unterschieden werden kann.

2. Verfahren gemäß Anspruch 1, das ferner Folgendes beinhaltet:
g) In-Kontakt-Bringen des Zielnukleinsäuremoleküls, das mit einer primären Sonde interagiert, mit einer Brückensonde in einer ersten Vielzahl von Brückensonden, wobei die Brückensonde eine Bindungssequenz, die spezifisch an die gesamte oder einen Teil der ersten Überhangsequenz einer primären Sonde der Vielzahl von primären Sonden bindet, und eine oder mehrere Auslesesonden, die in Reihe verbunden sind, beinhaltet.

3. Verfahren gemäß Anspruch 1, wobei jede Auslesesonde in einer beliebigen Vielzahl von Auslesesonden mit ihrem Bindungsziel durch Hybridisieren an eine Brückensonde interagiert, die Folgendes beinhaltet: (i) eine Sequenz, die zu der gesamten oder einem Teil der ersten Überhangsequenz einer primären Sonde der Vielzahl von primären Sonden komplementär ist, und (ii) eine Sequenz, an die die Auslesesonde bindet.

4. Verfahren gemäß einem der Ansprüche 1-3, wobei das Zielnukleinsäuremolekül eine RNA oder eine DNA ist oder wobei sich das Zielnukleinsäuremolekül innerhalb einer intakten Zelle befindet oder wobei die intakte Zelle eine prokaryotische Zelle ist oder wobei die intakte Zelle eine eukaryotische Zelle ist oder wobei die intakte Zelle eine Säugerzelle ist oder wobei die intakte Zelle eine menschliche Zelle ist.

5. Ein Verfahren zur sequenziellen Hybridisierung, das Folgendes beinhaltet:
a) In-Kontakt-Bringen eines Zielmoleküls mit einer Vielzahl von primären Antikörpern, wobei jeder primäre Antikörper ein oder mehrere Bindungsziele beinhaltet, die in Reihe verbunden und mit dem primären Antikörper verknüpft sind;
b) In-Kontakt-Bringen des primären Antikörpers mit einer ersten Vielzahl von Auslesesonden, wobei jede Auslesesonde eine Signalgruppierung beinhaltet und wobei jede Auslesesonde mit einem ersten Bindungsziel des einen oder der mehreren Bindungsziele eines primären Antikörpers der Vielzahl von primären Antikörpern interagiert;
wobei die Signalgruppierung in der Lage ist, bei der Interaktion jeder Auslesesonde von der ersten Vielzahl von Auslesesonden mit dem ersten Bindungsziel eines primären Antikörpers der Vielzahl von primären Antikörpern ein erstes detektierbares visuelles Signal zu emittieren;
c) Abbilden des Zielmoleküls nach Schritt b), sodass die Interaktionen zwischen der ersten Vielzahl von Auslesesonden und der Vielzahl von primären Antikörpern durch das Vorhandensein des ersten detektierbaren visuellen Signals detektiert werden;
d) In-Kontakt-Bringen des Zielmoleküls, der Vielzahl von primären Antikörpern und der ersten Vielzahl von Auslesesonden mit einer Lösung, die ein Denaturierungsmittel beinhaltet, wobei das Denaturierungsmittel Formamid, das in der Lösung in einer prozentualen Konzentration von etwa 40 %, 41 %, 42 %, 43 %, 44 %, 45 %, 46 %, 47 %, 48 %, 49 %, 50 %, 51 %, 52 %, 53 %, 54 % oder 55 % (v/v) vorhanden ist, oder Harnstoff, der in einer Lösung in einer Konzentration von 2 M bis 5 M vorhanden ist, beinhaltet, wobei der Kontakt der Lösung mit dem Zielmolekül, der Vielzahl von primären Antikörpern und der ersten Vielzahl von Auslesesonden die Interaktion zwischen der Vielzahl von primären Antikörpern und dem Zielmolekül nicht stört, aber die Interaktion zwischen der ersten Vielzahl von primären Antikörpern und der ersten Vielzahl von Auslesesonden stört, was die Entfernung der ersten Vielzahl von Auslesesonden ermöglicht;
e) In-Kontakt-Bringen der Vielzahl von primären Antikörpern mit einer zweiten Vielzahl von Auslesesonden, wobei jede Auslesesonde eine Signalgruppierung beinhaltet und wobei jede Auslesesonde mit einem zweiten Bindungsziel eines primären Antikörpers der Vielzahl von primären Antikörpern interagiert,
wobei die Signalgruppierung in der Lage ist, bei der Interaktion jeder Auslesesonde mit dem zweiten Bindungsziel eines primären Antikörpers der Vielzahl von primären Antikörpern ein zweites detektierbares visuelles Signal zu emittieren;
f) Abbilden des Zielmoleküls nach Schritt e), sodass Interaktionen zwischen der zweiten Vielzahl von Auslesesonden und der Vielzahl von primären Antikörpern durch das Vorhandensein des zweiten detektierbaren visuellen Signals detektiert werden, und
Wiederholen der Schritte d)-f) mit einer neuen Vielzahl von Auslesesonden je Wiederholung, sodass ein Zielmolekül in der Probe durch einen Barcode beschrieben wird und von einem anderen Zielmolekül in der Probe durch einen Unterschied in ihren Barcodes unterschieden werden kann.

6. Verfahren gemäß Anspruch 5, wobei jede Auslesesonde in einer beliebigen Vielzahl von Auslesesonden mit ihrem Bindungsziel durch Hybridisieren an ihr Bindungsziel in einem primären Antikörper der Vielzahl von primären Antikörpern interagiert.

7. Verfahren gemäß Anspruch 5 oder 6, wobei jede Auslesesonde in einer beliebigen Vielzahl von Auslesesonden mit ihrem Bindungsziel durch Hybridisieren an eine Brückensonde interagiert, die Folgendes beinhaltet: (i) eine Sequenz, die zu dem einen oder den mehreren Bindungszielen eines primären Antikörpers der Vielzahl von primären Antikörpern komplementär ist, und (ii) eine Sequenz, an die die Auslesesonde bindet.

8. Verfahren gemäß einem der Ansprüche 5-7, wobei das Zielmolekül eine RNA, eine DNA oder ein Protein ist oder wobei sich das Zielmolekül innerhalb einer intakten Zelle befindet oder wobei die intakte Zelle eine prokaryotische Zelle ist oder wobei die intakte Zelle eine eukaryotische Zelle ist oder wobei die intakte Zelle eine Säugerzelle ist oder wobei die intakte Zelle eine menschliche Zelle ist.

9. Verfahren gemäß Anspruch 1 oder Anspruch 5, wobei das eine oder die mehreren Bindungsziele drei oder mehr Bindungsziele beinhalten oder wobei das zusätzliche eine oder die zusätzlichen mehreren Bindungsziele drei oder mehr Auslesebindungsziele beinhalten.

10. Verfahren gemäß einem der Ansprüche 1 bis 9, wobei die Auslesesonden 17 Nukleotide lang sind oder wobei die Auslesesonden weniger als 17 Nukleotide lang sind oder wobei die Auslesesonden zwischen 10 und 17 Nukleotide lang sind oder wobei die Auslesesonden zwischen 11 und 17 Nukleotide lang sind oder wobei die Auslesesonden zwischen 12 und 17 Nukleotide lang sind oder wobei die Auslesesonden zwischen 13 und 17 Nukleotide lang sind oder wobei die Auslesesonden zwischen 14 und 17 Nukleotide lang sind oder wobei die Auslesesonden zwischen 15 und 17 Nukleotide lang sind oder wobei die Auslesesonden weniger als 10 Nukleotide lang sind oder wobei die Auslesesonden zwischen 5 und 10 Nukleotide lang sind oder wobei die Auslesesonden zwischen 6 und 9 Nukleotide lang sind oder wobei die Auslesesonden 7-8 Nukleotide lang sind.

11. Verfahren gemäß Anspruch 1, wobei jede Auslesesonde eine Haarnadelstruktur bildet und wobei das Vorhandensein einer Initiatorsequenz bewirkt, dass sich die Haarnadelstruktur entfaltet und eine Hybridisierungskettenreaktion (HCR) initiiert.

## Revendications

1. Un procédé d'hybridation séquentielle, comprenant :
a) la mise en contact d'une molécule d'acide nucléique cible avec une pluralité de sondes primaires, où chaque sonde primaire comprend :
une séquence de liaison primaire qui se lie à une séquence cible complémentaire au sein de la molécule d'acide nucléique cible, et
une première séquence en surplomb raccordée à une extrémité de la séquence de liaison primaire comprenant une ou plusieurs cibles de liaison raccordées en série et reliées à la séquence de liaison primaire ;
b) la mise en contact de la molécule d'acide nucléique cible avec une première pluralité de sondes de lecture, où chaque sonde de lecture comprend une partie signal, et où chaque sonde de lecture interagit avec une séquence en surplomb raccordée à une sonde primaire de la pluralité de sondes primaires,
où la partie signal est capable d'émettre un premier signal visuel détectable suite à l'interaction de chaque sonde de lecture parmi la première pluralité de sondes de lecture avec la première séquence en surplomb de la sonde primaire ;
c) la production d'une image de la molécule d'acide nucléique cible après l'étape b) de sorte que les interactions entre la première pluralité de sondes de lecture et la pluralité de sondes primaires sont détectées par la présence d'un premier signal visuel détectable ;
d) la mise en contact de la molécule d'acide nucléique cible, de la pluralité de sondes primaires, et de la première pluralité de sondes de lecture avec une solution comprenant un agent dénaturant, où l'agent dénaturant comprend du formamide présent dans la solution à une concentration en pourcentage d'environ 40 %, 41 %, 42 %, 43 %, 44 %, 45 %, 46 %, 47 %, 48 %, 49 %, 50 %, 51 %, 52 %, 53 %, 54 %, ou 55 % (v/v) ou de l'urée présente dans une solution à une concentration de 2 M à 5 M où un contact de la solution avec la molécule d'acide nucléique cible, la pluralité de sondes primaires, et la première pluralité de sondes de lecture ne perturbe pas l'interaction entre la pluralité de sondes primaires et la molécule d'acide nucléique cible mais perturbe l'interaction entre la pluralité de sondes primaires et la pluralité de sondes de lecture permettant le retrait de la pluralité de sondes de lecture ;
e) la mise en contact de la molécule d'acide nucléique cible avec une deuxième pluralité de sondes de lecture, où chaque sonde de lecture comprend une partie signal, et où chaque sonde de lecture interagit avec une séquence en surplomb de la sonde primaire,
où la partie signal est capable d'émettre un deuxième signal visuel détectable suite à l'interaction de chaque sonde de lecture parmi la deuxième pluralité de sondes de lecture avec la séquence en surplomb de la sonde primaire ; et
f) la production d'une image de la molécule d'acide nucléique cible après l'étape e) de sorte que les interactions entre la deuxième pluralité de sondes de lecture et la pluralité de sondes primaires sont détectées par la présence du deuxième signal visuel détectable ;
en répétant les étapes d) à f) avec à chaque répétition une nouvelle pluralité de sondes de lecture, de sorte qu'un acide nucléique cible dans l'échantillon est décrit par un code-barres, et peut être différencié d'un autre acide nucléique cible dans l'échantillon par une différence dans leurs codes-barres.

2. Le procédé de la revendication 1, comprenant en outre
g) la mise en contact de la molécule d'acide nucléique cible interagissant avec une sonde primaire avec une sonde de pont dans une première pluralité de sondes de pont, où la sonde de pont comprend une séquence de liaison qui se lie spécifiquement à la totalité ou à une partie de la première séquence en surplomb d'une sonde primaire de la pluralité de sondes primaires, et une ou plusieurs sondes de lecture raccordées en série.

3. Le procédé de la revendication 1, où chaque sonde de lecture dans n'importe quelle pluralité de sondes de lecture interagit avec sa cible de liaison par hybridation à une sonde de pont qui comprend : (i) une séquence qui est complémentaire à la totalité ou à une partie de la première séquence en surplomb d'une sonde primaire de la pluralité de sondes primaires, et (ii) une séquence à laquelle la sonde de lecture se lie.

4. Le procédé de n'importe laquelle des revendications 1 à 3, où la molécule d'acide nucléique cible est un ARN ou un ADN, ou bien où la molécule d'acide nucléique cible est au sein d'une cellule intacte, ou bien où la cellule intacte est une cellule procaryote, ou bien où la cellule intacte est une cellule eucaryote, ou bien où la cellule intacte est une cellule de mammifère, ou bien où la cellule intacte est une cellule humaine.

5. Un procédé d'hybridation séquentielle, comprenant :
a) la mise en contact d'une molécule cible avec une pluralité d'anticorps primaires, où chaque anticorps primaire comprend une ou plusieurs cibles de liaison raccordées en série et reliées à l'anticorps primaire ;
b) la mise en contact de l'anticorps primaire avec une première pluralité de sondes de lecture, où chaque sonde de lecture comprend une partie signal, et où chaque sonde de lecture interagit avec une première cible de liaison des une ou plusieurs cibles de liaison d'un anticorps primaire de la pluralité d'anticorps primaires ; où la partie signal est capable d'émettre un premier signal visuel détectable suite à l'interaction de chaque sonde de lecture parmi la première pluralité de sondes de lecture sur la première cible de liaison d'un anticorps primaire de la pluralité d'anticorps primaires ;
c) la production d'une image de la molécule cible après l'étape b) de sorte que les interactions entre la première pluralité de sondes de lecture et la pluralité d'anticorps primaires sont détectées par la présence du premier signal visuel détectable ;
d) la mise en contact de la molécule cible, de la pluralité d'anticorps primaires et de la première pluralité de sondes de lecture avec une solution comprenant un agent dénaturant, où l'agent dénaturant comprend du formamide présent dans la solution à une concentration en pourcentage d'environ 40 %, 41 %, 42 %, 43 %, 44 %, 45 %, 46 %, 47 %, 48 %, 49 %, 50 %, 51 %, 52 %, 53 %, 54 %, ou 55 % (v/v) ou de l'urée présente dans une solution à une concentration de 2 M à 5 M où un contact de la solution avec la molécule cible, la pluralité d'anticorps primaires, et la première pluralité de sondes de lecture ne perturbe pas l'interaction entre la pluralité d'anticorps primaires et la molécule cible mais perturbe l'interaction entre la première pluralité d'anticorps primaires et la première pluralité de sondes de lecture permettant le retrait de la première pluralité de sondes de lecture ;
e) la mise en contact de la pluralité d'anticorps primaires avec une deuxième pluralité de sondes de lecture, où chaque sonde de lecture comprend une partie signal, et où chaque sonde de lecture interagit avec une deuxième cible de liaison d'un anticorps primaire de la pluralité d'anticorps primaires,
où la partie signal est capable d'émettre un deuxième signal visuel détectable suite à l'interaction de chaque sonde de lecture avec la deuxième cible de liaison d'un anticorps primaire de la pluralité d'anticorps primaires ;
f) la production d'une image de la molécule cible après l'étape e) de sorte que des interactions entre la deuxième pluralité de sondes de lecture et la pluralité d'anticorps primaires sont détectées par la présence du deuxième signal visuel détectable, et
en répétant les étapes d) à f) avec à chaque répétition une nouvelle pluralité de sondes de lecture, de sorte qu'une molécule cible dans l'échantillon est décrite par un code-barres, et peut être différenciée d'une autre molécule cible dans l'échantillon par une différence dans leurs codes-barres.

6. Le procédé de la revendication 5, où chaque sonde de lecture dans n'importe quelle pluralité de sondes de lecture interagit avec sa cible de liaison par hybridation à sa cible de liaison dans un anticorps primaire de la pluralité d'anticorps primaires.

7. Le procédé de la revendication 5 ou 6, où chaque sonde de lecture dans n'importe quelle pluralité de sondes de lecture interagit avec sa cible de liaison par hybridation à une sonde de pont qui comprend : (i) une séquence qui est complémentaire aux une ou plusieurs cibles de liaison d'un anticorps primaire de la pluralité d'anticorps primaires, et (ii) une séquence à laquelle la sonde de lecture se lie.

8. Le procédé de n'importe laquelle des revendications 5 à 7, où la molécule cible est un ARN, un ADN, ou une protéine, ou bien où la molécule cible est au sein d'une cellule intacte, ou bien où la cellule intacte est une cellule procaryote, ou bien où la cellule intacte est une cellule eucaryote, ou bien où la cellule intacte est une cellule de mammifère, ou bien où la cellule intacte est une cellule humaine.

9. Le procédé de la revendication 1 ou de la revendication 5, où les une ou plusieurs cibles de liaison comprennent trois cibles de liaison ou plus, ou bien où les une ou plusieurs cibles de liaison additionnelles comprennent trois cibles de liaison de lecture ou plus.

10. Le procédé de n'importe laquelle des revendications 1 à 9, où les sondes de lecture ont une longueur de 17 nucléotides, ou bien où les sondes de lecture ont une longueur inférieure à 17 nucléotides, ou bien où les sondes de lecture ont une longueur comprise entre 10 et 17 nucléotides, ou bien où les sondes de lecture ont une longueur comprise entre 11 et 17 nucléotides, ou bien où les sondes de lecture ont une longueur comprise entre 12 et 17 nucléotides, ou bien où les sondes de lecture ont une longueur comprise entre 13 et 17 nucléotides, ou bien où les sondes de lecture ont une longueur comprise entre 14 et 17 nucléotides, ou bien où les sondes de lecture ont une longueur comprise entre 15 et 17 nucléotides, ou bien où les sondes de lecture ont une longueur inférieure à 10 nucléotides, ou bien où les sondes de lecture ont une longueur comprise entre 5 et 10 nucléotides, ou bien où les sondes de lecture ont une longueur comprise entre 6 et 9 nucléotides, ou bien où les sondes de lecture ont une longueur de 7 à 8 nucléotides.

11. Le procédé de la revendication 1, où chaque sonde de lecture forme une structure en épingle à cheveux, et où la présence d'une séquence d'initiateur amène la structure en épingle à cheveux à se déplier en initiant une réaction en chaîne d'hybridation (HCR).
